(19) Europäisches Patentamt
European Patent Office
Office européen des brevets

(11) EP 3 636 651 A1

(12) EUROPEAN PATENT APPLICATION

(43) Date of publication:
**15.04.2020 Bulletin 2020/16**

(21) Application number: **19212082.2**

(22) Date of filing: **24.11.2016**

(51) Int Cl.:
***C07D 498/04*** *(2006.01)* ***C07D 498/14*** *(2006.01)*
***A61K 31/553*** *(2006.01)* ***A61P 25/24*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.11.2015 US 201562259776 P**

(62) Document number(s) of the earlier application(s) in accordance with Art. 76 EPC:
**16804731.4 / 3 380 483**

(71) Applicant: **AbbVie Deutschland GmbH & Co. KG**
**65189 Wiesbaden (DE)**

(72) Inventors:
- **BACKFISCH, Gisela**
  **67061 Ludwigshafen (DE)**
- **BAKKER, Margaretha Henrica Maria**
  **67061 Ludwigshafen (DE)**
- **BLAICH, Günter**
  **67061 Ludwigshafen (DE)**
- **BRAJE, Wilfried**
  **67061 Ludwigshafen (DE)**
- **DRESCHER, Karla**
  **67061 Ludwigshafen (DE)**
- **ERHARD, Thomas**
  **67061 Ludwigshafen (DE)**
- **HAUPT, Andreas**
  **67061 Ludwigshafen (DE)**
- **HOFT, Carolin**
  **67061 Ludwigshafen (DE)**
- **LAKICS, Viktor**
  **67061 Ludwigshafen (DE)**
- **MACK, Helmut**
  **67061 Ludwigshafen (DE)**
- **OELLIEN, Frank**
  **67061 Ludwigshafen (DE)**
- **PETER, Raimund**
  **Wilmslow, Cheshire SK9 6JB (GB)**
- **RELO, Ana Lucia**
  **67061 Ludwigshafen (DE)**

(74) Representative: **Bensiek, Stephan**
**AbbVie Deutschland GmbH & Co. KG**
**Marken & Patente (LU72/15)**
**Knollstraße 50**
**67061 Ludwigshafen (DE)**

Remarks:
This application was filed on 28-11-2019 as a divisional application to the application mentioned under INID code 62.

(54) **HEXAHYDROPYRAZINOBENZ- OR -PYRIDO-OXAZEPINES CARRYING AN OXYGEN-CONTAINING SUBSTITUENT AND USE THEREOF IN THE TREATMENT OF 5-HT2C-DEPENDENT DISORDERS**

(57) The present invention relates to compound of formula (I)

EP 3 636 651 A1

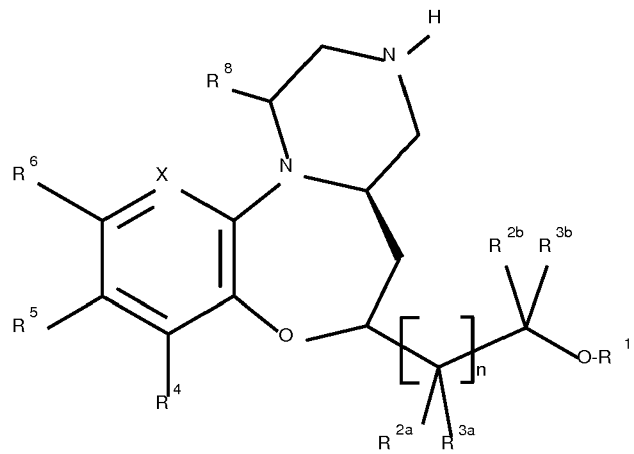

(I)

wherein the variables are as defined in the claims and the description. The invention further relates to a pharmaceutical composition containing such compounds, to their use as modulators, especially agonists or partial agonists, of the 5-$HT_{2C}$ receptor, to their use for preparing a medicament for the prevention or treatment of conditions and disorders which respond to the modulation of 5-$HT_{2C}$ receptor, and to methods for preventing or treating conditions and disorders which respond to the modulation of 5-$HT_{2C}$ receptor.

## Description

### FIELD OF THE INVENTION

The present invention relates to (4aR)-2,3,4,4a,5,6-hexahydro-1H-pyrazino[2,1-d][1,5]benzoxazepines and the analogous pyrido[3,2-b][1,4]oxazepine compounds carrying a hydroxyl, methoxy, deuterated methoxy or fluorinated methoxy substituent bound via a linking group, or a cyclic analogue thereof, to a pharmaceutical composition containing such compounds, to their use as modulators, especially agonists or partial agonists, of the 5-$HT_{2C}$ receptor, their use for preparing a medicament for the prevention or treatment of conditions and disorders which respond to the modulation of 5-$HT_{2C}$ receptor, to methods for preventing or treating conditions and disorders which respond to the modulation of 5-$HT_{2C}$ receptor, and processes for preparing such compounds and compositions.

### BACKGROUND OF THE INVENTION

Diseases, disorders and conditions where 5-$HT_{2C}$ modulation is desired are for example depression, anxiety, schizophrenia, bipolar disorder, obsessive compulsive disorder, migraine, pain, epilepsy, substance abuse, eating disorders, obesity, diabetes, erectile dysfunction and others.

Serotonin (5-hydroxytryptamine, 5-HT), a monoamine neurotransmitter and local hormone, is formed by the hydroxylation and decarboxylation of tryptophan. The greatest concentration is found in the enterochromaffin cells of the gastrointestinal tract, the remainder being predominantly present in platelets and in the Central Nervous System (CNS). 5-HT is implicated in a vast array of physiological and pathophysiological pathways. In the periphery, it contracts a number of smooth muscles and induces endothelium-dependent vasodilation. In the CNS, it is believed to be involved in a wide range of functions, including the control of appetite, mood, anxiety, hallucinations, sleep, vomiting and pain perception.

Neurons that secrete 5-HT are termed serotonergic. The function of 5-HT is exerted upon its interaction with specific (serotonergic) neurons. Seven types of 5-HT receptors have been identified: 5-$HT_1$ (with subtypes 5-$HT_{1A}$, 5-$HT_{1B}$, 5-$HT_{1D}$, 5-$HT_{1E}$ and 5-$HT_{1F}$), 5-$HT_2$ (with subtypes 5-$HT_{2A}$, 5-$HT_{2B}$ and 5-$HT_{2C}$), 5-$HT_3$, 5-$HT_4$, 5-$HT_5$ (with subtypes 5-$HT_{5A}$ and 5-$HT_{5B}$), 5-$HT_6$ and 5-$HT_7$. Most of these receptors are coupled to G-proteins that affect the activities of adenylate cyclase or phospholipase Cy.

Alterations in the activity of multiple neurotransmitter receptor systems (dopamine, serotonin, glutamate, GABA, acetylcholine) have been implicated in the manifestation of the symptoms of schizophrenia. The most widely accepted "Dopamine Hypothesis of Schizophrenia" in its simplest form states that the positive symptoms of this pathology relate to a functional hyperactivity of the mesolimbic dopaminergic system, while the negative and cognitive aspects can be traced to a functional hypoactivity of the mesocortical dopaminergic projections. Atypical antipsychotics block the mesolimbic dopaminergic neurotransmission, thereby controlling positive symptoms, with little or no effect on the nigrostriatal system, leading to less induction of extrapyramidal side effects (EPS).

Primary negative and cognitive symptoms of schizophrenia reflect a dysfunction of the frontal cortex ("hypofrontality"), which is thought to be induced by a decreased tone in the mesocortical dopaminergic projection field [Davis KL, Kahn RS, Ko G and Davidson M (1991). Dopamine in schizophrenia: a review and re-conceptualization. Am J Psychiatry 148: 1474 - 86. Weinberger DR and Berman KF (1996). Prefrontal function in schizophrenia: confounds and controversies. Philos Trans R Soc Lond B Biol Sci 351: 1495 - 503]. Agents that selectively enhance dopamine levels in the cortex have the potential to address the negative symptoms of this disorder. Atypical antipsychotics lack robust efficacy against negative and cognitive components of the schizophrenic syndrome.

The schizophrenic symptomatology is further complicated by the occurrence of drug-induced so-called secondary negative symptoms and cognitive impairment, which are difficult to distinguish from primary negative and cognitive symptoms [Remington G and Kapur S (2000). Atypical antipsychotics: are some more atypical than others? Psychopharmacol 148: 3 - 15]. The occurrence of secondary negative symptoms not only limits therapeutic efficacy but also, together with these side effects, negatively affects patient compliance.

It may thus be hypothesized that a novel mechanistic approach that blocks dopaminergic neurotransmission in the limbic system but does not affect the striatal and pituitary projection fields, and stimulates frontocortical projection fields, would provide an efficacious treatment for all parts of the schizophrenic pathology, including its positive, negative and cognitive symptoms. Moreover, a selective compound that is substantially free of the ancillary pharmacology that characterizes current agents would be expected to avoid a variety of off-target side effects that plague current treatments such as extrapyramidal side effects (EPS) and weight gain.

The 5-$HT_{2C}$ receptor, previously named 5-HT1C, is a G-protein-coupled receptor, which couples to multiple cellular effector systems including the phospholipase C, A and D pathways. It is found primarily in the brain and its distribution is particularly high in the plexus choroideus, where it is assumed to control cerebrospinal fluid production [Kaufman MJ, Hirata F (1996) Cyclic GMP inhibits phosphoinositide turnover in choroid plexus: evidence for interactions

between second messengers concurrently triggered by 5-HT2C receptors. Neurosci Lett 206:153-156]. Very high levels were also found in the retrosplenial, piriform and entorhinal cortex, anterior olfactory nucleus, lateral septal nucleus, subthalamic nucleus, amygdala, subiculum and ventral part of CA3, lateral habenula, substantia nigra pars compacta, several brainstem nuclei and the whole grey matter of the spinal cord [Pompeiano M, Palacios JM, Mengod G (1994). Distribution of the serotonin 5-HT2 receptor family mRNAs: comparison between 5-HT2A and 5-HT2C receptors. Brain Res Mol Brain Res 23:163-178]. A comparison of the distribution of 5-$HT_{2C}$ mRNA with that of 5-$HT_{2C}$ protein in monkey and human brains has revealed both pre- and postsynaptic localization [Lopez-Gimenez JF, Mengod G, Palacios JM, Vilaro MT (2001) Regional distribution and cellular localization of 5-HT2C receptor mRNA in monkey brain: comparison with [3H]mesulergine binding sites and choline acetyltransferase mRNA. Synapse 42:12-26].

It is anticipated that modulation of the 5-$HT_{2C}$ receptor will improve disorders such as depression, anxiety, schizophrenia, cognitive deficits of schizophrenia, obsessive compulsive disorder, bipolar disorder, neuropsychiatric symptoms in Parkinson' disease, in Alzheimer's disease, or Lewy Body dementia, migraine, epilepsy, substance abuse, eating disorders, obesity, diabetes, sexual dysfunction/erectile dysfunction, sleep disorders, psoriasis, Parkinson's disease, pain conditions and disorders, and spinal cord injury, smoking cessation, ocular hypertension, and Alzheimer's disease. Modulators of the 5-$HT_{2C}$ receptor are also shown to be useful in the modulation of bladder function, including the prevention or treatment of urinary incontinence.

Compounds with a structure similar to the compounds of the present invention have been described in WO 02/100350, WO 2010/124042, WO 2011/133182 and US 2011/0130382.

There is an ongoing need for providing compounds having high affinity and preferably also selectivity for the 5-$HT_{2C}$ receptor. In particular the compounds should have low affinity to adrenergic receptors, such as the $\alpha_1$-adrenergic receptor, histamine receptors, such as the Hi-receptor, and dopaminergic receptors, such as the $D_2$-receptor, in order to avoid or reduce side effects associated with modulation of these receptors, such as postural hypotension, reflex tachycardia, potentiation of the antihypertensive effect of prazosin, terazosin, doxazosin and labetalol or dizziness associated with the blockade of the $\alpha_1$-adrenergic receptor, weight gain, sedation, drowsiness or potentiation of central depressant drugs associated with the blockade of the Hi-receptor, or extrapyramidal movement disorder, such as dystonia, parkinsonism, akathisia, tardive dyskinesia or rabbit syndrome, or endocrine effects, such as prolactin elevation (galactorrhea, gynecomastia, mentstrual changes, sexual dysfunction in males), associated with the blockade of the $D_2$-receptor, and even more important no induction of weight gain in combination with severe metabolic dysfunction found for marketed antipsychotic drugs.

It is moreover desirable that the compounds have low affinity or alternatively an antagonistic effect to/on other serotonergic receptors, especially the 5-$HT_{2A}$ and/or 5-$HT_{2B}$ receptors, in order to avoid or reduce side effects associated with modulation of these receptors, such as changes (thickening) of the heart tissue associated with agonism at the 5-$HT_{2B}$ receptor, and psychotomimetic effect induced by agonism at the 5-$HT_{2A}$ receptor. Ideally they should show an agonistic action on the 5-$HT_{2C}$ receptor, an antagonistic action on the 5-$HT_{2A}$ receptor or alternatively no affinity to the 5-$HT_{2A}$ receptor and no affinity to the 5-$HT_{2B}$ receptor or alternatively an antagonistic action on the 5-$HT_{2B}$ receptor. Even more ideally the compounds should display an agonistic action on the 5-$HT_{2C}$ receptor in combination with an antagonistic action on the 5-$HT_{2A}$ receptor and no affinity to the 5-$HT_{2B}$ receptor.

Besides the affinity and selectivity for the 5-$HT_{2C}$ receptor, further properties may be advantageous for the treatment and/or prophylaxis of 5-$HT_{2C}$-related disorders, such as, for example:

1.) the metabolic stability, for example determined from the half-lives, measured in vitro, in liver microsomes from various species (e.g. rat or human);

2.) no or only low inhibition of cytochrome P450 (CYP) enzymes: cytochrome P450 (CYP) is the name for a superfamily of heme proteins having enzymatic activity (oxidase). They are also particularly important for the degradation (metabolism) of foreign substances such as drugs or xenobiotics in mammalian organisms. The principal representatives of the types and subtypes of CYP in the human body are: CYP 1A2, CYP 2C9, CYP 2D6 and CYP 3A4. If CYP 3A4 inhibitors (e.g. grapefruit juice, cimetidine, erythromycin) are used at the same time as medicinal substances which are degraded by this enzyme system and thus compete for the same binding site on the enzyme, the degradation thereof may be slowed down and thus effects and side effects of the administered medicinal substance may be undesirably enhanced;

3.) a suitable solubility in water (in mg/mL);

4.) suitable pharmacokinetics (time course of the concentration of the compound of the invention in plasma or in tissue, for example brain). The pharmacokinetics can be described by the following parameters: half-life (in h), volume of distribution (in l•kg-1), plasma clearance (in l•h-1•kg-1), AUC (area under the curve, area under the concentration-time curve, in ng•h•l-1), oral bioavailability (the dose-normalized ratio of AUC after oral administration and AUC after intravenous administration), the so-called brain-plasma ratio (the ratio of AUC in brain tissue and AUC in plasma);

5.) no or only low blockade of the hERG channel: compounds which block the hERG channel may cause a prolongation of the QT interval and thus lead to serious disturbances of cardiac rhythm (for example so-called "torsade de pointes"). The potential of compounds to block the hERG channel can be determined by means of the displacement assay with radiolabelled dofetilide which is described in the literature (G. J. Diaz et al., Journal of Pharmacological and Toxicological Methods, 50 (2004), 187 199). A smaller IC50 in this dofetilide assay means a greater probability of potent hERG blockade. In addition, the blockade of the hERG channel can be measured by electrophysiological experiments on cells which have been transfected with the hERG channel, by so-called whole-cell patch clamping (G. J. Diaz et al., Journal of Pharmacological and Toxicological Methods, 50 (2004), 187-199).

It was an object of the present invention to provide compounds for the treatment or prophylaxis of various 5-$HT_{2C}$-related diseases. The compounds were intended to have a high affinity to the 5-$HT_{2C}$ receptor and be potent and efficacious 5-$HT_{2C}$ agonists. In addition, the compounds of the invention were intended to have one or more of the aforementioned advantages, namely low affinity on other serotonergic receptors, and especially the lack of potent agonistic effect (antagonism preferred) on the 5-$HT_{2A}$ and/or 5-$HT_{2B}$ receptors, and additionally one or more of those advantages mentioned under 1.) to 5.), and especially under 1.) (metabolic stability).

The present invention provides compounds which have an affinity for the 5-$HT_{2C}$ receptor, thus allowing the treatment of disorders related to or affected by the 5-$HT_{2C}$ receptor.

## SUMMARY OF THE INVENTION

The present invention relates to (4aR)-2,3,4,4a,5,6-hexahydro-1H-pyrazino[2,1-d][1,5]benzoxazepines and the analogous pyrido[3,2-b][1,4]oxazepine compounds carrying a hydroxyl, methoxy, deuterated methoxy or fluorinated methoxy substituent bound via a linking group, or a cyclic analogue thereof, to a pharmaceutical composition containing such compounds, to their use as modulators, especially agonists or partial agonists, of the 5-$HT_{2C}$ receptor, their use for preparing a medicament for the prevention or treatment of conditions and disorders which respond to the modulation of 5-$HT_{2C}$ receptor, to methods for preventing or treating conditions and disorders which respond to the modulation of 5-$HT_{2C}$ receptor, and processes for preparing such compounds and compositions.

In one aspect, the present invention relates to compounds of the formula (I):

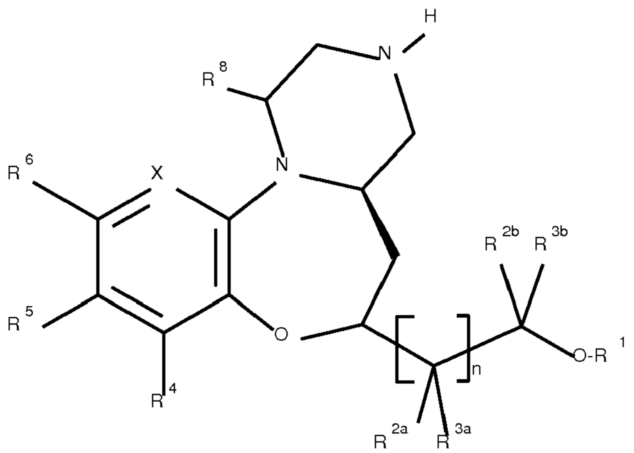

(I)

wherein

X is $CR^7$ or N;

$R^1$ is selected from the group consisting of hydrogen, methyl, deuterated methyl, and fluorinated methyl;

$R^{2a}$, $R^{2b}$, $R^{3a}$ and $R^{3b}$, independently of each other, are selected from the group consisting of hydrogen, $C_1$-$C_4$-alkyl, $C_1$-$C_4$-haloalkyl, $C_3$-$C_6$-cycloalkyl, and $C_3$-$C_6$-halocycloalkyl; or

$R^{2a}$ and $R^{3a}$, together with the carbon atom they are bound to, form a 3-membered saturated carbocyclic ring; or
$R^{2b}$ and $R^{3b}$, together with the carbon atom they are bound to, form a 3-membered saturated carbocyclic ring; or
$R^1$ and $R^{2a}$, if present (i.e. if n is 1), form together a group $-[CH_2]_s-$, where s is 1, 2, 3 or 4; or
$R^1$ and $R^{2b}$ form together a group $-[CH_2]_s-$, where s is 1, 2, 3 or 4;

$R^4$, $R^5$, $R^6$ and $R^7$, independently of each other, are selected from the group consisting of hydrogen, halogen, cyano, $C_1$-$C_4$-alkyl, fluorinated $C_1$-$C_4$-alkyl, $C_1$-$C_4$-hydroxyalkyl, $C_2$-$C_4$-alkenyl, fluorinated $C_2$-$C_4$-alkenyl, $C_2$-$C_4$-alkynyl,

fluorinated $C_2$-$C_4$-alkynyl, $C_3$-$C_6$-cycloalkyl, fluorinated $C_3$-$C_6$-cycloalkyl, $C_1$-$C_4$-alkoxy, fluorinated $C_1$-$C_4$-alkoxy, $C_1$-$C_4$-alkylthio, and fluorinated $C_1$-$C_4$-alkylthio;

$R^8$ is selected from the group consisting of hydrogen, $C_1$-$C_4$-alkyl, fluorinated $C_1$-$C_4$-alkyl, and $C_1$-$C_4$-hydroxyalkyl;

n is 0 or 1;

or an N-oxide, a stereoisomer or a pharmaceutically acceptable salt thereof, or a compound of the general formula (I), wherein at least one of the atoms has been replaced by its stable, non-radioactive isotope.

In another aspect, the invention relates to a pharmaceutical composition comprising a therapeutically effective amount of at least one compound of formula I or an N-oxide, a stereoisomer or a pharmaceutically acceptable salt thereof, in combination with at least one pharmaceutically acceptable carrier and/or auxiliary substance.

In yet another aspect, the invention relates to a compound of formula I or an N-oxide, a stereoisomer or a pharmaceutically acceptable salt thereof for use as a medicament.

In yet another aspect, the invention relates to a compound of formula I or an N-oxide, a stereoisomer or a pharmaceutically acceptable salt thereof for use in the treatment of disorders which responds to the modulation of the 5-$HT_{2C}$ receptor.

In yet another aspect, the invention relates to a compound of formula I or an N-oxide, a stereoisomer or a pharmaceutically acceptable salt thereof for use in the treatment of disorders selected from the group consisting of damage of the central nervous system, disorders of the central nervous system, eating disorders, ocular hypertension, cardiovascular disorders, gastrointestinal disorders and diabetes, and especially from the group consisting of bipolar disorder, depression, atypical depression, mood episodes, adjustment disorders, anxiety, panic disorders, post-traumatic syndrome, psychoses, schizophrenia, cognitive deficits of schizophrenia, memory loss, dementia of aging, Alzheimer’s disease, neuropsychiatric symptoms in Alzheimer’s disease (e.g. aggression), behavioral disorders associated with dementia, social phobia, mental disorders in childhood, attention deficit hyperactivity disorder, organic mental disorders, autism, mutism, disruptive behavior disorder, impulse control disorder, borderline personality disorder, obsessive compulsive disorder, migraine and other conditions associated with cephalic pain or other pain, raised intracranial pressure, seizure disorders, epilepsy, substance use disorders, alcohol abuse, cocaine abuse, tobacco abuse, smoking cessation, sexual dysfunction/erectile dysfunction in males, sexual dysfunction in females, premenstrual syndrome, late luteal phase syndrome, chronic fatigue syndrome, sleep disorders, sleep apnoea, chronic fatigue syndrome, psoriasis, Parkinson’s disease, neuropsychiatric symptoms in Parkinson’s disease (e.g. aggression), Lewy Body dementia, neuropsychiatric symptoms in Lewy Body dementia (e.g. aggression), spinal cord injury, trauma, stroke, pain, bladder dysfunction/urinary incontinence, encephalitis, meningitis, eating disorders, obesity, bulimia, weight loss, anorexia nervosa, ocular hypertension, cardiovascular disorders, gastrointestinal disorders, diabetes insipidus, diabetes mellitus, type I diabetes, type II diabetes, type III diabetes, diabetes secondary to pancreatic diseases, diabetes related to steroid use, diabetes complications, hyperglycemia, and insulin resistance; and to a compound of formula I or an N-oxide, a stereoisomer or a pharmaceutically acceptable salt thereof for the treatment of these disorders.

In yet another aspect, the invention relates to the use of a compound of formula I or of an N-oxide, a stereoisomer or a pharmaceutically acceptable salt thereof for the manufacture of a medicament for the treatment of disorders which respond to the modulation of the 5-$HT_{2C}$ receptor.

In yet another aspect, the invention relates to the use of a compound of formula I or of an N-oxide, a stereoisomer or a pharmaceutically acceptable salt thereof for the manufacture of a medicament for the treatment of disorders selected from the group consisting of damage of the central nervous system, disorders of the central nervous system, eating disorders, ocular hypertension, cardiovascular disorders, gastrointestinal disorders and diabetes, and especially from the group consisting of bipolar disorder, depression, atypical depression, mood episodes, adjustment disorders, anxiety, panic disorders, post-traumatic syndrome, psychoses, schizophrenia, cognitive deficits of schizophrenia, memory loss, dementia of aging, Alzheimer’s disease, neuropsychiatric symptoms in Alzheimer’s disease (e.g. aggression), behavioral disorders associated with dementia, social phobia, mental disorders in childhood, attention deficit hyperactivity disorder, organic mental disorders, autism, mutism, disruptive behavior disorder, impulse control disorder, borderline personality disorder, obsessive compulsive disorder, migraine and other conditions associated with cephalic pain or other pain, raised intracranial pressure, seizure disorders, epilepsy, substance use disorders, alcohol abuse, cocaine abuse, tobacco abuse, smoking cessation, sexual dysfunction/erectile dysfunction in males, sexual dysfunction in females, premenstrual syndrome, late luteal phase syndrome, chronic fatigue syndrome, sleep disorders, sleep apnoea, chronic fatigue syndrome, psoriasis, Parkinson’s disease, neuropsychiatric symptoms in Parkinson’s disease (e.g. aggression), Lewy Body dementia, neuropsychiatric symptoms in Lewy Body dementia (e.g. aggression), spinal cord injury, trauma, stroke, pain, bladder dysfunction/urinary incontinence, encephalitis, meningitis, eating disorders, obesity, bulimia, weight loss, anorexia nervosa, ocular hypertension, cardiovascular disorders, gastrointestinal disorders, diabetes insipidus, diabetes mellitus, type I diabetes, type II diabetes, type III diabetes, diabetes secondary to pancreatic diseases, diabetes related to steroid use, diabetes complications, hyperglycemia, and insulin resistance; and to a compound of formula I or an N-oxide, a stereoisomer or a pharmaceutically acceptable salt thereof for the treatment of these disorders.

In yet another aspect, the invention relates to a method for treating disorders which respond to the modulation of the 5-$HT_{2C}$ receptor, which method comprises administering to a subject in need thereof at least one compound of formula I or an N-oxide, a stereoisomer or a pharmaceutically acceptable salt thereof.

In yet another aspect, the invention relates to a method for treating disorders selected from the group consisting of damage of the central nervous system, disorders of the central nervous system, eating disorders, ocular hypertension, cardiovascular disorders, gastrointestinal disorders and diabetes, and especially from the group consisting of bipolar disorder, depression, atypical depression, mood episodes, adjustment disorders, anxiety, panic disorders, post-traumatic syndrome, psychoses, schizophrenia, cognitive deficits of schizophrenia, memory loss, dementia of aging, Alzheimer's disease, neuropsychiatric symptoms in Alzheimer's disease (e.g. aggression), behavioral disorders associated with dementia, social phobia, mental disorders in childhood, attention deficit hyperactivity disorder, organic mental disorders, autism, mutism, disruptive behavior disorder, impulse control disorder, borderline personality disorder, obsessive compulsive disorder, migraine and other conditions associated with cephalic pain or other pain, raised intracranial pressure, seizure disorders, epilepsy, substance use disorders, alcohol abuse, cocaine abuse, tobacco abuse, smoking cessation, sexual dysfunction/erectile dysfunction in males, sexual dysfunction in females, premenstrual syndrome, late luteal phase syndrome, chronic fatigue syndrome, sleep disorders, sleep apnoea, chronic fatigue syndrome, psoriasis, Parkinson's disease, neuropsychiatric symptoms in Parkinson's disease (e.g. aggression), Lewy Body dementia, neuropsychiatric symptoms in Lewy Body dementia (e.g. aggression), spinal cord injury, trauma, stroke, pain, bladder dysfunction/urinary incontinence, encephalitis, meningitis, eating disorders, obesity, bulimia, weight loss, anorexia nervosa, ocular hypertension, cardiovascular disorders, gastrointestinal disorders, diabetes insipidus, diabetes mellitus, type I diabetes, type II diabetes, type III diabetes, diabetes secondary to pancreatic diseases, diabetes related to steroid use, diabetes complications, hyperglycemia, and insulin resistance, which method comprises administering to a subject in need thereof at least one compound of formula I or an N-oxide, a stereoisomer or a pharmaceutically acceptable salt thereof.

## DETAILED DESCRIPTION

The compounds of the formula I may exist in different spatial arrangements. Apart from the compulsory stereoform shown in formula I (R configuration at 4a position), the compounds possess at least one more center of chirality, which is at the 6-position (i.e. at the carbon ring atom carrying the $[C(R^{2a})(R^{3a})]_n$-$C(R^{2b})(R^{3b})$-$OR^1$ substituent). Moreover, if $R^{2a}$ and $R^{3a}$ or if $R^{2b}$ and $R^{3b}$ are different, the carbon atom(s) carrying these radicals is/are also a center of chirality. The present invention contemplates the possible use of enantiomeric mixtures, in particular racemates or diastereomeric mixtures, as well as the respective essentially pure enantiomers and diastereomers of the compounds of formula I and/or their salts.

It is likewise possible to use physiologically tolerated salts of the compounds of the formula I, especially acid addition salts with physiologically tolerated acids. Examples of suitable physiologically tolerated organic and inorganic acids are hydrochloric acid, hydrobromic acid, phosphoric acid, sulfuric acid, acetic acid, trifluoroacetic acid, $C_1$-$C_4$-alkylsulfonic acids, such as methanesulfonic acid, aromatic sulfonic acids, such as benzenesulfonic acid and toluenesulfonic acid, oxalic acid, maleic acid, fumaric acid, lactic acid, tartaric acid, adipic acid and benzoic acid. Other utilizable acids are described in Fortschritte der Arzneimittelforschung [Advances in drug research], Volume 10, pages 224 et seq., Birkhäuser Verlag, Basel and Stuttgart, 1966.

The organic moieties mentioned in the above definitions of the variables are, like the term halogen, collective terms for individual listings of the individual group members. The prefix $C_n$-$C_m$ indicates in each case the possible number of carbon atoms in the group.

The term "halogen" denotes in each case fluorine, bromine, chlorine or iodine. In one aspect, the halogen may be fluorine, chlorine or bromine.

Deuterated methyl is methyl in which at least one hydrogen atom is replaced by a deuterium atom. Examples are $CDH_2$, $CD_2H$ and $CD_3$.

The term "alkyl" as used herein and in the alkyl moieties of alkoxy and the like refers to saturated straight-chain or branched hydrocarbon radicals having 1 to 2 ("$C_1$-$C_2$-alkyl"), 1 to 3 ("$C_1$-$C_3$-alkyl") or 1 to 4 ("$C_1$-$C_4$-alkyl) carbon atoms. $C_1$-$C_2$-Alkyl is methyl or ethyl. $C_1$-$C_3$-Alkyl is additionally propyl or isopropyl. $C_1$-$C_4$-Alkyl is additionally butyl, 1-methylpropyl (sec-butyl), 2-methylpropyl (isobutyl) or 1,1-dimethylethyl (tert-butyl).

The term "fluorinated methyl" as used herein refers to a methyl group where some or all of the hydrogen atoms in this group are replaced by fluorine atoms. Examples are fluoromethyl ($CH_2F$), difluoromethyl ($CHF_2$) and trifluoromethyl ($CF_3$).

The term "fluorinated alkyl" as used herein refers to straight-chain or branched alkyl groups having 1 to 2 ("fluorinated $C_1$-$C_2$-alkyl"), 1 to 3 ("fluorinated $C_1$-$C_3$-alkyl") or 1 to 4 ("fluorinated $C_1$-$C_4$-alkyl) carbon atoms (as mentioned above), where some or all of the hydrogen atoms in these groups are replaced by fluorine atoms. Fluorinated $C_1$-$C_2$-alkyl is an alkyl group having 1 or 2 carbon atoms (as mentioned above), where at least one of the hydrogen atoms, e.g. 1,

2, 3, 4 or 5 hydrogen atoms in these groups are replaced by fluorine atoms, such as fluoromethyl, difluoromethyl, trifluoromethyl, 1-fluoroethyl, (R)-1-fluoroethyl, (S)-1-fluoroethyl, 2-fluoroethyl, 2,2-difluoroethyl, 2,2,2-trifluoroethyl, or pentafluoroethyl. Fluorinated $C_1$-$C_3$-alkyl is a straight-chain or branched alkyl group having 1 to 3 carbon atoms (as mentioned above), where at least one of the hydrogen atoms, e.g. 1, 2, 3, 4, 5, 6 or 7 hydrogen atoms in these groups are replaced by fluorine atoms. Examples are, apart those listed above for fluorinated $C_1$-$C_2$-alkyl, 1-fluoropropyl, (R)-1-fluoropropyl, (S)-1-fluoropropyl, 2-fluoropropyl, (R)-2-fluoropropyl, (S)-2-fluoropropyl, 3-fluoropropyl, 1,1-difluoropropyl, 2,2-difluoropropyl, 1,2-difluoropropyl, 2,3-difluoropropyl, 1,3-difluoropropyl, 3,3-difluoropropyl, 1,1,2-trifluoropropyl, 1,2,2-trifluoropropyl, 1,2,3-trifluoropropyl, 2,2,3-trifluoropropyl, 3,3,3-trifluoropropyl, 1,1,1-trifluoroprop-2-yl, 2-fluoro-1-methylethyl, (R)-2-fluoro-1-methylethyl, (S)-2-fluoro-1-methylethyl, 2,2-difluoro-1-methylethyl, (R)-2,2-difluoro-1-methylethyl, (S)-2,2-difluoro-1-methylethyl, 1,2-difluoro-1-methylethyl, (R)-1,2-difluoro-1-methylethyl, (S)-1,2-difluoro-1-methylethyl, 2,2,2-trifluoro-1-methylethyl, (R)-2,2,2-trifluoro-1-methylethyl, (S)-2,2,2-trifluoro-1-methylethyl, 2-fluoro-1-(fluoromethyl)ethyl, 1-(difluoromethyl)-2,2-difluoroethyl, 1-(trifluoromethyl)-2,2,2-trifluoroethyl or 1-(trifluoromethyl)-1,2,2,2-tetrafluoroethyl. Fluorinated $C_1$-$C_4$-alkyl is a straight-chain or branched alkyl group having 1 to 4 carbon atoms (as mentioned above), where at least one of the hydrogen atoms, e.g. 1, 2, 3, 4, 5, 6, 7, 8 or 9 hydrogen atoms in these groups are replaced by fluorine atoms. Examples are, apart those listed above for fluorinated $C_1$-$C_3$-alkyl, 1-fluorobutyl, (R)-1-fluorobutyl, (S)-1-fluorobutyl, 2-fluorobutyl, (R)-2-fluorobutyl, (S)-2-fluorobutyl, 3-fluorobutyl, (R)-3-fluorobutyl, (S)-3-fluorobutyl, 4-fluorobutyl, 1,1-difluorobutyl, 2,2-difluorobutyl, 3,3-difluorobutyl, 4,4-difluorobutyl, 4,4,4-trifluorobutyl and the like.

The term "haloalkyl" as used herein refers to straight-chain or branched alkyl groups having 1 to 2 ("$C_1$-$C_2$-haloalkyl"), 1 to 3 ("$C_1$-$C_3$-haloalkyl") or 1 to 4 ("$C_1$-$C_4$-haloalkyl) carbon atoms (as mentioned above), where some or all of the hydrogen atoms in these groups are replaced by halogen atoms. $C_1$-$C_2$-Haloalkyl is an alkyl group having 1 or 2 carbon atoms (as mentioned above), where at least one of the hydrogen atoms, e.g. 1, 2, 3, 4 or 5 hydrogen atoms in these groups are replaced by halogen atoms. Examples are, apart from those mentioned above for fluorinated $C_1$-$C_2$-alkyl, chloromethyl, bromomethyl, dichloromethyl, trichloromethyl, chlorofluoromethyl, dichlorofluoromethyl, chlorodifluoromethyl, 1-chloroethyl, 1-bromoethyl, 2-chloro-2-fluoroethyl, 2-chloro-2,2-difluoroethyl, 2,2-dichloro-2-fluoroethyl or 2,2,2-trichloroethyl. Examples for $C_1$-$C_3$-haloalkyl are, apart those listed above for $C_1$-$C_2$-haloalkyl and for fluorinated $C_1$-$C_3$-alkyl, 3-chloropropyl and the like. Examples for $C_1$-$C_4$-haloalkyl are, apart those mentioned above for $C_1$-$C_3$-haloalkyl and for fluorinated $C_1$-$C_4$-alkyl, 4-chlorobutyl and the like.

The term "hydroxyalkyl" as used herein refers to straight-chain or branched alkyl groups having 1 to 2 ("$C_1$-$C_2$-hydroxyalkyl"), 1 to 3 ("$C_1$-$C_3$-hydroxyalkyl") or 1 to 4 ("$C_1$-$C_4$-hydroxyalkyl) carbon atoms (as mentioned above), where one hydrogen atom in these groups is replaced by a hydroxyl group. Examples for $C_1$-$C_2$-hydroxyalkyl are hydroxymethyl, 1-hydroxyethyl and 2-hydroxyethyl. Examples for $C_1$-$C_3$-hydroxyalkyl are, apart from those mentioned above for $C_1$-$C_2$-hydroxyalkyl, 1-hydroxy-1-propyl, 2-hydroxy-1-propyl, 1-hydroxy-2-propyl and 2-hydroxy2-propyl. Examples for $C_1$-$C_4$-hydroxyalkyl are, apart from those mentioned above for $C_1$-$C_3$-hydroxyalkyl, 1-hydroxy-1-butyl, 2-hydroxy-1-butyl, 3-hydroxy-1-butyl, 4-hydroxy-1-butyl, 1-hydroxy-2-butyl, 2-hydroxy-2-butyl, 3-hydroxy-2-butyl, 4-hydroxy-2-butyl and the like.

The term "alkenyl" as used herein refers to monounsaturated straight-chain or branched hydrocarbon radicals having 2 to 3 ("$C_2$-$C_3$-alkenyl") or 2 to 4 ("$C_2$-$C_4$-alkenyl") carbon atoms and a double bond in any position. Examples for $C_2$-$C_3$-alkenyl are ethenyl, prop-1-en-1-yl, prop-2-en-1-yl or 1-methylethenyl. Examples for $C_2$-$C_4$-alkenyl are ethenyl, prop-1-en-1-yl, prop-2-en-1-yl, 1-methylethenyl, but-1-en-1-yl, but-2-en-1-yl, but-3-en-1-yl, but-1-en-2-yl, but-1-en-3-yl, but-2-en-2-yl, 2-methyl-prop-1-en-1-yl or 2-methyl-prop-2-en-1-yl.

The term "fluorinated alkenyl" as used herein refers to unsaturated straight-chain or branched hydrocarbon radicals having 2 to 3 ("fluorinated $C_2$-$C_3$-alkenyl") or 2 to 4 ("fluorinated $C_2$-$C_4$-alkenyl") carbon atoms and a double bond in any position (as mentioned above), where some or all of the hydrogen atoms in these groups are replaced by fluorine atoms, such as, fluorovinyl, fluoroallyl and the like.

The term "alkynyl" as used herein refers to straight-chain or branched hydrocarbon groups having 2 to 3 ("$C_2$-$C_3$-alkynyl") or 2 to 4 ("$C_2$-$C_4$-alkynyl") carbon atoms and one triple bond in any position, Examples for $C_2$-$C_3$-alkynyl are ethynyl, 1-propynyl or 2-propynyl (propargyl). Examples for $C_2$-$C_4$-alkynyl are ethynyl, 1-propynyl, 2-propynyl (propargyl), 1-butynyl, 2-butynyl, 3-butynyl, 1-methyl-2-propynyl and the like. $C_3$-alkynyl is 1-propynyl or 2-propynyl (propargyl)

The term "fluorinated alkynyl" as used herein refers to unsaturated straight-chain or branched hydrocarbon radicals having 2 to 3 ("fluorinated $C_2$-$C_3$-alkynyl") or 2 to 4 ("fluorinated $C_2$-$C_4$-alkynyl") carbon atoms and one triple bond in any position (as mentioned above), where some or all of the hydrogen atoms in these groups are replaced by fluorine atoms.

The term "cycloalkyl" as used herein refers to monocyclic saturated hydrocarbon radicals having 3 to 6 carbon atoms ("$C_3$-$C_6$-cycloalkyl"). Examples of $C_3$-$C_6$-cycloalkyl are cyclopropyl, cyclobutyl, cyclopentyl and cyclohexyl.

The term "fluorinated cycloalkyl" as used herein refers to monocyclic saturated hydrocarbon groups having 3 to 6 ("fluorinated $C_3$-$C_6$-cycloalkyl") carbon ring members (as mentioned above) in which some or all of the hydrogen atoms are replaced by fluorine atoms. Examples include 1-fluorocyclopropyl, 2-fluorocyclopropyl, (S)- and (R)-2,2-difluorocyclopropyl, 1,2-difluorocyclopropyl, 2,3-difluorocyclopropyl, pentafluorocyclopropyl, 1-fluorocyclobutyl, 2-fluorocyclobutyl, 3-fluorocyclobutyl, 2,2-difluorocyclobutyl, 3,3-difluorocyclobutyl, 1,2-difluorocyclobutyl, 1,3-difluorocyclobutyl, 2,3-difluorocyclobutyl, 2,4-difluorocyclobutyl, 1,2,2-trifluorocyclobutyl, 1-fluorocyclopentyl, 2-fluorocyclopentyl, 1,2-difluorocyclopentyl, 1,3-difluorocyclopentyl, 2,2-difluorocyclopentyl, 2,3-difluorocyclopentyl, 2,4-difluorocyclopentyl, 2,5-difluorocyclopentyl, 3,3-difluorocyclopentyl, 3,4-difluorocyclopentyl, 1-fluorocyclohexyl, 2-fluorocyclohexyl, 3-fluorocyclohexyl, 4-fluorocyclohexyl, 1,2-difluorocyclohexyl, 1,3-difluorocyclohexyl, 1,4-difluorocyclohexyl, 2,2-difluorocyclohexyl, 2,3-difluorocyclohexyl, 2,4-difluorocyclohexyl, 2,5-difluorocyclohexyl, 2,6-difluorocyclohexyl, 3,3-difluorocyclohexyl, 3,4-difluorocyclohexyl, 3,5-difluorocyclohexyl, 4,4-difluorocyclohexyl, and the like.

The term " halocycloalkyl" as used herein refers to monocyclic saturated hydrocarbon groups having 3 to 6 ("$C_3$-$C_6$-halocycloalkyl") carbon ring members (as mentioned above) in which some or all of the hydrogen atoms are replaced by halogen atoms. Examples are, apart those mentioned above for fluorinated $C_3$-$C_6$-cycloalkyl, 1-chlorocyclopropyl, 2-chlorocyclopropyl, 2,3-dichlorocyclopropyl, 2-chloro-1-fluorocyclopropyl, 3-chloro-2-fluorocyclopropyl, 2-bromocyclopropyl, 1-chlorocyclobutyl, 2-chlorocyclobutyl, 3-chlorocyclobutyl, 2,2-dichlorocyclobutyl, 2,3-dichlorocyclobutyl, 3,3-dichlorocyclobutyl, 2-bromocyclobutyl, 3-bromocyclobutyl, 1-chlorocyclopentyl, 2-chlorocyclopentyl, 3-chlorocyclopentyl, 1-chlorocyclohexyl, 2-chlorocyclohexyl, 3-chlorocyclohexyl, 4-chlorocyclohexyl, and the like.

A a 3-membered saturated carbocyclic ring formed by $R^{2a}$ and $R^{3a}$ or $R^{2b}$ and $R^{3b}$ together with the carbon atom they are bound to is a (spiro-bound) cyclopropan-1,1-diyl ring.

The term "$C_1$-$C_2$-alkoxy" is a $C_1$-$C_2$-alkyl group, as defined above, attached via an oxygen atom. The term "$C_1$-$C_3$-alkoxy" is a $C_1$-$C_3$-alkyl group, as defined above, attached via an oxygen atom. The term "$C_1$-$C_4$-alkoxy" is a $C_1$-$C_4$-alkyl group, as defined above, attached via an oxygen atom. $C_1$-$C_2$-Alkoxy is methoxy or ethoxy. $C_1$-$C_3$-Alkoxy is additionally, for example, n-propoxy and 1-methylethoxy (isopropoxy). $C_1$-$C_4$-Alkoxy is additionally, for example, butoxy, 1-methylpropoxy (sec-butoxy), 2-methylpropoxy (isobutoxy) or 1,1-dimethylethoxy (tert-butoxy).

The term "fluorinated $C_1$-$C_2$-alkoxy" is a fluorinated $C_1$-$C_2$-alkyl group, as defined above, attached via an oxygen atom. The term "fluorinated $C_1$-$C_3$-alkoxy" is a fluorinated $C_1$-$C_3$-alkyl group, as defined above, attached via an oxygen atom. The term "fluorinated $C_1$-$C_4$-haloalkoxy" is a fluorinated $C_1$-$C_4$-alkyl group, as defined above, attached via an oxygen atom. Fluorinated $C_1$-$C_2$-alkoxy is, for example, OCH2F, $OCHF_2$, $OCF_3$, 1-fluoroethoxy, (R)-1-fluoroethoxy, (S)-1-fluoroethoxy, 2-fluoroethoxy, 1,1-difluoroethoxy, 1,2-difluoroethoxy, 2,2-difluoroethoxy, 1,1,2-trifluoroethoxy, 1,2,2-trifluoroethoxy, 2,2,2-trifluoroethoxy, 1,1,2,2-tetrafluoroethoxy or $OC_2F_5$. Fluorinated $C_1$-$C_3$-alkoxy is additionally, for example, 1-fluoropropoxy, (R)-1-fluoropropoxy, (S)-1-fluoropropoxy, 2-fluoropropoxy, (R)-2-fluoropropoxy, (S)-2-fluoropropoxy, 3-fluoropropoxy, 1,1-difluoropropoxy, 2,2-difluoropropoxy, 2,3-difluoropropoxy, 3,3-difluoropropoxy, 3,3,3-trifluoropropoxy, (R)-2-fluoro-1-methylethoxy, (S)-2-fluoro-1-methylethoxy, (R)-2,2-difluoro-1-methylethoxy, (S)-2,2-difluoro-1-methylethoxy, (R)-1,2-difluoro-1-methylethoxy, (S)-1,2-difluoro-1-methylethoxy, (R)-2,2,2-trifluoro-1-methylethoxy, (S)-2,2,2-trifluoro-1-methylethoxy, 2-fluoro-1-(fluoromethyl)ethoxy, 1-(difluoromethyl)-2,2-difluoroethoxy, $OCH_2$-$C_2F_5$, $OCF_2$-$C_2F_5$ or 1-($CH_2F$)-2-fluoroethoxy. Fluorinated $C_1$-$C_4$-alkoxy is additionally, for example, 1-fluorobutoxy, (R)-1-fluorobutoxy, (S)-1-fluorobutoxy, 2-fluorobutoxy, 3-fluorobutoxy, 4-fluorobutoxy, 1,1-difluorobutoxy, 2,2-difluorobutoxy, 3,3-difluorobutoxy, 4,4-difluorobutoxy, 4,4,4-trifluorobutoxy or nonafluorobutoxy.

Fluorinated methoxy is $OCH_2F$, $OCHF_2$ or $OCF_3$.

The term "$C_1$-$C_2$-alkylthio" is a $C_1$-$C_2$-alkyl group, as defined above, attached via a sulfur atom. The term "$C_1$-$C_3$-alkylthio" refers to a $C_1$-$C_3$-alkyl group, as defined above, attached via a sulfur atom. The term "$C_1$-$C_4$-alkylthio" is a $C_1$-$C_4$-alkyl group, as defined above, attached via a sulfur atom. $C_1$-$C_2$-Alkylthio is methylthio or ethylthio. $C_1$-$C_3$-Alkylthio is additionally, for example, n-propylthio or 1-methylethylthio (isopropylthio). $C_1$-$C_4$-Alkylthio is additionally, for example, butylthio, 1-methylpropylthio (sec-butylthio), 2-methylpropylthio (isobutylthio) or 1,1-dimethylethylthio (tert-butylthio).

The term "fluorinated $C_1$-$C_2$-alkylthio" refers to a fluorinated $C_1$-$C_2$-alkyl group, as defined above, attached via a sulfur atom. The term "fluorinated $C_1$-$C_3$-alkylthio" refers to a fluorinated $C_1$-$C_3$-alkyl group, as defined above, attached via a sulfur atom. The term "fluorinated $C_1$-$C_4$-alkylthio" refers to a fluorinated $C_1$-$C_4$-alkyl group, as defined above, attached via a sulfur atom. Fluorinated $C_1$-$C_2$-alkylthio refers to, for example, $SCH_2F$, $SCHF_2$, $SCF_3$, 2-fluoroethylthio, 2,2-difluoroethylthio, 2,2,2-trifluoroethylthio, or $SC_2F_5$. Fluorinated $C_1$-$C_3$-alkylthio may additionally, for example, include 2-fluoropropylthio, 3-fluoropropylthio, 2,2-difluoropropylthio, 2,3-difluoropropylthio, 3,3,3-trifluoropropylthio, $SCH_2$-$C_2F_5$, $SCF_2$-$C_2F_5$ or 1-($CH_2F$)-2-fluoroethylthio. Fluorinated $C_1$-$C_4$-alkylthio may additionally, for example, include 4-fluorobutylthio or nonafluorobutylthio.

Fluorinated methylthio is $SCH_2F$, $SCHF_2$ or $SCF_3$.

The remarks made above and in the following with respect to preferred aspects of the invention, e.g. to preferred meanings of the variables X, $R^1$, $R^{2a}$, $R^{2b}$, $R^{3a}$, $R^{3b}$, $R^4$, $R^5$, $R^6$, $R^7$, $R^8$ and n of compounds I, to preferred compounds I and to preferred embodiments of the methods or the use according to the invention, apply in each case on their own or in particular to combinations thereof.

In a preferred embodiment (embodiment 1) n is 0 and $R^1$ is selected from the group consisting of hydrogen, methyl, deuterated methyl (especially $CD_3$), and fluorinated methyl. In a more preferred embodiment (embodiment 1.1)

n is 0 and $R^1$ is selected from the group consisting of hydrogen, methyl, and deuterated methyl (especially $CD_3$). Particularly (embodiment 1.1.1) n is 0 and $R^1$ is selected from the group consisting of methyl and deuterated methyl (especially $CD_3$). Specifically (embodiment 1.1.1.1) n is 0 and $R^1$ is methyl.

In a preferred embodiment (embodiment 2) n is 0 and $R^{2b}$ and $R^{3b}$, independently of each other, are selected from the group consisting of hydrogen, methyl, $CHF_2$, $CF_3$, and cyclopropyl. Particularly (embodiment 2.1), n is 0 and $R^{2b}$ and $R^{3b}$ are both hydrogen.

In a particular embodiment (embodiment 2.2), n is 0 and $R^{2b}$ and $R^{3b}$, independently of each other, are selected from the group consisting of hydrogen, methyl, $CHF_2$, $CF_3$, and cyclopropyl, and $R^1$ is as defined in one of embodiments 1, 1.1, 1.1.1 or 1.1.1.1. Specifically (embodiment 2.2.1), n is 0, $R^{2b}$ and $R^{3b}$ are both hydrogen and $R^1$ is as defined in one of embodiments 1, 1.1, 1.1.1 or 1.1.1.1.

In an alternatively preferred embodiment (embodiment 3) n is 0 and $R^1$ and $R^{2b}$ form together a group $-[CH_2]_s-$, where s is 1, 2, 3 or 4. In particular (embodiment 3.1) n is 0, $R^1$ and $R^{2b}$ form together a group $-[CH_2]_s-$, where s is 1, 2, 3 or 4 and $R^{3b}$ is selected from the group consisting of hydrogen, methyl, $CHF_2$, $CF_3$, and cyclopropyl. In a more preferred embodiment (embodiment 3.1.1), n is 0, $R^1$ and $R^{2b}$ form together a group $-[CH_2]_s-$, where s is 2 (so that the substituent in 6-position of the tricyclic scaffold is oxetan-2-yl substituted in 2-position by $R^{3b}$), and $R^{3b}$ is selected from the group consisting of hydrogen, methyl, $CHF_2$, $CF_3$, and cyclopropyl. In a particular embodiment (embodiment 3.2), n is 0, $R^1$ and $R^{2b}$ form together a group $-[CH_2]_s-$, where s is 1, 2, 3 or 4, and $R^{3b}$ is hydrogen. Specifically (embodiment 3.2.1), n is 0, $R^1$ and $R^{2b}$ form together a group $-[CH_2]_s-$, where s is 2 (so that the substituent in 6-position of the tricyclic scaffold is oxetan-2-yl substituted in 2-position by $R^{3b}$), and $R^{3b}$ is hydrogen.

In another alternatively preferred embodiment (embodiment 4) n is 1 and $R^1$ and $R^{2a}$ form together a group $-[CH_2]_s-$, where s is 1, 2, 3 or 4. In particular (embodiment 4.1) n is 1, $R^1$ and $R^{2a}$ form together a group $-[CH_2]_s-$, where s is 1, 2, 3 or 4, and $R^{2b}$, and $R^{3a}$ and $R^{3b}$, independently of each other, are selected from the group consisting of hydrogen, methyl, $CHF_2$, $CF_3$, and cyclopropyl. In a more preferred embodiment (embodiment 4.1.1), n is 1, $R^1$ and $R^{2a}$ form together a group $-CH_2-$ (so that the substituent in 6-position of the tricyclic scaffold is oxetan-3-yl substituted in 3-position by $R^{3a}$ and in 2,2-position by $R^{2b}$ and $R^{3b}$), and $R^{2b}$, $R^{3a}$ and $R^{3b}$, independently of each other, are selected from the group consisting of hydrogen, methyl, $CHF_2$, $CF_3$, and cyclopropyl. In a particular embodiment (embodiment 4.2), n is 1, $R^1$ and $R^{2a}$ form together a group $-[CH_2]_s-$, where s is 1, 2, 3 or 4, and $R^{2b}$, $R^{3a}$ and $R^{3b}$ are hydrogen. Specifically (embodiment 4.2.1), n is 1, $R^1$ and $R^{2a}$ form together a group $-CH_2-$ (so that the substituent in 6-position of the tricyclic scaffold is oxetan-3-yl substituted in 3-position by $R^{3a}$ and in 2,2-position by $R^{2b}$ and $R^{3b}$), and $R^{2b}$, $R^{3a}$ and $R^{3b}$ are hydrogen.

In a specific embodiment (embodiment 234) the moiety $-[C(R^{2a})(R^{3a})]_n-C(R^{2b})(R^{3b})-OR^1$ stands for $-CH_2-OR^1$, oxetan-2-yl or oxetan-3-yl, where $R^1$ has one of the above general meanings or is as defined in one of embodiments 1, 1.1, 1.1.1 or 1.1.1.1. Very specifically (embodiment 234.1), the moiety $-[C(R^{2a})(R^{3a})]_n-C(R^{2b})(R^{3b})-OR^1$ stands for $-CH_2-OR^1$, where $R^1$ has one of the above general meanings or is as defined in one of embodiments 1, 1.1, 1.1.1 or 1.1.1.1.

In a preferred embodiment (embodiment 5)

$R^4$ is selected from the group consisting of hydrogen, halogen, cyano, $C_1$-$C_3$-alkyl, fluorinated $C_1$-$C_3$-alkyl, $C_1$-$C_3$-hydroxyalkyl, $C_2$-$C_3$-alkynyl, cyclopropyl, $C_1$-$C_3$-alkoxy, fluorinated $C_1$-$C_3$-alkoxy, $C_1$-$C_3$-alkylthio, and fluorinated $C_1$-$C_3$-alkylthio;

$R^5$ is selected from the group consisting of hydrogen, halogen, cyano, $C_1$-$C_3$-alkyl, fluorinated $C_1$-$C_3$-alkyl, $C_1$-$C_3$-hydroxyalkyl, $C_2$-$C_3$-alkynyl, cyclopropyl, $C_1$-$C_3$-alkoxy, fluorinated $C_1$-$C_3$-alkoxy, $C_1$-$C_3$-alkylthio, and fluorinated $C_1$-$C_3$-alkylthio;

$R^6$ is selected from the group consisting of hydrogen, halogen, cyano, $C_1$-$C_3$-alkyl, fluorinated $C_1$-$C_3$-alkyl, $C_1$-$C_3$-hydroxyalkyl, $C_2$-$C_3$-alkynyl, cyclopropyl, $C_1$-$C_3$-alkoxy, fluorinated $C_1$-$C_3$-alkoxy, $C_1$-$C_3$-alkylthio, and fluorinated $C_1$-$C_3$-alkylthio; and

$R^7$ is selected from the group consisting of hydrogen, halogen, cyano, $C_1$-$C_3$-alkyl, fluorinated $C_1$-$C_3$-alkyl, $C_1$-$C_3$-hydroxyalkyl, $C_2$-$C_3$-alkynyl, cyclopropyl, $C_1$-$C_3$-alkoxy, fluorinated $C_1$-$C_3$-alkoxy, $C_1$-$C_3$-alkylthio, and fluorinated $C_1$-$C_3$-alkylthio.

In a particular embodiment (embodiment 5.1)

$R^4$ is selected from the group consisting of hydrogen, fluorine, chlorine, cyano, $C_1$-$C_2$-alkyl, fluorinated $C_1$-$C_2$-alkyl, $C_2$-$C_3$-alkynyl, cyclopropyl, $C_1$-$C_2$-alkoxy, fluorinated $C_1$-$C_2$-alkoxy, $C_1$-$C_2$-alkylthio, and fluorinated $C_1$-$C_2$-alkylthio;

$R^5$ is selected from the group consisting of hydrogen, halogen, $C_1$-$C_2$-alkyl, and $C_1$-$C_2$-alkoxy;

$R^6$ is selected from the group consisting of hydrogen, fluorine, chlorine, $C_1$-$C_2$-alkyl, fluorinated $C_1$-$C_2$-alkyl, $C_2$-$C_3$-alkynyl, $C_1$-$C_2$-alkoxy, and fluorinated $C_1$-$C_2$-alkoxy; and

$R^7$ is selected from the group consisting of hydrogen, fluorine, chlorine, $C_1$-$C_2$-alkyl, fluorinated $C_1$-$C_2$-alkyl, and

$C_1$-$C_2$-hydroxyalkyl.

In a more particular embodiment (embodiment 5.1.1)

$R^4$ is selected from the group consisting of hydrogen, fluorine, chlorine, cyano, $C_1$-$C_2$-alkyl, fluorinated $C_1$-$C_2$-alkyl, $C_2$-$C_3$-alkynyl, cyclopropyl, $C_1$-$C_2$-alkoxy, and fluorinated $C_1$-$C_2$-alkoxy;

$R^5$ is selected from the group consisting of hydrogen, fluorine, chlorine, $C_1$-$C_2$-alkyl, and $C_1$-$C_2$-alkoxy;

$R^6$ is selected from the group consisting of hydrogen, fluorine, chlorine, $C_1$-$C_2$-alkyl, fluorinated $C_1$-$C_2$-alkyl, $C_2$-$C_3$-alkynyl, $C_1$-$C_2$-alkoxy, and fluorinated $C_1$-$C_2$-alkoxy; and

$R^7$ is selected from the group consisting of hydrogen, fluorine, and chlorine.

In another more particular embodiment (embodiment 5.1.2)

$R^4$ is selected from the group consisting of fluorine, chlorine, cyano, methyl, fluorinated methyl, $C_2$-$C_3$-alkynyl, cyclopropyl, methoxy, fluorinated methoxy, methylthio, and fluorinated methylthio;

$R^5$ is selected from the group consisting of hydrogen, halogen, and $C_1$-$C_2$-alkyl;

$R^6$ is selected from the group consisting of fluorine, chlorine, methyl, fluorinated methyl, methoxy, and fluorinated methoxy; and

$R^7$ is selected from the group consisting of hydrogen, fluorine, chlorine, $C_1$-$C_2$-alkyl, fluorinated $C_1$-$C_2$-alkyl, and $C_1$-$C_2$-hydroxyalkyl.

Specifically (embodiment 5.1.1.1) $R^4$ is selected from the group consisting of fluorine, chlorine, cyano, methyl, $CHF_2$, $CF_3$, and $C_3$-alkynyl. In this case, $R^5$, $R^6$ and $R^7$ are specifically as defined in embodiment 5.1 or 5.1.1 or 5.1.2.

In a particular embodiment (embodiment 5.2) $R^4$, $R^5$, $R^6$ and $R^7$ are as defined in embodiment 5 and $R^1$, $R^{2a}$, $R^{2b}$, $R^{3a}$ and $R^{3b}$ are as defined in embodiments 1, 1.1, 1.1.1, 1.1.1.1, 2, 2.1, 2.2, 2.2.1, 3, 3.1, 3.1.1, 3.2, 3.2.1, 4, 4.1, 4.1.1, 4.2, 4.2.1, 234 or 234.1. In a more particular embodiment (embodiment 5.2.1) $R^4$, $R^5$, $R^6$ and $R^7$ are as defined in embodiment 5.1 and $R^1$, n, $R^{2a}$, $R^{2b}$, $R^{3a}$ and $R^{3b}$ are as defined in embodiments 1, 1.1, 1.1.1, 1.1.1.1, 2, 2.1, 2.2, 2.2.1, 3, 3.1, 3.1.1, 3.2, 3.2.1, 4, 4.1, 4.1.1, 4.2, 4.2.1, 234 or 234.1. In an even more particular embodiment (embodiment 5.2.1.1) $R^4$, $R^5$, $R^6$ and $R^7$ are as defined in embodiment 5.1.1 and $R^1$, n, $R^{2a}$, $R^{2b}$, $R^{3a}$ and $R^{3b}$ are as defined in embodiments 1, 1.1, 1.1.1, 1.1.1.1, 2, 2.1, 2.2, 2.2.1, 3, 3.1, 3.1.1, 3.2, 3.2.1, 4, 4.1, 4.1.1, 4.2, 4.2.1, 234 or 234.1. In an alternative even more particular embodiment (embodiment 5.2.1.2) $R^4$, $R^5$, $R^6$ and $R^7$ are as defined in embodiment 5.1.2 and $R^1$, n, $R^{2a}$, $R^{2b}$, $R^{3a}$ and $R^{3b}$ are as defined in embodiments 1, 1.1, 1.1.1, 1.1.1.1, 2, 2.1, 2.2, 2.2.1, 3, 3.1, 3.1.1, 3.2, 3.2.1, 4, 4.1, 4.1.1, 4.2, 4.2.1, 234 or 234.1. In a specific embodiment (embodiment 5.2.2.1) $R^4$ is as defined in embodiment 5.1.1.1, $R^5$, $R^6$ and $R^7$ are as defined in embodiment 5.1 or 5.1.1 or 5.1.2 and $R^1$, n, $R^{2a}$, $R^{2b}$, $R^{3a}$ and $R^{3b}$ are as defined in embodiments 1, 1.1, 1.1.1, 1.1.1.1, 2, 2.1, 2.2, 2.2.1, 3, 3.1, 3.1.1, 3.2, 3.2.1, 4, 4.1, 4.1.1, 4.2, 4.2.1, 234 or 234.1.

In a particular embodiment (embodiment 6) $R^8$ is hydrogen. In particular (embodiment 6.1), $R^8$ is hydrogen and $R^1$, n, $R^{2a}$, $R^{2b}$, $R^{3a}$, $R^{3b}$, $R^4$, $R^5$, $R^6$ and $R^7$ are as defined in embodiments 1, 1.1, 1.1.1, 1.1.1.1, 2, 2.1, 2.2, 2.2.1, 3, 3.1, 3.1.1, 3.2, 3.2.1, 4, 4.1, 4.1.1, 4.2, 4.2.1, 234, 234.1, 5, 5.1, 5.1.1, 5.1.2, 5.1.1.1, 5.2, 5.2.1, 5.2.1.1 or 5.2.2.1.

In a preferred embodiment X is $CR^7$ (embodiment 7). In particular (embodiment 7.1), X is $CR^7$ and $R^1$, n, $R^{2a}$, $R^{2b}$, $R^{3a}$, $R^{3b}$, $R^4$, $R^5$, $R^6$, $R^7$ and $R^8$ are as defined in embodiments 1, 1.1, 1.1.1, 1.1.1.1, 2, 2.1, 2.2, 2.2.1, 3, 3.1, 3.1.1, 3.2, 3.2.1, 4, 4.1, 4.1.1, 4.2, 4.2.1, 234, 234.1, 5, 5.1, 5.1.1, 5.1.2, 5.1.1.1, 5.2, 5.2.1, 5.2.1.1, 5.2.2.1, 6 or 6.1.

In particular, the compounds of formula I are compounds of formula I.cis:

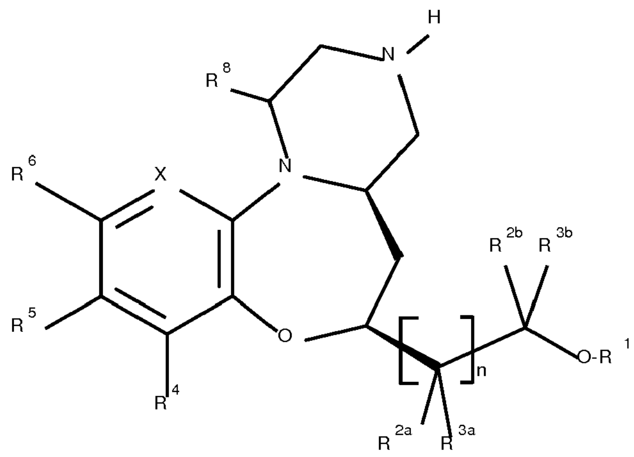

(I.cis)

where X, $R^1$, n, $R^{2a}$, $R^{2b}$, $R^{3a}$, $R^{3b}$, $R^4$, $R^5$, $R^6$ and $R^8$ have one of the above general definitions or, in particular, have one of the above preferred definitions, and are in particular defined as in embodiments 1, 1.1, 1.1.1, 1.1.1.1, 2, 2.1, 2.2, 2.2.1, 3, 3.1, 3.1.1, 3.2, 3.2.1, 4, 4.1, 4.1.1, 4.2, 4.2.1, 234, 234.1, 5, 5.1, 5.1.1, 5.1.2, 5.1.1.1, 5.2, 5.2.1, 5.2.1.1, 5.2.2.1, 6, 6.1, 7 or 7.1.

Examples of preferred compounds are compounds of the following formulae Ia. 1 to Ia. 18 and the stereoisomers thereof, where the variables have one of the general or preferred meanings given above. Examples of preferred compounds are the individual compounds compiled in the tables 1 to 108 below. Moreover, the meanings mentioned below for the individual variables in the tables are per se, independently of the combination in which they are mentioned, a particularly preferred embodiment of the substituents in question.

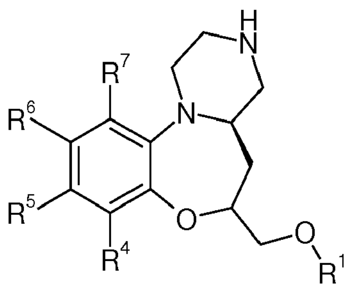

(Ia.1)

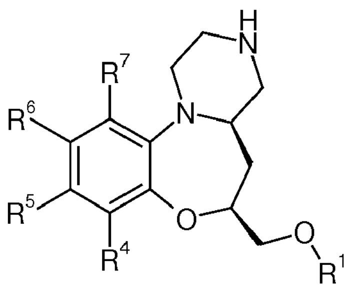

(Ia.2)

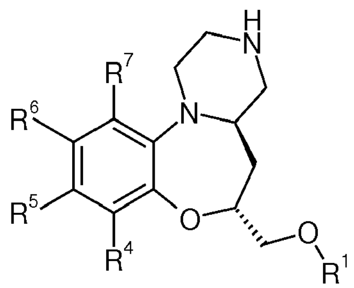

(Ia.3)

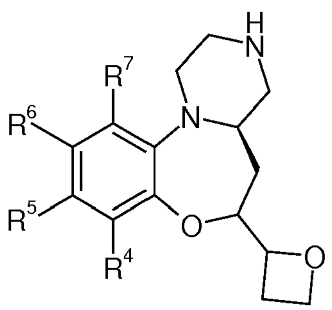

(Ia.4)

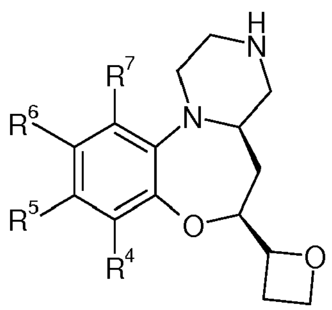

(Ia.5)

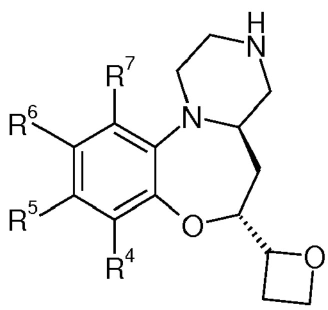

(Ia.6)

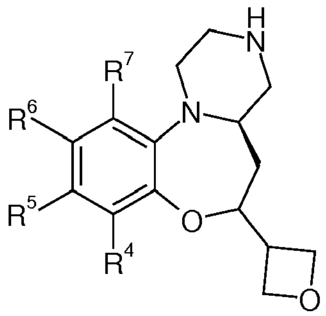

(Ia.7)

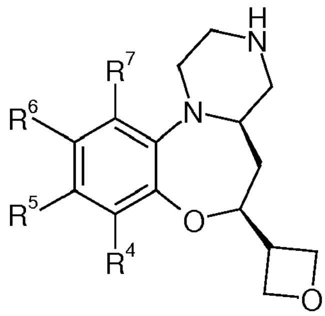

(Ia.8)

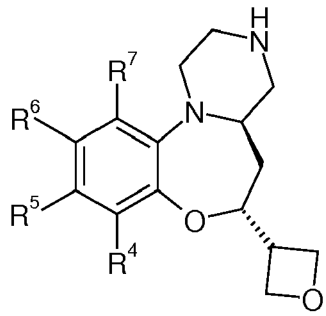

(Ia.9)

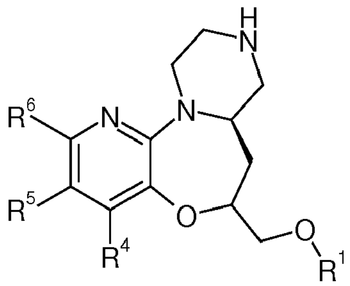

(Ia.10)

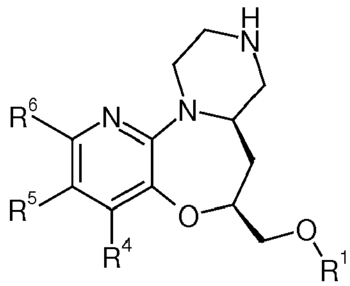

(Ia.11)

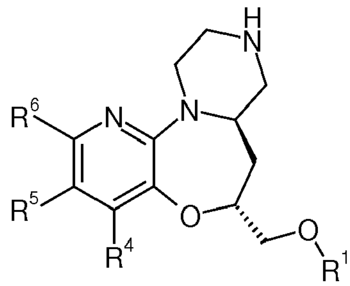

(Ia.12)

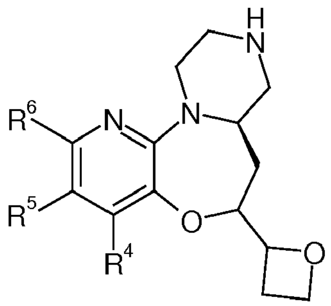

(Ia.13)

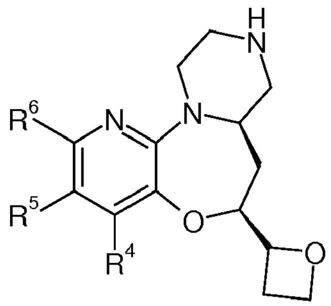

(Ia.14)

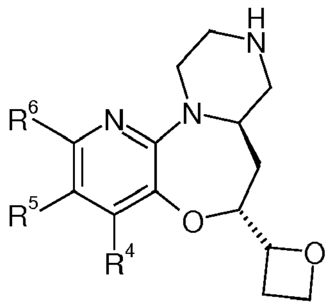

(Ia.15)

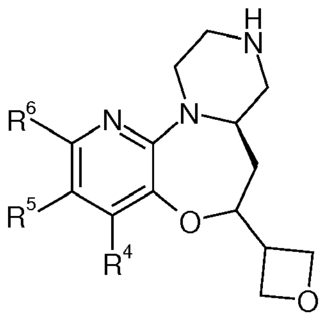

(Ia.16)

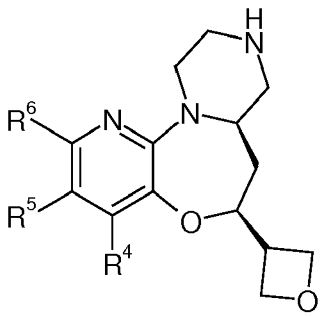

(Ia.17)

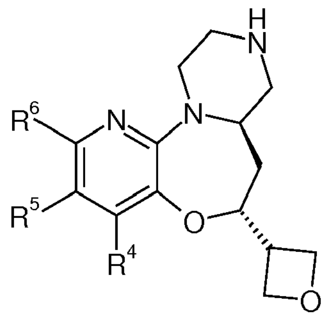

(Ia.18)

Table 1

Compounds of the formula Ia.1 in which $R^1$ is H and the combination of $R^4$, $R^5$, $R^6$ and $R^7$ for a compound corresponds in each case to one row of Table A

Table 2

Compounds of the formula Ia.1 in which $R^1$ is methyl and the combination of $R^4$, $R^5$, $R^6$ and $R^7$ for a compound corresponds in each case to one row of Table A

Table 3
Compounds of the formula Ia.1 in which $R^1$ is $CD_3$ and the combination of $R^4$, $R^5$, $R^6$ and $R^7$ for a compound corresponds in each case to one row of Table A
Table 4
Compounds of the formula Ia.1 in which $R^1$ is $CH_2F$ and the combination of $R^4$, $R^5$, $R^6$ and $R^7$ for a compound corresponds in each case to one row of Table A
Table 5
Compounds of the formula Ia.1 in which $R^1$ is $CHF_2$ and the combination of $R^4$, $R^5$, $R^6$ and $R^7$ for a compound corresponds in each case to one row of Table A
Table 6
Compounds of the formula Ia.1 in which $R^1$ is $CF_3$ and the combination of $R^4$, $R^5$, $R^6$ and $R^7$ for a compound corresponds in each case to one row of Table A
Tables 7 to 12
Compounds of the formula Ia.2 in which $R^1$ is as defined in tables 1 to 6 and the combination of $R^4$, $R^5$, $R^6$ and $R^7$ for a compound corresponds in each case to one row of Table A
Tables 13 to 18
Compounds of the formula Ia.3 in which $R^1$ is as defined in tables 1 to 6 and the combination of $R^4$, $R^5$, $R^6$ and $R^7$ for a compound corresponds in each case to one row of Table A
Table 19
Compounds of the formula Ia.4 in which the combination of $R^4$, $R^5$, $R^6$ and $R^7$ for a compound corresponds in each case to one row of Table A
Table 20
Compounds of the formula Ia.5 in which the combination of $R^4$, $R^5$, $R^6$ and $R^7$ for a compound corresponds in each case to one row of Table A
Table 21
Compounds of the formula Ia.6 in which the combination of $R^4$, $R^5$, $R^6$ and $R^7$ for a compound corresponds in each case to one row of Table A
Table 22
Compounds of the formula Ia.7 in which the combination of $R^4$, $R^5$, $R^6$ and $R^7$ for a compound corresponds in each case to one row of Table A
Table 23
Compounds of the formula Ia.8 in which the combination of $R^4$, $R^5$, $R^6$ and $R^7$ for a compound corresponds in each case to one row of Table A
Table 24
Compounds of the formula Ia.9 in which the combination of $R^4$, $R^5$, $R^6$ and $R^7$ for a compound corresponds in each case to one row of Table A
Tables 25 to 30
Compounds of the formula Ia.10 in which $R^1$ is as defined in tables 1 to 6 and the combination of $R^4$, $R^5$ and $R^6$ for a compound corresponds in each case to one row of Table B
Tables 31 to 36
Compounds of the formula Ia.11 in which $R^1$ is as defined in tables 1 to 6 and the combination of $R^4$, $R^5$ and $R^6$ for a compound corresponds in each case to one row of Table B
Tables 37 to 42
Compounds of the formula Ia.12 in which $R^1$ is as defined in tables 1 to 6 and the combination of $R^4$, $R^5$ and $R^6$ for a compound corresponds in each case to one row of Table B
Table 43
Compounds of the formula Ia.13 in which the combination of $R^4$, $R^5$ and $R^6$ for a compound corresponds in each case to one row of Table B
Table 44
Compounds of the formula Ia.14 in which the combination of $R^4$, $R^5$ and $R^6$ for a compound corresponds in each case to one row of Table B
Table 45
Compounds of the formula Ia.15 in which the combination of $R^4$, $R^5$ and $R^6$ for a compound corresponds in each case to one row of Table B
Table 46
Compounds of the formula Ia.16 in which the combination of $R^4$, $R^5$ and $R^6$ for a compound corresponds in each case to one row of Table B
Table 47

Compounds of the formula Ia.17 in which the combination of $R^4$, $R^5$ and $R^6$ for a compound corresponds in each case to one row of Table B

Table 48

Compounds of the formula Ia.18 in which the combination of $R^4$, $R^5$ and $R^6$ for a compound corresponds in each case to one row of Table B

Table A

| No. | $R^4$ | $R^5$ | $R^6$ | $R^7$ |
|---|---|---|---|---|
| A-1. | H | H | H | H |
| A-2. | F | H | H | H |
| A-3. | Cl | H | H | H |
| A-4. | Br | H | H | H |
| A-5. | CN | H | H | H |
| A-6. | $CH_3$ | H | H | H |
| A-7. | $CH_2F$ | H | H | H |
| A-8. | $CHF_2$ | H | H | H |
| A-9. | $CF_3$ | H | H | H |
| A-10. | $OCH_3$ | H | H | H |
| A-11. | $OCH_2F$ | H | H | H |
| A-12. | $OCHF_2$ | H | H | H |
| A-13. | $OCF_3$ | H | H | H |
| A-14. | c-Pr* | H | H | H |
| A-15. | c-Bu** | H | H | H |
| A-16. | $-CH=CH_2$ | H | H | H |
| A-17. | $-C\equiv CH$ | H | H | H |
| A-18. | H | F | H | H |
| A-19. | H | Cl | H | H |
| A-20. | H | Br | H | H |
| A-21. | H | CN | H | H |
| A-22. | H | $CH_3$ | H | H |
| A-23. | H | $CH_2F$ | H | H |
| A-24. | H | $CHF_2$ | H | H |
| A-25. | H | $CF_3$ | H | H |
| A-26. | H | $OCH_3$ | H | H |
| A-27. | H | $OCH_2F$ | H | H |
| A-28. | H | $OCHF_2$ | H | H |
| A-29. | H | $OCF_3$ | H | H |
| A-30. | H | c-Pr* | H | H |
| A-31. | H | c-Bu** | H | H |
| A-32. | H | $-CH=CH_2$ | H | H |
| A-33. | H | $-C\equiv CH$ | H | H |

(continued)

| No. | $R^4$ | $R^5$ | $R^6$ | $R^7$ |
|---|---|---|---|---|
| A-34. | H | H | F | H |
| A-35. | H | H | Cl | H |
| A-36. | H | H | Br | H |
| A-37. | H | H | CN | H |
| A-38. | H | H | $CH_3$ | H |
| A-39. | H | H | $CH_2F$ | H |
| A-40. | H | H | $CHF_2$ | H |
| A-41. | H | H | $CF_3$ | H |
| A-42. | H | H | $OCH_3$ | H |
| A-43. | H | H | $OCH_2F$ | H |
| A-44. | H | H | $OCHF_2$ | H |
| A-45. | H | H | $OCF_3$ | H |
| A-46. | H | H | c-Pr* | H |
| A-47. | H | H | c-Bu** | H |
| A-48. | H | H | $-CH=CH_2$ | H |
| A-49. | H | H | $-C\equiv CH$ | H |
| A-50. | H | H | H | F |
| A-51. | H | H | H | Cl |
| A-52. | H | H | H | Br |
| A-53. | H | H | H | CN |
| A-54. | H | H | H | $CH_3$ |
| A-55. | H | H | H | $CH_2F$ |
| A-56. | H | H | H | $CHF_2$ |
| A-57. | H | H | H | $CF_3$ |
| A-58. | H | H | H | $OCH_3$ |
| A-59. | H | H | H | $OCH_2F$ |
| A-60. | H | H | H | $OCHF_2$ |
| A-61. | H | H | H | $OCF_3$ |
| A-62. | H | H | H | c-Pr* |
| A-63. | H | H | H | c-Bu** |
| A-64. | H | H | H | $-CH=CH_2$ |
| A-65. | H | H | H | $-C\equiv CH$ |
| A-66. | F | F | H | H |
| A-67. | F | H | F | H |
| A-68. | F | H | H | F |
| A-69. | H | F | F | H |
| A-70. | H | F | H | F |
| A-71. | H | H | F | F |

(continued)

| No. | $R^4$ | $R^5$ | $R^6$ | $R^7$ |
|---|---|---|---|---|
| A-72. | Cl | Cl | H | H |
| A-73. | Cl | H | Cl | H |
| A-74. | Cl | H | H | Cl |
| A-75. | H | Cl | Cl | H |
| A-76. | H | Cl | H | Cl |
| A-77. | H | H | Cl | Cl |
| A-78. | F | Cl | H | H |
| A-79. | F | H | Cl | H |
| A-80. | F | H | H | Cl |
| A-81. | Cl | F | H | H |
| A-82. | Cl | H | F | H |
| A-83. | Cl | H | H | F |
| A-84. | H | F | Cl | H |
| A-85. | H | F | H | Cl |
| A-86. | H | Cl | F | H |
| A-87. | H | Cl | H | F |
| A-88. | H | H | F | Cl |
| A-89. | H | H | Cl | F |
| A-90. | F | F | F | H |
| A-91. | F | F | H | F |
| A-92. | F | H | F | F |
| A-93. | H | F | F | F |
| A-94. | F | F | Cl | H |
| A-95. | F | F | H | Cl |
| A-96. | F | H | F | Cl |
| A-97. | H | F | F | Cl |
| A-98. | F | Cl | F | H |
| A-99. | F | Cl | H | F |
| A-100. | F | H | Cl | F |
| A-101. | H | F | Cl | F |
| A-102. | Cl | F | F | H |
| A-103. | Cl | F | H | F |
| A-104. | Cl | H | F | F |
| A-105. | H | Cl | F | F |
| A-106. | F | F | F | Cl |
| A-107. | F | F | Cl | F |
| A-108. | F | Cl | F | F |
| A-109. | Cl | F | F | F |

(continued)

| No. | $R^4$ | $R^5$ | $R^6$ | $R^7$ |
|---|---|---|---|---|
| A-110. | Cl | Cl | F | H |
| A-111. | Cl | Cl | H | F |
| A-112. | Cl | H | Cl | F |
| A-113. | Cl | F | Cl | H |
| A-114. | Cl | F | H | Cl |
| A-115. | Cl | H | F | Cl |
| A-116. | H | Cl | Cl | F |
| A-117. | H | Cl | F | Cl |
| A-118. | H | F | Cl | Cl |
| A-119. | F | Cl | Cl | H |
| A-120. | F | Cl | H | Cl |
| A-121. | F | H | Cl | Cl |
| A-122. | F | F | Cl | Cl |
| A-123. | F | Cl | F | Cl |
| A-124. | F | Cl | Cl | F |
| A-125. | Cl | F | F | Cl |
| A-126. | Cl | F | Cl | F |
| A-127. | Cl | Cl | F | Cl |
| A-128. | Cl | Cl | Cl | F |
| A-129. | Cl | Cl | F | Cl |
| A-130. | Cl | F | Cl | Cl |
| A-131. | F | Cl | Cl | Cl |
| A-132. | F | F | F | F |
| A-133. | Cl | Cl | Cl | Cl |
| A-134. | Br | F | H | H |
| A-135. | CN | F | H | H |
| A-136. | $CH_3$ | F | H | H |
| A-137. | $CH_2F$ | F | H | H |
| A-138. | $CHF_2$ | F | H | H |
| A-139. | $CF_3$ | F | H | H |
| A-140. | $OCH_3$ | F | H | H |
| A-141. | $OCH_2F$ | F | H | H |
| A-142. | $OCHF_2$ | F | H | H |
| A-143. | $OCF_3$ | F | H | H |
| A-144. | c-Pr* | F | H | H |
| A-145. | c-Bu** | F | H | H |
| A-146. | $-CH=CH_2$ | F | H | H |
| A-147. | $-C\equiv CH$ | F | H | H |

(continued)

| No. | $R^4$ | $R^5$ | $R^6$ | $R^7$ |
|---|---|---|---|---|
| A-148. | Br | H | F | H |
| A-149. | CN | H | F | H |
| A-150. | $CH_3$ | H | F | H |
| A-151. | $CH_2F$ | H | F | H |
| A-152. | $CHF_2$ | H | F | H |
| A-153. | $CF_3$ | H | F | H |
| A-154. | $OCH_3$ | H | F | H |
| A-155. | $OCH_2F$ | H | F | H |
| A-156. | $OCHF_2$ | H | F | H |
| A-157. | $OCF_3$ | H | F | H |
| A-158. | c-Pr* | H | F | H |
| A-159. | c-Bu** | H | F | H |
| A-160. | $-CH=CH_2$ | H | F | H |
| A-161. | $-C\equiv CH$ | H | F | H |
| A-162. | Br | H | H | F |
| A-163. | CN | H | H | F |
| A-164. | $CH_3$ | H | H | F |
| A-165. | $CH_2F$ | H | H | F |
| A-166. | $CHF_2$ | H | H | F |
| A-167. | $CF_3$ | H | H | F |
| A-168. | $OCH_3$ | H | H | F |
| A-169. | $OCH_2F$ | H | H | F |
| A-170. | $OCHF_2$ | H | H | F |
| A-171. | $OCF_3$ | H | H | F |
| A-172. | c-Pr* | H | H | F |
| A-173. | c-Bu** | H | H | F |
| A-174. | $-CH=CH_2$ | H | H | F |
| A-175. | $-C\equiv CH$ | H | H | F |
| A-176. | Br | H | F | F |
| A-177. | CN | H | F | F |
| A-178. | $CH_3$ | H | F | F |
| A-179. | $CH_2F$ | H | F | F |
| A-180. | $CHF_2$ | H | F | F |
| A-181. | $CF_3$ | H | F | F |
| A-182. | $OCH_3$ | H | F | F |
| A-183. | $OCH_2F$ | H | F | F |
| A-184. | $OCHF_2$ | H | F | F |
| A-185. | $OCF_3$ | H | F | F |

(continued)

| No. | $R^4$ | $R^5$ | $R^6$ | $R^7$ |
|---|---|---|---|---|
| A-186. | c-Pr* | H | F | F |
| A-187. | c-Bu** | H | F | F |
| A-188. | $-CH=CH_2$ | H | F | F |
| A-189. | $-C\equiv CH$ | H | F | F |
| A-190. | F | Br | H | H |
| A-191. | F | CN | H | H |
| A-192. | F | $CH_3$ | H | H |
| A-193. | F | $CH_2F$ | H | H |
| A-194. | F | $CHF_2$ | H | H |
| A-195. | F | $CF_3$ | H | H |
| A-196. | F | $OCH_3$ | H | H |
| A-197. | F | $OCH_2F$ | H | H |
| A-198. | F | $OCHF_2$ | H | H |
| A-199. | F | $OCF_3$ | H | H |
| A-200. | F | c-Pr* | H | H |
| A-201. | F | c-Bu** | H | H |
| A-202. | F | $-CH=CH_2$ | H | H |
| A-203. | F | $-C\equiv CH$ | H | H |
| A-204. | F | H | Br | H |
| A-205. | F | H | CN | H |
| A-206. | F | H | $CH_3$ | H |
| A-207. | F | H | $CH_2F$ | H |
| A-208. | F | H | $CHF_2$ | H |
| A-209. | F | H | $CF_3$ | H |
| A-210. | F | H | $OCH_3$ | H |
| A-211. | F | H | $OCH_2F$ | H |
| A-212. | F | H | $OCHF_2$ | H |
| A-213. | F | H | $OCF_3$ | H |
| A-214. | F | H | c-Pr* | H |
| A-215. | F | H | c-Bu** | H |
| A-216. | F | H | $-CH=CH_2$ | H |
| A-217. | F | H | $-C\equiv CH$ | H |
| A-218. | F | H | H | Br |
| A-219. | F | H | H | CN |
| A-220. | F | H | H | $CH_3$ |
| A-221. | F | H | H | $CH_2F$ |
| A-222. | F | H | H | $CHF_2$ |
| A-223. | F | H | H | $CF_3$ |

(continued)

| No. | $R^4$ | $R^5$ | $R^6$ | $R^7$ |
|---|---|---|---|---|
| A-224. | F | H | H | $OCH_3$ |
| A-225. | F | H | H | $OCH_2F$ |
| A-226. | F | H | H | $OCHF_2$ |
| A-227. | F | H | H | $OCF_3$ |
| A-228. | F | H | H | c-Pr* |
| A-229. | F | H | H | c-Bu** |
| A-230. | F | H | H | $-CH=CH_2$ |
| A-231. | F | H | H | $-C\equiv CH$ |
| A-232. | Cl | Br | H | H |
| A-233. | Cl | CN | H | H |
| A-234. | Cl | $CH_3$ | H | H |
| A-235. | Cl | $CH_2F$ | H | H |
| A-236. | Cl | $CHF_2$ | H | H |
| A-237. | Cl | $CF_3$ | H | H |
| A-238. | Cl | $OCH_3$ | H | H |
| A-239. | Cl | $OCH_2F$ | H | H |
| A-240. | Cl | $OCHF_2$ | H | H |
| A-241. | Cl | $OCF_3$ | H | H |
| A-242. | Cl | c-Pr* | H | H |
| A-243. | Cl | c-Bu** | H | H |
| A-244. | Cl | $-CH=CH_2$ | H | H |
| A-245. | Cl | $-C\equiv CH$ | H | H |
| A-246. | Cl | H | Br | H |
| A-247. | Cl | H | CN | H |
| A-248. | Cl | H | $CH_3$ | H |
| A-249. | Cl | H | $CH_2F$ | H |
| A-250. | Cl | H | $CHF_2$ | H |
| A-251. | Cl | H | $CF_3$ | H |
| A-252. | Cl | H | $OCH_3$ | H |
| A-253. | Cl | H | $OCH_2F$ | H |
| A-254. | Cl | H | $OCHF_2$ | H |
| A-255. | Cl | H | $OCF_3$ | H |
| A-256. | Cl | H | c-Pr* | H |
| A-257. | Cl | H | c-Bu** | H |
| A-258. | Cl | H | $-CH=CH_2$ | H |
| A-259. | Cl | H | $-C\equiv CH$ | H |
| A-260. | Cl | H | H | Br |
| A-261. | Cl | H | H | CN |

(continued)

| No. | $R^4$ | $R^5$ | $R^6$ | $R^7$ |
|---|---|---|---|---|
| A-262. | Cl | H | H | $CH_3$ |
| A-263. | Cl | H | H | $CH_2F$ |
| A-264. | Cl | H | H | $CHF_2$ |
| A-265. | Cl | H | H | $CF_3$ |
| A-266. | Cl | H | H | $OCH_3$ |
| A-267. | Cl | H | H | $OCH_2F$ |
| A-268. | Cl | H | H | $OCHF_2$ |
| A-269. | Cl | H | H | $OCF_3$ |
| A-270. | Cl | H | H | c-Pr* |
| A-271. | Cl | H | H | c-Bu** |
| A-272. | Cl | H | H | $-CH=CH_2$ |
| A-273. | Cl | H | H | $-C\equiv CH$ |
| A-274. | $CHF_2$ | H | $OCH_3$ | F |
| A-275. | Cl | $OCH_3$ | F | F |

Table B

| No. | $R^4$ | $R^5$ | $R^6$ |
|---|---|---|---|
| B-1 | H | H | H |
| B-2 | F | H | H |
| B-3 | Cl | H | H |
| B-4 | Br | H | H |
| B-5 | CN | H | H |
| B-6 | $CH_3$ | H | H |
| B-7 | $CH_2F$ | H | H |
| B-8 | $CHF_2$ | H | H |
| B-9 | $CF_3$ | H | H |
| B-10 | $OCH_3$ | H | H |
| B-11 | $OCH_2F$ | H | H |
| B-12 | $OCHF_2$ | H | H |
| B-13 | $OCF_3$ | H | H |
| B-14 | c-Pr* | H | H |
| B-15 | c-Bu** | H | H |
| B-16 | $-CH=CH_2$ | H | H |
| B-17 | $-C\equiv CH$ | H | H |
| B-18 | H | F | H |
| B-19 | H | Cl | H |
| B-20 | H | Br | H |

(continued)

| No. | $R^4$ | $R^5$ | $R^6$ |
|---|---|---|---|
| B-21 | H | CN | H |
| B-22 | H | $CH_3$ | H |
| B-23 | H | $CH_2F$ | H |
| B-24 | H | $CHF_2$ | H |
| B-25 | H | $CF_3$ | H |
| B-26 | H | $OCH_3$ | H |
| B-27 | H | $OCH_2F$ | H |
| B-28 | H | $OCHF_2$ | H |
| B-29 | H | $OCF_3$ | H |
| B-30 | H | c-Pr* | H |
| B-31 | H | c-Bu** | H |
| B-32 | H | $-CH=CH_2$ | H |
| B-33 | H | $-C \equiv CH$ | H |
| B-34 | H | H | F |
| B-35 | H | H | Cl |
| B-36 | H | H | Br |
| B-37 | H | H | CN |
| B-38 | H | H | $CH_3$ |
| B-39 | H | H | $CH_2F$ |
| B-40 | H | H | $CHF_2$ |
| B-41 | H | H | $CF_3$ |
| B-42 | H | H | $OCH_3$ |
| B-43 | H | H | $OCH_2F$ |
| B-44 | H | H | $OCHF_2$ |
| B-45 | H | H | $OCF_3$ |
| B-46 | H | H | c-Pr* |
| B-47 | H | H | c-Bu** |
| B-48 | H | H | $-CH=CH_2$ |
| B-49 | H | H | $-C \equiv CH$ |
| B-50 | F | F | H |
| B-51 | F | H | F |
| B-52 | H | F | F |
| B-53 | Cl | Cl | H |
| B-54 | Cl | H | Cl |
| B-55 | H | Cl | Cl |
| B-56 | F | Cl | H |
| B-57 | F | H | Cl |
| B-58 | Cl | F | H |

(continued)

| No. | $R^4$ | $R^5$ | $R^6$ |
|---|---|---|---|
| B-59 | Cl | H | F |
| B-60 | H | F | Cl |
| B-61 | H | Cl | F |
| B-62 | F | F | Cl |
| B-63 | F | Cl | F |
| B-64 | Cl | F | F |
| B-65 | Cl | Cl | F |
| B-66 | Cl | F | Cl |
| B-67 | F | Cl | Cl |
| B-68 | F | F | F |
| B-69 | Cl | Cl | Cl |
| B-70 | Br | F | H |
| B-71 | CN | F | H |
| B-72 | $CH_3$ | F | H |
| B-73 | $CH_2F$ | F | H |
| B-74 | $CHF_2$ | F | H |
| B-75 | $CF_3$ | F | H |
| B-76 | $OCH_3$ | F | H |
| B-77 | $OCH_2F$ | F | H |
| B-78 | $OCHF_2$ | F | H |
| B-79 | $OCF_3$ | F | H |
| B-80 | c-Pr* | F | H |
| B-81 | c-Bu** | F | H |
| B-82 | $-CH=CH_2$ | F | H |
| B-83 | -C≡CH | F | H |
| B-84 | Br | H | F |
| B-85 | CN | H | F |
| B-86 | $CH_3$ | H | F |
| B-87 | $CH_2F$ | H | F |
| B-88 | $CHF_2$ | H | F |
| B-89 | $CF_3$ | H | F |
| B-90 | $OCH_3$ | H | F |
| B-91 | $OCH_2F$ | H | F |
| B-92 | $OCHF_2$ | H | F |
| B-93 | $OCF_3$ | H | F |
| B-94 | c-Pr* | H | F |
| B-95 | c-Bu** | H | F |
| B-96 | $-CH=CH_2$ | H | F |

(continued)

| No. | $R^4$ | $R^5$ | $R^6$ |
|---|---|---|---|
| B-97 | -C≡CH | H | F |
| B-98 | Br | F | F |
| B-99 | CN | F | F |
| B-100 | $CH_3$ | F | F |
| B-101 | $CH_2F$ | F | F |
| B-102 | $CHF_2$ | F | F |
| B-103 | $CF_3$ | F | F |
| B-104 | $OCH_3$ | F | F |
| B-105 | $OCH_2F$ | F | F |
| B-106 | $OCHF_2$ | F | F |
| B-107 | $OCF_3$ | F | F |
| B-108 | c-Pr* | F | F |
| B-109 | c-Bu** | F | F |
| B-110 | $-CH=CH_2$ | F | F |
| B-111 | -C≡CH | F | F |
| B-112 | F | Br | H |
| B-113 | F | CN | H |
| B-114 | F | $CH_3$ | H |
| B-115 | F | $CH_2F$ | H |
| B-116 | F | $CHF_2$ | H |
| B-117 | F | $CF_3$ | H |
| B-118 | F | $OCH_3$ | H |
| B-119 | F | $OCH_2F$ | H |
| B-120 | F | $OCHF_2$ | H |
| B-121 | F | $OCF_3$ | H |
| B-122 | F | c-Pr* | H |
| B-123 | F | c-Bu** | H |
| B-124 | F | $-CH=CH_2$ | H |
| B-125 | F | -C≡CH | H |
| B-126 | F | H | Br |
| B-127 | F | H | CN |
| B-128 | F | H | $CH_3$ |
| B-129 | F | H | $CH_2F$ |
| B-130 | F | H | $CHF_2$ |
| B-131 | F | H | $CF_3$ |
| B-132 | F | H | $OCH_3$ |
| B-133 | F | H | $OCH_2F$ |
| B-134 | F | H | $OCHF_2$ |

(continued)

| No. | $R^4$ | $R^5$ | $R^6$ |
|---|---|---|---|
| B-135 | F | H | $OCF_3$ |
| B-136 | F | H | c-Pr* |
| B-137 | F | H | c-Bu** |
| B-138 | F | H | $-CH=CH_2$ |
| B-139 | F | H | $-C\equiv CH$ |
| B-140 | H | F | Br |
| B-141 | H | F | CN |
| B-142 | H | F | $CH_3$ |
| B-143 | H | F | $CH_2F$ |
| B-144 | H | F | $CHF_2$ |
| B-145 | H | F | $CF_3$ |
| B-146 | H | F | $OCH_3$ |
| B-147 | H | F | $OCH_2F$ |
| B-148 | H | F | $OCHF_2$ |
| B-149 | H | F | $OCF_3$ |
| B-150 | H | F | c-Pr* |
| B-151 | H | F | c-Bu** |
| B-152 | H | F | $-CH=CH_2$ |
| B-153 | H | F | $-C\equiv CH$ |
| B-154 | H | Br | F |
| B-155 | H | CN | F |
| B-156 | H | $CH_3$ | F |
| B-157 | H | $CH_2F$ | F |
| B-158 | H | $CHF_2$ | F |
| B-159 | H | $CF_3$ | F |
| B-160 | H | $OCH_3$ | F |
| B-161 | H | $OCH_2F$ | F |
| B-162 | H | $OCHF_2$ | F |
| B-163 | H | $OCF_3$ | F |
| B-164 | H | c-Pr* | F |
| B-165 | H | c-Bu** | F |
| B-166 | H | $-CH=CH_2$ | F |
| B-167 | H | $-C\equiv CH$ | F |
| B-168 | F | Br | F |
| B-169 | F | CN | F |
| B-170 | F | $CH_3$ | F |
| B-171 | F | $CH_2F$ | F |
| B-172 | F | $CHF_2$ | F |

(continued)

| No. | $R^4$ | $R^5$ | $R^6$ |
|---|---|---|---|
| B-173 | F | $CF_3$ | F |
| B-174 | F | $OCH_3$ | F |
| B-175 | F | $OCH_2F$ | F |
| B-176 | F | $OCHF_2$ | F |
| B-177 | F | $OCF_3$ | F |
| B-178 | F | c-Pr* | F |
| B-179 | F | c-Bu** | F |
| B-180 | F | $-CH=CH_2$ | F |
| B-181 | F | -C≡CH | F |
| * c-Pr = cyclopropyl<br>** c-Bu = cyclobutyl | | | |

Among the above compounds preference is given to compounds of formulae Ia. 1 to Ia.3 and Ia.10 to Ia.12.

In a specific embodiment, the invention relates to compounds I selected from the compounds of the examples, either in form of free bases or of any pharmaceutically acceptable salt thereof or a stereoisomer, the racemate or any mixture of stereoisomers thereof or a tautomer or a tautomeric mixture or an N-oxide thereof.

The compounds of the present invention can be prepared by using routine techniques familiar to a skilled person. In particular, the compounds of the formula I can be prepared according to the following schemes, wherein the variables, if not stated otherwise, are as defined above.

Compounds I can be prepared as shown in scheme 1 below. A is either the group $-[-C(R^{2a})(R^{3a})]_n-C(R^{2b})(R^{3b})-OR^1$ or a precursor thereof (therefore the final compounds of scheme 1 are named I'). For instance compounds wherein $R^1$ is methyl can be a precursor of compounds wherein $R^1$ is H and compounds wherein $R^1$ is H can in turn be a precursor for compounds wherein $R^1$ is methyl, deuterated methyl or fluorinated methyl. $PG^1$ is a protective group. Suitable protective groups are for example $C_1$-$C_4$-alkylcarbonyl (e.g. acetyl), $C_1$-$C_4$-haloalkylcarbonyl (e.g. trifluoroacetyl), $C_3$-$C_4$-alkenylcarbonyl (e.g. allylcarbonyl), $C_1$-$C_4$-alkoxycarbonyl (e.g. ethoxycarbonyl, Boc), $C_3$-$C_4$-alkenyloxycarbonyl, $C_1$-$C_4$-alkylaminocarbonyl, di-($C_1$-$C_4$-alkyl)-aminocarbonyl, $C_1$-$C_4$-alkylsulfonyl, $C_1$-$C_4$-haloalkylsulfonyl or benzyl. Specifically benzyl or $C_1$-$C_4$-alkoxycarbonyl (especially ethoxycarbonyl, Boc) are used. Y is a suitable leaving group, such as Cl, Br, I, triflate or tosylate. Y can be Cl, Br, I, triflate or tosylate if $R^4$ is F. If $R^4$ is Cl and $R^5$ and $R^7$ (in case that X is $R^7$) are not identical, Y is Br, I, triflate or tosylate; if $R^4$ is Br and $R^5$ and $R^7$ (in case that X is $R^7$) are not identical, Y is I; otherwise the C-$R^4$ site would compete with the C-Y site in the ring-closing reaction. In case that $R^5$ and $R^7$ are identical, Y and $R^4$ can have the same meaning, e.g. can both be Cl or Br or I. Compounds in which $R^4$ is I and $R^5$ and $R^7$ (in case that X is $R^7$) are not identical can be prepared by either reacting a compound **1** wherein both Y and $R^4$ are I and separating the two ring-closing isomers, or by introducing I as $R^4$ only after the ring-closing reaction. Preferably Y is Cl or Br and in particular Br (of course with the above provisos). The ring closing reaction of **1** to **2** is carried out in the presence of a transition metal catalyst, especially a Pd catalyst, with ligands such as SPhos (2-dicyclohexylphosphino-2',6'-dimethoxybiphenyl), dppf (1,1'-bis(diphenylphosphino)-ferrocene), BINAP (2,2'-bis(diphenylphosphino)-1,1'-binaphthyl), XPhos (2-dicyclohexylphosphino-2',4',6'-triisopropylbiphenyl), RuPhos (2-dicyclohexylphosphino-2',6'-diisopropoxybiphenyl), BrettPhos (2-(dicyclohexylphosphino)3,6-dimethoxy-2',4',6'-triisopropyl-1,1'-biphenyl) and the like, in the presence of a base. Suitable bases are advantageously non-nucleophilic, e.g. a carbonate, such as lithium, sodium, potassium or caesium carbonate, DBU (1,8-diazabicyclo[5.4.0]undec-7-en), DBN (1,5-Diazabicyclo[4.3.0]non-5-en) and the like, or a sterically hindered nucleophilic alcoholate, like sodium or potassium tert-butanolate. The reaction is generally carried out in a solvent, suitably an aprotic solvent, such as toluene, the xylenes, dioxane, tetrahydrofurane, DMSO or DMF. Deprotection of **3** yields **I'.** Deprotection conditions depend on the protective group used. For instance, benzyl groups are cleaved under hydrogenolysis, suitably in the presence of a hydrogenation catalyst, such as Pd. Alternatively, and avoiding hydrogenolysis, the benzyl protective group can be first converted into a carbamate group which can be removed by acid, neutral or basic treatment, such as ethoxycarbonyl or 1-chloroethoxycarbonyl. Conversion of the benzyl group is for example carried out by reaction with the respective carbonic ester chloride.

Scheme 1

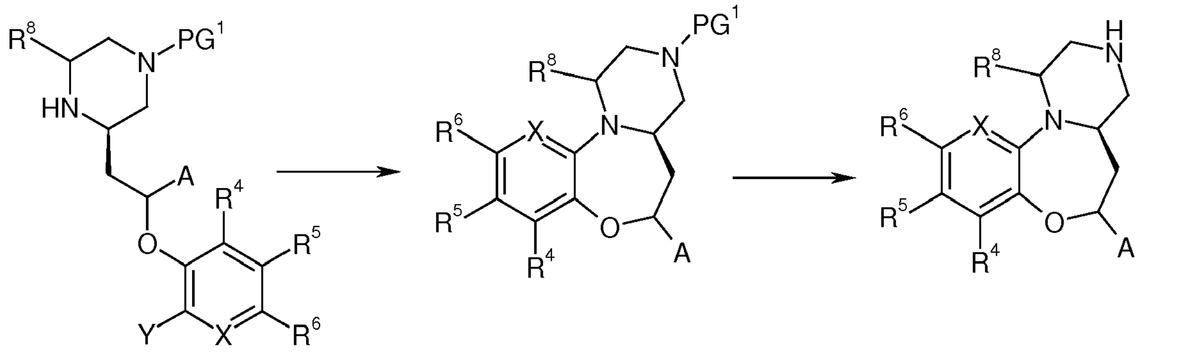

1 2 I'

Compounds 1 can be prepared as shown in scheme 2 below. Y is as defined above. $PG^1$ and $PG^2$ are different, orthogonal protective groups which can be removed under different conditions, so that $PG^2$ can be readily cleaved without affecting $PG^1$. For instance $PG^1$ is benzyl or ethoxycarbonyl and $PG^2$ is Boc. Other orthogonal pairs of $PG^1$ and $PG^2$ are known in the art. Deprotection of **3** to singly protected **4** is carried out under conditions suitable for the respective $PG^2$ group, which however do not influence $PG^1$. For instance, Boc is removed under acidic conditions if $PG^1$ is a group which cannot be cleaved under these circumstances, such as benzyl or ethoxycarbonyl.

Nucleophilic aromatic substitution of the fluorine atom in **5** by **4** is carried out by reacting **4** and **5** under basic conditions. Alternatively **4** is first deprotonated by a base and then the resulting alkoxylate is reacted with **5.** Suitable bases are advantageously non-nucleophilic, e.g. a carbonate, such as lithium, sodium, potassium or caesium carbonate, DBU, DBN and the like, or a sterically hindered nucleophilic alcoholate, like sodium or potassium tert-butanolate. Sterically non-demanding nucleophilic bases can be used if they are first reacted with the alcohol **4** before compound **5** is added. Suitable bases for this purpose are e.g. hydroxides, such as sodium or potassium hydroxide, hydrides, such as sodium or potassium hydride, and LDA. Non-nucleophilic bases or sterically hindered nucleophilic alcoholates can of course also be used for first deprotonating the alcohol **4** before compound **5** is added, as long as they are strong enough for the deprotonation.

Scheme 2

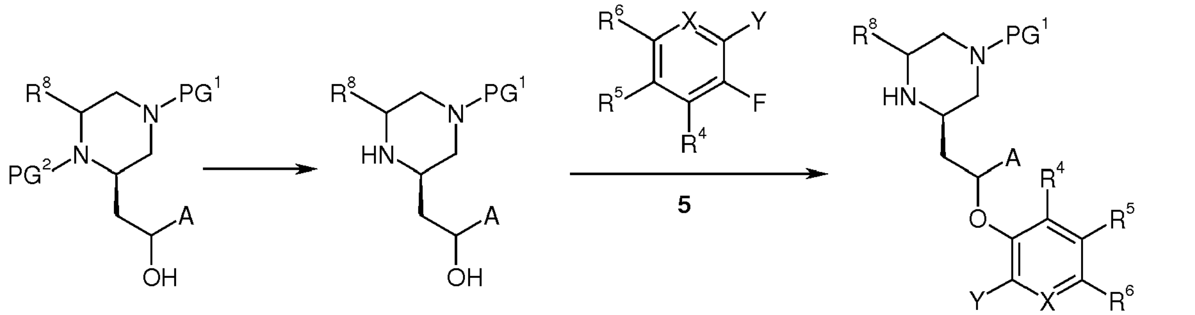

5

3 4 1

Alternatively compounds **1** can be prepared by first reacting **3** with compound **5** in a nucleophilic aromatic substitution reaction under basic conditions and then removing from the resulting compound **6** the protective group $PG^2$. Y, $PG^1$ and $PG^2$ are as defined above. Suitable bases and reaction conditions for the nucleophilic aromatic substitution reaction as well as for deprotection correspond to those described above for scheme 2.

Scheme 3

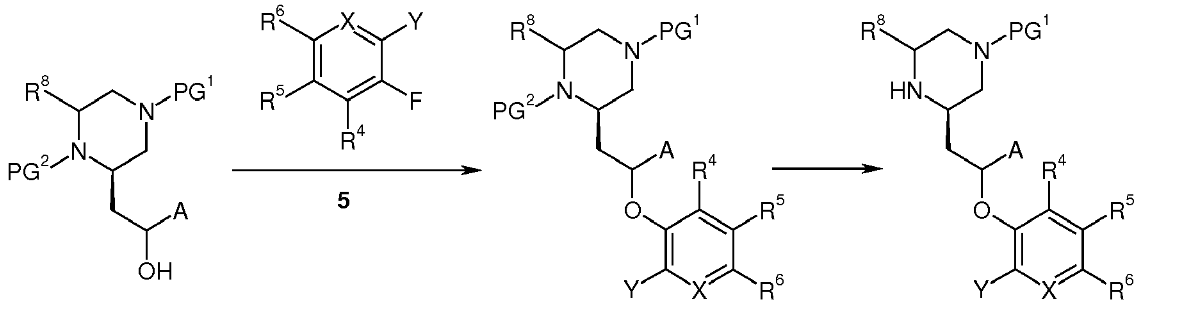

5

3 6 1

In yet another alternative compounds **1** can be prepared by first reacting **3** with compound **7** in a Mitsunobu reaction using triphenylphosphine and an azodicarboxylate such as diethyl azodicarboxylate (DEAD) or diisopropyl azodicarboxylate (DIAD) as shown in scheme 4 below. Selective deprotection of **6** yields **1.** Y, $PG^1$ and $PG^2$ are as defined above.

Scheme 4

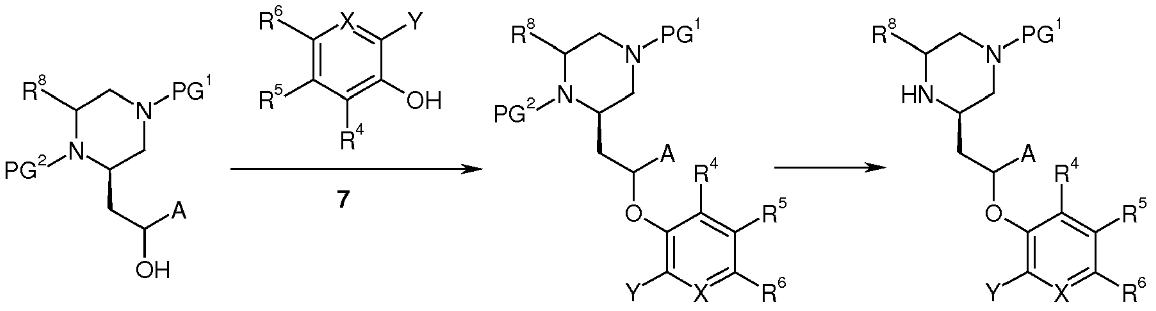

7

3 6 1

Compounds **3** wherein A is $-CH_2-O-CH_3$ (named **3'** in the following) or $-CH_2-O-$(deuterated $CH_3$) can be prepared as outlined in scheme 5 (scheme 5 shows only the non-deuterated group A) below by first oxidizing the primary alcohol 8 to the respective aldehyde **9.** Suitable oxidizing agents are those known as suitable for the selective oxidation of primary alcohols to aldehydes, such as dimethylsulfoxide (in combination with oxalyl chloride and trimethylamine; Swern oxidation), Dess-Martin periodinane, pyridiniumchlorochromate, pyridiniumdichromate, chromiumtrioxide pyridine complex, manganese dioxide, HOCl, quinones, such as DDQ and the like. Reductive alkylation of aldehyde **9** to alcohol **3'** can be carried out via Grignard reduction using methoxymethyl chloride (or deuterated methoxymethyl chloride) and magnesium. The reaction can be carried out in the catalytic presence of mercury salts, e.g. HgCl. Alternatively the reaction can be carried out under Barbier conditions using magnesium, aluminum, zinc, indium, tin or salts thereof. In yet another alternative the respective lithium compounds can be used.

Scheme 5

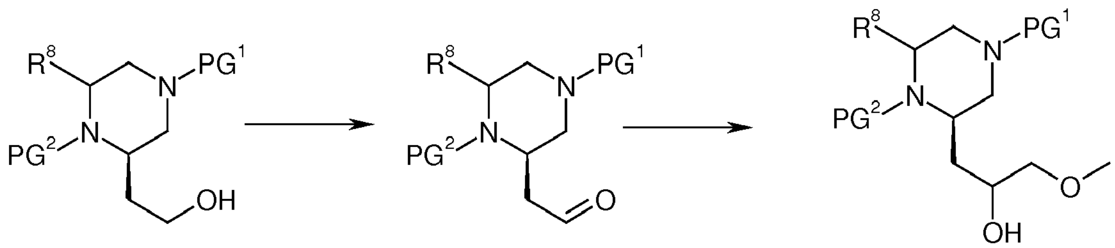

8 9 3'

Compounds **8** wherein $R^8$ is hydrogen (termed **8'** in the following) can be prepared as shown in scheme 6 below. (R)-2-aminosuccinic acid **10** is converted into its monomethyl ester **11.** The esterification reaction can be carried

out by standard esterification reactions, such as conversion of the acid into its chloride, e.g. by reaction with acetyl chloride, thionyl chloride, oxalyl chloride or the like, and reaction of the chloride with methanol. Protection of the amino group of **11** with boc, e.g. by reaction with boc anhydride, yields **12,** which is subjected to an amidation reaction. The amidation is suitably carried out in the presence of a coupling reagent. Suitable coupling reagents (activators) are well known and are for instance selected from carbodiimides, such as EDCI (1-ethyl-3-(3-dimethylaminopropyl)carbodiimide), DCC (dicyclohexylcarbodiimide) and DCI (diisopropylcarbodiimide), benzotriazol derivatives, such as HOBt (1-hydroxybenzotriazole), HATU (O-(7-azabenzotriazol-1-yl)-N,N,N',N'-tetramethyluronium hexafluorophosphate), HBTU ((O-benzotriazol-1-yl)-N,N,N',N'-tetramethyluronium hexafluorophosphate) and HCTU (1H-benzotriazolium-1-[bis(dimethylamino)methylene]-5-chloro tetrafluoroborate) and phosphonium-derived activators, such as BOP ((benzotriazol-1-yloxy)-tris(dimethyl-amino)phosphonium hexafluorophosphate), Py-BOP ((benzotriazol-1-yloxy)-tripyrrolidinphosphonium hexafluorophosphate) and Py-BrOP (bromotripyrrolidinphosphonium hexafluorophosphate). Generally, the activator is used in excess. The benzotriazol and phosphonium coupling reagents are generally used in a basic medium. The ring closing reaction to **14** occurs under basic conditions after removal of the boc group. Reduction of **14** with a suitable reduction agent, e.g. suitable hydride complexes such as lithium aluminum hydride (LAH) or diisobutylaluminium hydride (DIBAL-H) yields **15,** which is then N-protected with $PG^2$ to yield **8'.**

Scheme 6

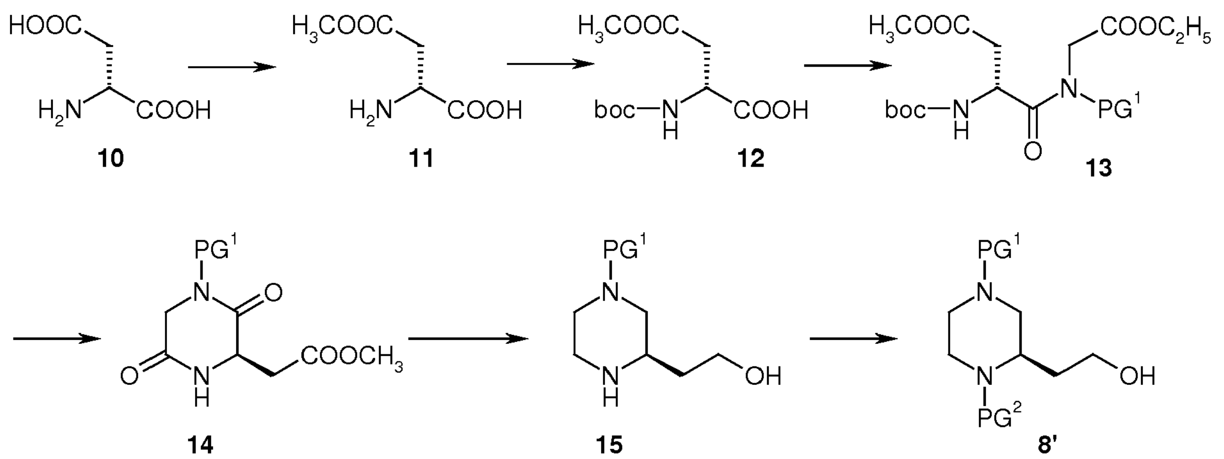

10 11 12 13 14 15 8'

Compounds wherein $R^1$ is methyl can be converted into compounds wherein $R^1$ is hydrogen by demethylating the former under suitable ether cleavage conditions, such as $BX_3$, where X is Cl, Br or I, $BF_3$-etherate in the presence of 1,2-ethanedithiole, $AlX_3$, where X is Cl or Br (suitably in the presence of ethynethiole), $SiCl_4$+NaI, aq. HBr or aq. HI (in high concentration), or trimethylsilyliodide (TMSI) and the like.

Compounds wherein $R^1$ is hydrogen can be converted into compounds wherein $R^1$ is methyl or deuterated methyl by reaction with a compound $R^{1'}$-Z wherein $R^{1'}$ is methyl or deuterated methyl and Z is a leaving group, such as Cl, Br, I, triflate, tosylate and the like, usually under basic conditions. Suitable bases are for example those listed above in context with scheme 2.

Compounds wherein $R^1$ is hydrogen can be converted into compounds wherein $R^1$ is difluoromethyl by reaction with a difluorocarbene precursor, such E-$CF_2$-LG, where E is H, C(O)OH, TMS or $P(O)(OC_2H_5)_2$ and LG is Cl, Br or $S(O)_2F$; for example $CHF_2Cl$, $C(Si(CH_3)_3)F_2Cl$, $C(Si(CH_3)_3)F_2Br$, $CHF_2S(O)_2F$, 2,2-difluoro-2-(fluorosulfonyl) acetic acid or $CF_2BrP(O)(OC_2H_5)_2$, suitably in the presence of a Cu(I) salt and a base.

Compounds I or I' wherein $R^4$, $R^5$, $R^6$ or $R^7$ (in case that X is $CR^7$) is $C_1$-$C_4$-alkyl, fluorinated $C_1$-$C_4$-alkyl, $C_1$-$C_4$-hydroxyalkyl, $C_2$-$C_4$-alkenyl, fluorinated $C_2$-$C_4$-alkenyl, $C_2$-$C_4$-alkynyl, fluorinated $C_2$-$C_4$-alkynyl, $C_3$-$C_6$-cycloalkyl or fluorinated $C_3$-$C_6$-cycloalkyl can be prepared from compounds I or I' wherein $R^4$, $R^5$, $R^6$ or $R^7$ (in case that X is $CR^7$) is Cl, Br or I and wherein the secondary nitrogen atom carries a protective group by reaction with a nucleophile compound $R^4$-M or $R^5$-M, $R^6$-M or $R^7$-M in the presence of a Pd catalyst (e.g. one of the Pd catalysts mention in context with scheme 1), where $R^4$, $R^5$, $R^6$ or $R^7$ in the four latter compounds is $C_1$-$C_4$-alkyl, fluorinated $C_1$-$C_4$-alkyl, $C_1$-$C_4$-hydroxyalkyl, $C_2$-$C_4$-alkenyl, fluorinated $C_2$-$C_4$-alkenyl, $C_2$-$C_4$-alkynyl, fluorinated $C_2$-$C_4$-alkynyl, $C_3$-$C_6$-cycloalkyl or fluorinated $C_3$-$C_6$-cycloalkyl and M is a suitable metal (group), such as MgCl, MgBr or Li.

Alternatively, such compounds, and especially compounds wherein $R^4$, $R^5$, $R^6$ or $R^7$ (in case that X is $CR^7$) is $C_1$-$C_4$-alkyl, fluorinated $C_1$-$C_4$-alkyl, $C_1$-$C_4$-hydroxyalkyl, $C_2$-$C_4$-alkenyl, fluorinated $C_2$-$C_4$-alkenyl, $C_2$-$C_4$-alkynyl, fluorinated $C_2$-$C_4$-alkynyl, $C_3$-C6-cycloalkyl or fluorinated $C_3$-$C_6$-cycloalkyl, can be prepared from compounds I or I' wherein

$R^4$, $R^5$, $R^6$ or $R^7$ (in case that X is $CR^7$) is Cl, Br or I and wherein the secondary nitrogen atom carries a protective group by Suzuki coupling with the respective organoboronic acid in the presence of a Pd catalyst and a base. Suitable Pd catalysts and bases are those mentioned in context with scheme 1.

Compounds I or I' wherein $R^4$, $R^5$, $R^6$ or $R^7$ (in case that X is $CR^7$) is -C=CH can be prepared from compounds I or I' wherein $R^4$, $R^5$, $R^6$ or $R^7$ (in case that X is $CR^7$) is Cl, Br or I by reaction with trimethylsilylacetylene in the presence of a Pd catalyst and a rather weak base in order to avoid removal of the TMS group, such as a carbonate, e.g. sodium, potassium or caesium carbonate. Suitable Pd catalysts are those mentioned in context with scheme 1. Removal of the TMS group, e.g. with a strong base, such a hydroxide, e.g. NaOH or KOH, yields the free acetylene group.

Compounds I or I' wherein $R^4$, $R^5$, $R^6$ or $R^7$ (in case that X is $CR^7$) is H can be prepared from compounds I or I' wherein $R^4$, $R^5$, $R^6$ or $R^7$ (in case that X is $CR^7$) is Cl by a hydrogenation reaction in the presence of a hydrogenation catalyst, such as platinum, palladium, rhodium, ruthenium or nickel; especially Pd, e.g. Pd/C.

Compounds I or I' wherein $R^4$, $R^5$, $R^6$ or $R^7$ (in case that X is $CR^7$) is CN can be prepared from compounds I or I' wherein $R^4$, $R^5$, $R^6$ or $R^7$ (in case that X is $CR^7$) is Cl, Br or I and wherein the secondary nitrogen atom carries a protective group by reaction with dicyanozinc in the presence of a Pd catalyst, e.g. one oft he Pd catalysts mentioned in context with scheme 1.

For obtaining compounds I with a specific configuration at the 6-position (i.e. at the carbon ring atom carrying the $-[-C(R^{2a})(R^{3a})]_n-C(R^{2b})(R^{3b})-OR^1$ substituent), diastereomeric separation at the level of compound 3 is expedient. Subsequent reaction steps, such as shown in schemes 2 and 1, is then carried out starting from the desired enantiomer of 3.

If not otherwise indicated, the above-described reactions are generally carried out in a solvent at temperatures between room temperature and the boiling temperature of the solvent employed. Alternatively, the activation energy which is required for the reaction can be introduced into the reaction mixture using microwaves, something which has proved to be of value, in particular, in the case of the reactions catalyzed by transition metals (with regard to reactions using microwaves, see Tetrahedron 2001, 57, p. 9199 ff. p. 9225 ff. and also, in a general manner, "Microwaves in Organic Synthesis", André Loupy (Ed.), Wiley-VCH 2002).

The acid addition salts of compounds I are prepared in a customary manner by mixing the free base with a corresponding acid, where appropriate in solution in an organic solvent, for example a lower alcohol, such as methanol, ethanol or propanol, an ether, such as methyl tert-butyl ether or diisopropyl ether, a ketone, such as acetone or methyl ethyl ketone, or an ester, such as ethyl acetate.

The N-oxides of compound I may be prepared from the compounds of formula I according to conventional oxidation methods, for example by treating said compounds with an organic peracid; such as metachloroperbenzoic acid (3-chloroperbenzoic acid) [Journal of Medicinal Chemistry 38(11), 1892-1903 (1995), WO 03/64572]; or with inorganic oxidizing agents; such as hydrogen peroxide [cf. Journal of Heterocyclic Chemistry 18 (7), 1305-1308 (1981)] or oxone [cf. Journal of the American Chemical Society 123(25), 5962-5973 (2001)]. The oxidation may lead to pure mono-N-oxides or to a mixture of different N-oxides, which can be separated by conventional methods; such as chromatography.

Routine experimentations, including appropriate manipulation of the reaction conditions, reagents and sequence of the synthetic route, protection of any chemical functionality that may not be compatible with the reaction conditions, and deprotection at a suitable point in the reaction sequence of the preparation methods are within routine techniques.

Suitable protecting groups and the methods for protecting and deprotecting different substituents using such suitable protecting groups are well known to those skilled in the art; examples of which may be found in T. Greene and P. Wuts, Protective Groups in Organic Synthesis (3rd ed.), John Wiley & Sons, NY (1999), which is herein incorporated by reference in its entirety. Synthesis of the compounds of the invention may be accomplished by methods analogous to those described in the synthetic schemes described hereinabove and in specific examples.

Starting materials, if not commercially available, may be prepared by procedures selected from standard organic chemical techniques, techniques that are analogous to the synthesis of known, structurally similar compounds, or techniques that are analogous to the above described schemes or the procedures described in the synthetic examples section.

When an optically active form of a compound of the invention is required, it may be obtained by carrying out one of the procedures described herein using an optically active starting material (prepared, for example, by asymmetric induction of a suitable reaction step or by using chiral pool), or by resolution of a mixture of the stereoisomers of the compound or intermediates using a standard procedure (such as chromatographic separation, recrystallization or enzymatic resolution).

Similarly, when a pure geometric isomer of a compound of the invention is required, it may be obtained by carrying out one of the above procedures using a pure geometric isomer as a starting material, or by resolution of a mixture of the geometric isomers of the compound or intermediates using a standard procedure such as chromatographic separation.

The present invention moreover relates to compounds of formula I as defined above, wherein at least one of the atoms has been replaced by its stable, non-radioactive isotope (e.g., hydrogen by deuterium, $^{12}C$ by $^{13}C$, $^{14}N$ by $^{15}N$, $^{16}O$ by $^{18}O$) and preferably wherein at least one hydrogen atom has been replaced by a deuterium atom.

Of course, the unlabeled compounds according to the invention might naturally include certain amounts of these respective isotopes. Therefore, when referring to compounds I, wherein at least one of the atoms has been replaced by its stable, non-radioactive isotope, it will be understood that the isotope is present in a higher amount than would naturally occur.

Stable isotopes (e.g., deuterium, $^{13}C$, $^{15}N$, $^{18}O$) are nonradioactive isotopes which contain one additional neutron than the normally abundant isotope of the respective atom. Deuterated compounds have been used in pharmaceutical research to investigate the in vivo metabolic fate of the compounds by evaluation of the mechanism of action and metabolic pathway of the non deuterated parent compound (Blake et al. J. Pharm. Sci. 64, 3, 367-391 (1975)). Such metabolic studies are important in the design of safe, effective therapeutic drugs, either because the in vivo active compound administered to the patient or because the metabolites produced from the parent compound prove to be toxic or carcinogenic (Foster et al., Advances in Drug Research Vol. 14, pp. 2-36, Academic press, London, 1985; Kato et al., J. Labelled Comp. Radiopharmaceut., 36(10):927-932 (1995); Kushner et al., Can. J. Physiol. Pharmacol., 77, 79-88 (1999).

Incorporation of a heavy atom, particularly substitution of deuterium for hydrogen, can give rise to an isotope effect that could alter the pharmacokinetics of the drug.

Stable isotope labeling of a drug can alter its physico-chemical properties such as pKa and lipid solubility. These changes may influence the fate of the drug at different steps along its passage through the body. Absorption, distribution, metabolism or excretion can be changed. Absorption and distribution are processes that depend primarily on the molecular size and the lipophilicity of the substance. These effects and alterations can affect the pharmacodynamic response of the drug molecule if the isotopic substitution affects a region involved in a ligand-receptor interaction.

Drug metabolism can give rise to large isotopic effect if the breaking of a chemical bond to a deuterium atom is the rate limiting step in the process. While some of the physical properties of a stable isotope-labeled molecule are different from those of the unlabeled one, the chemical and biological properties are the same, with one important exception: because of the increased mass of the heavy isotope, any bond involving the heavy isotope and another atom will be stronger than the same bond between the light isotope and that atom. In any reaction in which the breaking of this bond is the rate limiting step, the reaction will proceed slower for the molecule with the heavy isotope due to "kinetic isotope effect". A reaction involving breaking a C-D bond can be up to 700 percent slower than a similar reaction involving breaking a C-H bond. If the C-D bond is not involved in any of the steps leading to the metabolite, there may not be any effect to alter the behavior of the drug. If a deuterium is placed at a site involved in the metabolism of a drug, an isotope effect will be observed only if breaking of the C-D bond is the rate limiting step. There is evidence to suggest that whenever cleavage of an aliphatic C-H bond occurs, usually by oxidation catalyzed by a mixed-function oxidase, replacement of the hydrogen by deuterium will lead to observable isotope effect. It is also important to understand that the incorporation of deuterium at the site of metabolism slows its rate to the point where another metabolite produced by attack at a carbon atom not substituted by deuterium becomes the major pathway a process called "metabolic switching".

Deuterium tracers, such as deuterium-labeled drugs and doses, in some cases repeatedly, of thousands of milligrams of deuterated water, are also used in healthy humans of all ages, including neonates and pregnant women, without reported incident (e.g. Pons G and Rey E, Pediatrics 1999 104: 633; Coward W A et al., Lancet 1979 7: 13; Schwarcz H P, Control. Clin. Trials 1984 5(4 Suppl): 573; Rodewald L E et al., J. Pediatr. 1989 114: 885; Butte N F et al. Br. J. Nutr. 1991 65: 3; MacLennan A H et al. Am. J. Obstet Gynecol. 1981 139: 948). Thus, it is clear that any deuterium released, for instance, during the metabolism of compounds of this invention poses no health risk.

The weight percentage of hydrogen in a mammal (approximately 9%) and natural abundance of deuterium (approximately 0.015%) indicates that a 70 kg human normally contains nearly a gram of deuterium. Furthermore, replacement of up to about 15% of normal hydrogen with deuterium has been effected and maintained for a period of days to weeks in mammals, including rodents and dogs, with minimal observed adverse effects (Czajka D M and Finkel A J, Ann. N.Y. Acad. Sci. 1960 84: 770; Thomson J F, Ann. New York Acad. Sci 1960 84: 736; Czakja D M et al., Am. J. Physiol. 1961 201: 357). Higher deuterium concentrations, usually in excess of 20%, can be toxic in animals. However, acute replacement of as high as 15%-23% of the hydrogen in humans' fluids with deuterium was found not to cause toxicity (Blagojevic N et al. in "Dosimetry & Treatment Planning for Neutron Capture Therapy", Zamenhof R, Solares G and Harling O Eds. 1994. Advanced Medical Publishing, Madison Wis. pp.125-134; Diabetes Metab. 23: 251 (1997)).

Increasing the amount of deuterium present in a compound above its natural abundance is called enrichment or deuterium-enrichment. Examples of the amount of enrichment include from about 0.5, 1, 2, 3, 4, 5, 6, 7, 8, 9, 10, 12, 16, 21, 25, 29, 33, 37, 42, 46, 50, 54, 58, 63, 67, 71, 75, 79, 84, 88, 92, 96, to about 100 mol %.

The hydrogens present on a particular organic compound have different capacities for exchange with deuterium. Certain hydrogen atoms are easily exchangeable under physiological conditions and, if replaced by deuterium atoms, it is expected that they will readily exchange for protons after administration to a patient. Certain hydrogen atoms may be exchanged for deuterium atoms by the action of a deuteric acid such as $D_2SO_4/D_2O$. Alternatively, deuterium atoms may be incorporated in various combinations during the synthesis of compounds of the invention. Certain hydrogen

atoms are not easily exchangeable for deuterium atoms. However, deuterium atoms at the remaining positions may be incorporated by the use of deuterated starting materials or intermediates during the construction of compounds of the invention.

Deuterated and deuterium-enriched compounds of the invention can be prepared by using known methods described in the literature. Such methods can be carried out utilizing corresponding deuterated and optionally, other isotope-containing reagents and/or intermediates to synthesize the compounds delineated herein, or invoking standard synthetic protocols known in the art for introducing isotopic atoms to a chemical structure. Relevant procedures and intermediates are disclosed, for instance in Lizondo, J et al., Drugs Fut, 21(11), 1116 (1996); Brickner, S J et al., J Med Chem, 39(3), 673 (1996); Mallesham, B et al., Org Lett, 5(7), 963 (2003); PCT publications WO1997010223, WO2005099353, WO1995007271, WO2006008754; US Patent Nos. 7538189; 7534814; 7531685; 7528131; 7521421; 7514068; 7511013; and US Patent Application Publication Nos. 20090137457; 20090131485; 20090131363; 20090118238; 20090111840; 20090105338; 20090105307; 20090105147; 20090093422; 20090088416; 20090082471, the methods are hereby incorporated by reference.

The present invention further relates to a pharmaceutical composition comprising a therapeutically effective amount of at least one compound I as defined above or an N-oxide, a tautomeric form, a stereoisomer or a pharmaceutically acceptable salt thereof, in combination with at least one pharmaceutically acceptable carrier and/or auxiliary substance; or comprising at least one compound I wherein at least one of the atoms has been replaced by its stable, non-radioactive isotope, preferably wherein at least one hydrogen atom has been replaced by a deuterium atom, in combination with at least one pharmaceutically acceptable carrier and/or auxiliary substance.

The present invention further relates to a compound I as defined above or an N-oxide, a tautomeric form, a stereoisomer or a pharmaceutically acceptable salt thereof for use as a medicament.

The present invention also relates to a compound I as defined above or an N-oxide, a tautomeric form, a stereoisomer or a pharmaceutically acceptable salt thereof for use in the treatment of disorders which respond to the modulation of the 5-$HT_{2C}$ receptor.

The present invention also relates to the use of a compound I as defined above or of an N-oxide, a tautomeric form, a stereoisomer or a pharmaceutically acceptable salt thereof for the manufacture of a medicament for the treatment of disorders which respond to the modulation of the 5-$HT_{2C}$ receptor, and to a method for treating disorders which respond to the modulation of the 5-$HT_{2C}$ receptor, which method comprises administering to a subject in need thereof at least one compound I as defined above or an N-oxide, a tautomeric form, a stereoisomer or a pharmaceutically acceptable salt thereof.

The compounds of the present invention are modulators of the 5-$HT_{2C}$ receptor. Specifically, the compounds of formula I are agonists or partial agonists of the 5-$HT_{2C}$ receptor. Thus, in a specific embodiment, the invention relates to a compound I as defined above or an N-oxide, a tautomeric form, a stereoisomer or a pharmaceutically acceptable salt thereof for use in the treatment of disorders which respond to 5-$HT_{2C}$ receptor agonists, further to the use of a compound I as defined above or of an N-oxide, a tautomeric form, a stereoisomer or a pharmaceutically acceptable salt thereof for the manufacture of a medicament for the treatment of disorders which respond to 5-$HT_{2C}$ receptor agonists, and to a method for treating disorders which respond to 5-$HT_{2C}$ receptor agonists, which method comprises administering to a subject in need thereof at least one compound I as defined above or an N-oxide, a tautomeric form, a stereoisomer or a pharmaceutically acceptable salt thereof.

Within the meaning of the invention, the term "disorder" denotes disturbances and/or anomalies which are as a rule regarded as being pathological conditions or functions and which can manifest themselves in the form of particular signs, symptoms and/or malfunctions. While the treatment according to the invention can be directed toward individual disorders, i.e. anomalies or pathological conditions, it is also possible for several anomalies, which may be causatively linked to each other, to be combined into patterns, i.e. syndromes, which can be treated in accordance with the invention.

In one aspect of the invention, the diseases to be treated are disorders are damage of the central nervous system, disorders of the central nervous system, eating disorders, ocular hypertension, cardiovascular disorders, gastrointestinal disorders and diabetes.

Disorders or diseases of the central nervous system are understood as meaning disorders which affect the spinal cord and, in particular, the brain. These are, for example, cognitive dysfunction, attention deficit disorder/hyperactivity syndrome and cognitive deficits related with schizophrenia, attention deficit/hyperactivity syndrome, personality disorders, affective disorders, motion or motor disorders, pain, migraine, sleep disorders (including disturbances of the Circadian rhythm), feeding disorders, diseases associated with neurodegeneration, addiction diseases, obesity or psoriasis.

Examples of cognitive dysfunction are deficits in memory, cognition, and learning, Alzheimer's disease, age-related cognitive decline, and mild cognitive impairment, or any combinations thereof. Examples of personality disorders are schizophrenia and cognitive deficits related to schizophrenia. Examples of affective disorders are depression, anxiety, bipolar disorder and obsessive compulsive disorders, or any combination thereof. Examples of motion or motor disorders are Parkinson's disease and epilepsy. Examples of feeding disorders are obesity, bulimia, weight loss and anorexia,

especially anorexia nervosa. Examples of diseases associated with neurodegeneration are stroke, spinal or head trauma, and head injuries, such as hydrocephalus.

Pain condition includes nociceptive pain, neuropathic pain or a combination thereof. Such pain conditions or disorders can include, but are not limited to, postoperative pain, osteoarthritis pain, pain due to inflammation, rheumatoid arthritis pain, musculoskeletal pain, burn pain (including sunburn), ocular pain, the pain associated with dental conditions (such as dental caries and gingivitis), post-partum pain, bone fracture, herpes, HIV, traumatic nerve injury, stroke, post-ischemia, fibromyalgia, reflex sympathetic dystrophy, complex regional pain syndrome, spinal cord injury, sciatica, phantom limb pain, diabetic neuropathy, hyperalgesia and cancer.

In certain other embodiments, the disease condition is bladder dysfunction, including urinary incontinence.

Diabetes includes diabetes insipidus, diabetes mellitus, type I diabetes, type II diabetes, type III diabetes, diabetes secondary to pancreatic diseases, diabetes related to steroid use, diabetes complications, hyperglycemia, and insulin resistance.

The addiction diseases include psychiatric disorders and behavioral disturbances which are caused by the abuse of psychotropic substances, such as pharmaceuticals or narcotics, and also other addiction diseases, such as addiction to gaming (impulse control disorders not elsewhere classified). Examples of addictive substances are: opioids (e.g. morphine, heroin and codeine), cocaine; nicotine; alcohol; substances which interact with the GABA chloride channel complex, sedatives, hypnotics and tranquilizers, for example benzodiazepines; LSD; cannabinoids; psychomotor stimulants, such as 3,4-methylenedioxy-*N*-methylamphetamine (ecstasy); amphetamine and amphetamine-like substances such as methylphenidate, other stimulants including caffeine and nicotine. Addictive substances which come particularly into consideration are opioids, cocaine, amphetamine or amphetamine-like substances, nicotine and alcohol. Especially, addiction disorders include alcohol abuse, cocaine abuse, tobacco abuse and smoking cessation.

With regard to the treatment of addiction diseases, particular preference is given to those compounds according to the invention of the formula (I) which themselves do not possess any psychotropic effect. This can also be observed in a test using rats, which, after having been administered compounds which can be used in accordance with the invention, reduce their self administration of psychotropic substances, for example cocaine.

Examples of gastrointestinal disorders are irritable bowel syndrome.

Preferably, the disorders are selected from the group consisting of bipolar disorder, depression, atypical depression, mood episodes, adjustment disorders, anxiety, panic disorders, post-traumatic syndrome, psychoses, schizophrenia, cognitive deficits of schizophrenia, memory loss, dementia of aging, Alzheimer's disease, neuropsychiatric symptoms in Alzheimer's disease (e.g. aggression), behavioral disorders associated with dementia, social phobia, mental disorders in childhood, attention deficit hyperactivity disorder, organic mental disorders, autism, mutism, disruptive behavior disorder, impulse control disorder, borderline personality disorder, obsessive compulsive disorder, migraine and other conditions associated with cephalic pain or other pain, raised intracranial pressure, seizure disorders, epilepsy, substance use disorders, alcohol abuse, cocaine abuse, tobacco abuse, smoking cessation, sexual dysfunction/erectile dysfunction in males, sexual dysfunction in females, premenstrual syndrome, late luteal phase syndrome, chronic fatigue syndrome, sleep disorders, sleep apnoea, chronic fatigue syndrome, psoriasis, Parkinson's disease, psychosis in Parkinson's disease, neuropsychiatric symptoms in Parkinson's disease (e.g. aggression), Lewy Body dementia, neuropsychiatric symptoms in Lewy Body dementia (e.g. aggression), spinal cord injury, trauma, stroke, pain, bladder dysfunction/urinary incontinence, encephalitis, meningitis, eating disorders, obesity, bulimia, weight loss, anorexia nervosa, ocular hypertension, cardiovascular disorders, gastrointestinal disorders, diabetes insipidus, diabetes mellitus, type I diabetes, type II diabetes, type III diabetes, diabetes secondary to pancreatic diseases, diabetes related to steroid use, diabetes complications, hyperglycemia, and insulin resistance, and are specifically schizophrenia, depression, bipolar disorders, obesity, substance use disorders, neuropsychiatric symptoms in Alzheimer's disease (e.g. aggression), or neuropsychiatric symptoms in Parkinson's disease (e.g. aggression).

The compounds of the invention may be used for a preventive treatment (prophylaxis), in particular as relapse prophylaxis or phase prophylaxis, but are preferably used for a treatment in its proper sense (i.e. non-prophylactic), i.e. for the treatment of acute or chronic signs, symptoms and/or malfunctions. The treatment can be orientated symptomatically, for example as the suppression of symptoms. It can be effected over a short period, be orientated over the medium term or can be a long-term treatment, for example within the context of a maintenance therapy.

In another embodiment, the present invention relates to the compound I as defined above or an N-oxide, a tautomeric form, a stereoisomer or a pharmaceutically acceptable salt thereof for use in the prevention of (the development of) a disease condition as described above and to a method for preventing (the development of) a disease condition as described above; to the use of a compound I as defined above or an N-oxide, a tautomeric form, a stereoisomer or a pharmaceutically acceptable salt thereof for preparing a medicament for preventing (the development of) a disease condition as described above and to a method for preventing (the development of) a disease condition as described above which comprises administering to the subject in need thereof (e.g., a mammal, such as a human) a therapeutically effective amount of a compound I as defined above or an N-oxide, a tautomeric form, a stereoisomer or a pharmaceutically acceptable salt thereof. As used herein, the term "prevent" a disease condition by administration of any of the compounds

described herein means that the detectable physical characteristics or symptoms of the disease or condition do not develop following the administration of the compound described herein. Alternatively, the method comprises administering to the subject a therapeutically effective amount of a compound I as defined above or an N-oxide, a tautomeric form, a stereoisomer or a pharmaceutically acceptable salt thereof, in combination with a therapeutically effective amount of at least one cognitive enhancing drug.

In yet another embodiment, the present invention relates to the compound I as defined above or an N-oxide, a tautomeric form, a stereoisomer or a pharmaceutically acceptable salt thereof for use in the prevention of the progression (e.g., worsening) of a disease condition, to the use a compound I as defined above or an N-oxide, a tautomeric form, a stereoisomer or a pharmaceutically acceptable salt thereof for preparing a medicament for preventing the progression (e.g., worsening) of a disease condition and to a method for preventing the progression (e.g., worsening) of a disease condition, which method comprises administering to the subject in need of treatment thereof (e.g., a mammal, such as a human) a therapeutically effective amount of a compound I as defined above or an N-oxide, a tautomeric form, a stereoisomer or a pharmaceutically acceptable salt thereof.

There are several lines of evidence suggesting that 5-$HT_{2C}$ agonists or partial agonists would have therapeutic use in a variety of diseases, disorders and conditions.

Knockout mice models lacking the 5-$HT_{2C}$ receptor exhibit hyperphagia, obesity and are more prone to seizures and sudden death [Tecott LH, Sun LM, Akana SF, Strack AM, Lowenstein DH, Dallman MF, Julius D (1995) Eating disorder and epilepsy in mice lacking 5-HT2C serotonin receptors. Nature 374:542-546]. They also exhibit compulsive-like behavior [Chou-Green JM, Holscher TD, Dallman MF, Akana SF (2003). Compulsive behavior in the 5-HT2C receptor knockout mouse. Phys. Behav. 78:641-649], hyperresponsiveness to repeated stress [Chou-Green JM, Holscher TD, Dallman MF, Akana SF (2003). Repeated stress in young and old 5-HT2C receptor knockout mouse. Phys. Behav. 79:217-226], wakefulness [Frank MG, Stryker MP, Tecott LH (2002). Sleep and sleep homeostasis in mice lacking the 5-HT2C receptor. Neuropsychopharmacology 27:869-873], hyperactivity and drug dependence [Rocha BA, Goulding EH, O'Dell LE, Mead AN, Coufal NG, Parsons LH, Tecott LH (2002). Enhanced locomotor, reinforcing and neurochemical effects of cocaine in serotonin 5-hydroxytryptamine 2C receptor mutant mice. J. Neurosci. 22:10039-10045].

5-$HT_{2C}$ is unique among other G-protein-coupled receptors (GPCRs) in that its pre-mRNA is a substrate for base modification via hydrolytic deamination of adenosines to yield inosines. Five adenosines, located within a sequence encoding the putative second intracellular domain can be converted to inosines. This editing can alter the coding potential of the triplet codons and allows for the generation of multiple different receptor isoforms. The edited receptor isoforms were shown to have reduced ability to interact with G-proteins in the absence of agonist stimulation [Werry, TD, Loiacono R, Sexton PA, Christopoulos A (2008). RNA editing of the serotonin 5-HT2C receptor and its effects on cell signaling, pharmacology and brain function. Pharmac. Therap. 119:7-23].

Edited 5-$HT_{2C}$ isoforms with reduced function are significantly expressed in the brains of depressed suicide victims [Schmauss C (2003) Serotonin 2C receptors: suicide, serotonin, and runaway RNA editing. Neuroscientist 9:237-242. Iwamoto K, Kato T (2003). RNA editing of serotonin 2C receptor in human postmortem brains of major mental disorders. Neurosci. Lett. 346:169-172] and in the learned helplessness rats (a well established animal model of depression) [Iwamotoa K, Nakatanib N, Bundoa M, Yoshikawab T, Katoa T (2005). Altered RNA editing of serotonin 2C receptor in a rat model of depression. Neurosci. Res.53: 69-76] suggesting a link between 5-$HT_{2C}$ function and depression. There are also implications of edited 5-$HT_{2C}$ isoforms and spatial memory [Du Y, Stasko M, Costa AC, Davissone MT, Gardiner KJ (2007). Editing of the serotonin 2C receptor pre-mRNA Effects of the Morris Water Maze. Gene 391:186-197]. In addition, fully edited isoforms of the human 5-$HT_{2C}$ receptor display a striking reduction in sensitivity to lysergic acid diethylamide (LSD) and to atypical antipsychotic drugs clozapine and loxapine, suggesting a possible role of the receptor in the etiology and pharmacology of schizophrenia [Niswender CM, Herrick-Davis K,. Dilley GE, Meltzer HY, Overholser JC, Stockmeier CA, Emeson RB, Sanders-Bush E (2001). RNA Editing of the Human Serotonin 5-HT2C Receptor: Alterations in Suicide and Implications for Serotonergic Pharmacotherapy. Neuropsychopharm. 24:478-491].

Recently, the availability of potent and selective 5-$HT_{2C}$ receptor agonists made it possible to directly investigate the effects of 5-$HT_{2C}$ agonists and their therapeutic potential. Thus recent studies demonstrated that selective 5-$HT_{2C}$ agonists resulted in decreased food intake and body weight gain in normal and obese rats [Smith BM, et al. (2008). Discovery and structure-activity relationship of (1R)-8-chloro-2,3,4,5-tetrahydro-1-methyl-1H-3-benzazepine (Lorcaserin), a selective serotonin 5-HT2C receptor agonist for the treatment of obesity. J Med Chem 51:305-313. Thomsen WJ, Grottick AJ, Menzaghi F, Reyes-Saldana H, Espitia S, Yuskin D, Whelan K, Martin M, Morgan M, Chen W, Al-Shama H, Smith B, Chalmers D, Behan D (2008) Lorcaserin, A Novel Selective Human 5-HT2C Agonist: In Vitro and In Vivo Pharmacological Characterization. J Pharmacol Exp Ther. 325:577-587. Rosenzweig-Lipson S, Zhang J, Mazandarani H, Harrison BL, Sabb A, Sabalski J, Stack G, Welmaker G, Barrett JE, Dunlop J (2006) Antiobesity-like effects of the 5-HT2C receptor agonist WAY-161503. Brain Res. 1073-1074:240-251. Dunlop J, Sabb AL, Mazandarani H, Zhang J, Kalgaonker S, Shukhina E, Sukoff S, Vogel RL, Stack G, Schechter L, Harrison BL, Rosenzweig-Lipson S (2005). WAY-163909 [97bR, 10aR)-1,2,3,4,8,9,10,10a-octahydro-7bH-cyclopenta-[b][1,4]diazepino[6,7,1hi]indole], a novel 5-hydroxytryptamine 2C receptor -selective agonist with anorectic activity. J Pharmacol Exp Ther. 313:862-869.].

Furthermore, selective 5-$HT_{2C}$ receptor agonists produce antidepressant effects in animal models of depression comparable to those of SSRIs but with a much faster onset of action and a therapeutic window that avoids antidepressant-induced sexual dysfunction. These agonists were also effective in animal models of compulsive behavior such as scheduled induced polydipsia and they also exhibited decreased hyperactivity and aggression in rodents [Rosenzweig-Lipson S, Sabb A, Stack G, Mitchell P, Lucki I, Malberg JE, Grauer S, Brennan J, Cryan JF, Sukoff Rizzo SJ, Dunlop J, Barrett JE, Marquis KL (2007) Antidepressant-like effects of the novel, selective, 5-HT2C receptor agonist WAY-163909 in rodents. Psychopharmacology (Berlin) 192:159-170. Rosenzweig-Lipson S, Dunlop J, Marquis KL (2007) 5-HT2C receptor agonists as an innovative approach for psychiatric disorders. Drug news Perspect, 20: 565-571. Cryan, JF, Lucki I (2000). Antidepressant-like behavioral effects mediated by 5-Hydroxytryptamine 2C receptors. J. Pharm. Exp. Ther. 295:1120-1126.].

Acute or chronic administration of 5-$HT_{2C}$ agonists decreases the firing rate of ventral tegmental area dopamine neurons but not that of substantia nigra. In addition 5-$HT_{2C}$ agonists reduce dopamine levels in the nucleus accumbens but not in the striatum (the region of the brain mostly associated with extrapyramidal side effects) [Di Matteo, V., Di Giovanni, G., Di Mascio, M., & Esposito, E. (1999). SB 242084, a selective serotonin 2C receptor antagonist, increases dopaminergic transmission in the mesolimbic system. Neuropharmacology 38, 1195 - 1205. Di Giovanni, G., Di Matteo, V., Di Mascio, M., & Esposito, E. (2000). Preferential modulation of mesolimbic vs. nigrostriatal dopaminergic function by serotonin2C/2B receptor agonists: a combined in vivo electrophysiological and microdialysis study. Synapse 35, 53 - 61. Marquis KL, Sabb AL, Logue SF, Brennan JA, Piesla MJ, Comery TA, Grauer SM, Ashby CR, Jr., Nguyen HQ, Dawson LA, Barrett JE, Stack G, Meltzer HY, Harrison BL, Rosenzweig-Lipson S (2007) WAY-163909 [(7bR,10aR)-1,2,3,4,8,9,10,10a-octahydro-7bH-cyclopenta-[b][1,4]diazepino[6,7,1hi]indole]: A novel 5-hydroxytryptamine 2C receptor-selective agonist with preclinical antipsychotic-like activity. J Pharmacol Exp Ther 320:486-496.]. Therefore it is expected that 5-$HT_{2C}$ receptor agonists will selectively decrease mesolimibic dopamine levels without affecting the nigrostriatal pathway thus avoiding the EPS side effects of typical antipsychotics. Several 5-$HT_{2C}$ receptor agonists have shown antipsychotic activity in animal models of schizophrenia without EPS based on the lack of effect in catalepsy [Marquis KL, Sabb AL, Logue SF, Brennan JA, Piesla MJ, Comery TA, Grauer SM, Ashby CR, Jr., Nguyen HQ, Dawson LA, Barrett JE, Stack G, Meltzer HY, Harrison BL, Rosenzweig-Lipson S (2007) WAY-163909 [(7bR,10aR)-1,2,3,4,8,9,10,10a-octahydro-7bH-cyclopenta-[b][1,4]diazepino[6,7,1hi]indole]: A novel 5-hydroxytryptamine 2C receptor-selective agonist with preclinical antipsychotic-like activity. J Pharmacol Exp Ther 320:486-496. Siuciak JA, Chapin DS, McCarthy SA, Guanowsky V, Brown J, Chiang P, Marala R, Patterson T, Seymour PA, Swick A, Iredale PA (2007) CP-809,101, a selective 5-HT2C agonist, shows activity in animal models of antipsychotic activity. Neuropharmacology 52:279-290]. The antipsychotic activity of 5-$HT_{2C}$ receptor agonists without EPS coupled with their beneficial effects in mood disorders and cognition and their antiobesity like effects render 5-$HT_{2C}$ receptor agonists as unique agents to treat schizophrenia [Rosenzweig-Lipson S, Dunlop J, Marquis KL (2007) 5-HT2C receptor agonists as an innovative approach for psychiatric disorders. Drug news Perspect, 20: 565-571. Dunlop J, Marquis KL, Lim HK, Leung L, Kao J, Cheesman C, Rosenzweig-Lipson S (2006). Pharmacological profile of the 5-HT2C receptor agonist WAY-163909; therapeutic potential in multiple indications. CNS Dug Rev. 12:167-177.].

In addition 5-$HT_{2C}$ modulation has been implicated in epilepsy [Isaac M (2005). Serotonergic 5-HT2C receptors as a potential therapeutic target for the antiepileptic drugs. Curr. Topics Med. Chem. 5:59:67], psoriasis [Thorslund K, Nordlind K (2007). Serotonergic drugs-a possible role in the treatment of psoriasis? Drug News Perspect 20:521-525], Parkinson's disease and related motor disorders [Esposito E, Di Matteo V, Pierucci M, Benigno A, Di Giavanni, G (2007). Role of central 5-HT2C receptor in the control of basal ganglia functions. The Basal Ganglia Pathophysiology: Recent Advances 97-127], behavioral deficits [Barr AM, Lahmann-Masten V, Paulus M, Gainetdinov RP, Caron MG, Geyer MA (2004). The selective serotonin-2A receptor antagonist M100907 reverses behavioral deficits in dopamine transporter knockout mice. Neuropsychopharmacology 29:221-228], anxiety [Dekeyne A, Mannoury la Cour C, Gobert A, Brocco M, Lejuene F, Serres F, Sharp T, Daszuta A, Soumier A, Papp M, Rivet JM, Flik G, Cremers TI, Muller O, Lavielle G, Millan MJ (2208). S32006, a novel 5-HT2C receptor antagonists displaying broad-based antidepressant and anxiolytic properties in rodent models. Psychopharmacology 199:549-568. Nunes-de-Souza V, Nunes-de-Souza RL, Rodgers RJ, Canto-de-Souza A (2008). 5-HT2 receptor activation in the midbrain periaqueductal grey (PAG) reduces anxiety-like behavior in mice. Behav. Brain Res. 187:72-79.], migraine [Leone M, Rigamonti A, D'Amico D, Grazzi L, Usai S, Bussone G (2001). The serotonergic system in migraine. Journal of Headache and Pain 2(Suppl. 1):S43-S46], Alzheimer's disease [Arjona AA, Pooler AM, Lee RK, Wurtman RJ (2002). Effect of a 5-HT2C serotonin agonist, dexnorfenfluramine, on amyloid precursor protein metabolism in guinea pigs. Brain Res. 951:135-140], pain and spinal cord injury [Nakae A, Nakai K, Tanaka T, Hagihira S, Shibata M, Ueda K, Masimo T (2008). The role of RNA editing of the serotonin 2C receptor in a rat model of oro-facial neuropathic pain. The European Journal of Neuroscience 27:2373-2379. Nakae A, Nakai K, Tanaka T, Takashina M, Hagihira S, Shibata M, Ueda K, Mashimo T (2008). Serotonin 2C receptor mRNA editing in neuropathic pain model. Neurosci. Res. 60:228-231. Kao T, Shumsky JS, Jacob-Vadakot S, Timothy HB, Murray M, Moxon, KA (2006). Role of the 5-HT2C receptor in improving weight-supported stepping in adult rats spinalized as neonates. Brain Res.1112:159-168.], sexual dysfunction [Motofei IG (2008). A dual physiological character for sexual

function: the role of serotonergic receptors. BJU International 101:531-534. Shimada I, Maeno K, Kondoh Y, Kaku H, Sugasawa K, Kimura Y, Hatanaka K,; Naitou Y, Wanibuchi F, Sakamoto S,; Tsukamoto S (2008). Synthesis and structure-activity relationships of a series of benzazepine derivatives as 5-HT2C receptor agonists. Bioorg. Med. Chem. 16:3309-3320.], smoking cessation [Fletcher PJ, Le AD, Higgins GA (2008). Serotonin receptors as potential targets for modulation of nicotine use and dependence. Progress Brain Res. 172:361-83], substance dependence [Bubar MJ, Cunningham KA (2008). Prospects for serotonin 5-HT2R pharmacotherapy in psychostimulant abuse. Progress Brain Res. 172:319-46], and ocular hypertension [Sharif NA, McLaughlin MA, Kelly CR (2006). AL-34662: a potent, selective, and efficacious ocular hypotensive serotonin-2 receptor agonist. J Ocul Pharmacol Ther. 23:1-13].

Further, 5HT modulation can be useful in the treatment of pain, both neuropathic and nociceptive pain, see for example U.S. Patent application publication US2007/0225277. Obata, Hideaki; Ito, Naomi; Sasaki, Masayuki; Saito, Shigeru; Goto, Fumio. Possible involvement of spinal noradrenergic mechanisms in the antiallodynic effect of intrathecally administered 5 - HT2C receptor agonists in the rats with peripheral nerve injury. European Journal of Pharmacology (2007), 567(1-2), 89-94. Serotonin2C receptor mRNA editing in neuropathic pain model. Nakae, Aya; Nakai, Kunihiro; Tanaka, Tatsuya; Takashina, Masaki; Hagihira, Satoshi; Shibata, Masahiko; Ueda, Koichi; Mashimo, Takashi. Department of Anesthesiology & Intensive Care Medicine, Graduate School of Medicine, Osaka University, Neuroscience Research (Amsterdam, Netherlands) (2008), 60(2), 228-231. Antiallodynic effects of intrathecally administered 5 - HT2C receptor agonists in rats with nerve injury. Obata, Hideaki; Saito, Shigeru; Sakurazawa, Shinobu; Sasaki, Masayuki; Usui, Tadashi; Goto, Fumio. Department of Anesthesiology, Gunma University Graduate School of Medicine, Maebashi, Gunma, Japan. Pain (2004), 108(1-2), 163-169. Influence of 5 ,7-dihydroxytryptamine (5 ,7-DHT) on the antinociceptive effect of serotonin (5 -HT) 5 - HT2C receptor agonist in male and female rats. Brus, Ryszard; Kasperska, Alicja; Oswiecimska, Joanna; Szkilnik, Ryszard. Department of Pharmacology, Silesian Medical University, Zabrze, Pol. Medical Science Monitor (1997), 3(5), 654-656.

Modulation of 5HT2 receptors may be beneficial in the treatment of conditions related to bladder function, in particular, urinary incontinence. [Discovery of a novel azepine series of potent and selective 5 - HT2C agonists as potential treatments for urinary incontinence. Brennan, Paul E.; Whitlock, Gavin A.; Ho, Danny K. H.; Conlon, Kelly; McMurray, Gordon. Bioorganic & Medicinal Chemistry Letters (2009), 19(17), 4999-5003. Investigation of the role of 5 -HT2 receptor subtypes in the control of the bladder and the urethra in the anesthetized female rat. Mbaki, Y.; Ramage, A. G. Department of Pharmacology, University College London, London, UK. British Journal of Pharmacology (2008), 155(3), 343-356.] In particular, compounds with agonist activity at 5-$HT_{2C}$ have been shown to be useful in treating urinary incontinence, see for example U.S. Patent application publications US2008/0146583 and US 2007/0225274.

Further pre-clinical data suggest that 5-$HT_{2C}$ agonists could be useful for the treatment of a number of psychiatric diseases, including schizophrenia, bipolar disorders, depression/anxiety, substance use disorders and especially disorders like neuropsychiatric symptoms in Alzheimer's disease: Aggression, psychosis/ agitation represent key unmet medical needs. Clinical (Shen JHQ et al., A 6-week randomized, double-blind, placebo-controlled, comparator referenced trial of vabicaserin in acute schizophrenia. Journal of Psychiatric Research 53 (2014) 14-22; Liu J et al., Prediction of Efficacy of Vabicaserin, a 5-HT2C Agonist, for the Treatment of Schizophrenia Using a Quantitative Systems Pharmacology Model. CPT Pharmacometrics Syst. Pharmacol. (2014) 3, e111;) and preclinical data (Dunlop J et al., Characterization of Vabicaserin (SCA-136), a Selective 5-Hydroxytryptamine 2C Receptor Agonist. J Pharmacol Exp Ther (2011) 337, 673-80; Siuciak J et al., CP-809,101, a selective 5-HT2C agonist, shows activity in animal models of antipsychotic activity. Neuropharmacology 52 (2007) 279-290; Mosienko V et al., Exaggerated aggression and decreased anxiety in mice deficient in brain serotonin. Transl Psychiatry (2012) 2, e122; Del Guidice T et al., Stimulation of 5-HT2C Receptors Improves Cognitive Deficits Induced by Human Tryptophan Hydroxylase2 Loss of Function Mutation. Neuropsychopharmacology (2014) 39, 1125-1134; Rosenzweig-Lipson et al., Antidepressant-like effects of the novel, selective, 5-HT2C receptor agonist WAY-163909 in rodents. Psychopharmacology (2007) 192:159-170) suggest 5-$HT_{2C}$ receptor stimulation to result in therapeutic efficacy in aggression, psychosis agitation and moderate pro-cognitive effects (Del Guidice T et al., Stimulation of 5-HT2C Receptors Improves Cognitive Deficits Induced by Human Tryptophan Hydroxylase2 Loss of Function Mutation. Neuropsychopharmacology (2014) 39, 1125-1134; Siuciak J et al., CP-809,101, a selective 5-HT2C agonist, shows activity in animal models of antipsychotic activity. Neuropharmacology 52 (2007) 279-290).

In the use and the method of the invention, an effective quantity of one or more compounds, as a rule formulated in accordance with pharmaceutical and veterinary practice, is administered to the individual to be treated, preferably a mammal, in particular a human being, productive animal or domestic animal. Whether such a treatment is indicated, and in which form it is to take place, depends on the individual case and is subject to medical assessment (diagnosis) which takes into consideration signs, symptoms and/or malfunctions which are present, the risks of developing particular signs, symptoms and/or malfunctions, and other factors.

Actual dosage levels of active ingredients in the pharmaceutical compositions of the present invention can be varied so as to obtain an amount of the active compound(s) that is effective to achieve the desired therapeutic response for a particular subject (e.g., a mammal, preferably, a human (patient)), compositions and mode of administration. The

selected dosage level will depend upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved.

Compounds of the present invention can also be administered to a subject as a pharmaceutical composition comprising the compounds of interest in combination with at least one pharmaceutically acceptable carrier. The phrase "therapeutically effective amount" of the compound of the present invention means a sufficient amount of the compound to treat disorders, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well-known in the medical arts. For example, it is well within the skill of the art to start doses of the compound at levels lower than required to achieve the desired therapeutic effect and to gradually increase the dosage until the desired effect is achieved.

The total daily dose of the compounds of this invention administered to a subject (namely, a mammal, such as a human) ranges from about 0.01 mg/kg body weight to about 100 mg/kg body weight. More preferable doses can be in the range of from about 0.01 mg/kg body weight to about 30 mg/kg body weight. If desired, the effective daily dose can be divided into multiple doses for purposes of administration. Consequently, single dose compositions may contain such amounts or submultiples thereof to make up the daily dose.

In one aspect, the present invention provides pharmaceutical compositions. The pharmaceutical compositions of the present invention comprise the compounds of the present invention or an N-oxide, a tautomeric form, a stereoisomer or a pharmaceutically acceptable salt or solvate thereof. The pharmaceutical compositions of the present invention comprise compounds of the present invention that can be formulated together with at least one non-toxic pharmaceutically acceptable carrier.

In yet another embodiment, the present invention provides a pharmaceutical composition comprising compounds of the present invention or an N-oxide, a tautomeric form, a stereoisomer or a pharmaceutically acceptable salt thereof, and one or more pharmaceutically acceptable carriers, alone or in combination with one or more compounds that are not the compounds of the present invention. Examples of one or more compounds that can be combined with the compounds of the present invention in pharmaceutical compositions, include, but are not limited to, one or more cognitive enhancing drugs.

The pharmaceutical compositions of this present invention can be administered to a subject (e.g., a mammal, such as a human) orally, rectally, parenterally, intracisternally, intravaginally, intraperitoneally, topically (as by powders, ointments or drops), bucally or as an oral or nasal spray. The term "parenterally" as used herein, refers to modes of administration which include intravenous, intramuscular, intraperitoneal, intrasternal, subcutaneous and intraarticular injection and infusion.

The term "pharmaceutically acceptable carrier" as used herein, means a non-toxic, inert solid, semi-solid or liquid filler, diluent, encapsulating material or formulation auxiliary of any type. Some examples of materials which can serve as pharmaceutically acceptable carriers are sugars such as, but not limited to, lactose, glucose and sucrose; starches such as, but not limited to, corn starch and potato starch; cellulose and its derivatives such as, but not limited to, sodium carboxymethyl cellulose, ethyl cellulose and cellulose acetate; powdered tragacanth; malt; gelatin; talc; excipients such as, but not limited to, cocoa butter and suppository waxes; oils such as, but not limited to, peanut oil, cottonseed oil, safflower oil, sesame oil, olive oil, corn oil and soybean oil; glycols; such a propylene glycol; esters such as, but not limited to, ethyl oleate and ethyl laurate; agar; buffering agents such as, but not limited to, magnesium hydroxide and aluminum hydroxide; alginic acid; pyrogen-free water; isotonic saline; Ringer’s solution; ethyl alcohol, and phosphate buffer solutions, as well as other non-toxic compatible lubricants such as, but not limited to, sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Pharmaceutical compositions of the present invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), vegetable oils (such as olive oil), injectable organic esters (such as ethyl oleate) and suitable mixtures thereof. Proper fluidity can be maintained, for example, by the use of coating materials such as lecithin, by the maintenance of the required particle size in the case of dispersions and by the use of surfactants.

These compositions may also contain adjuvants such as preservatives, wetting agents, emulsifying agents and dispersing agents. Prevention of the action of microorganisms can be ensured by the inclusion of various antibacterial and antifungal agents, for example, paraben, chlorobutanol, phenol sorbic acid and the like. It may also be desirable to include isotonic agents such as sugars, sodium chloride and the like. Prolonged absorption of the injectable pharmaceutical form can be brought about by the inclusion of agents which delay absorption such as aluminum monostearate and gelatin.

In some cases, in order to prolong the effect of the drug, it is desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Solid dosage forms for oral administration include capsules, tablets, pills, powders and granules. In such solid dosage forms, the active compound may be mixed with at least one inert, pharmaceutically acceptable excipient or carrier, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such carriers as lactose or milk sugar as well as high molecular weight polyethylene glycols and the like.

The solid dosage forms of tablets, dragees, capsules, pills and granules can be prepared with coatings and shells such as enteric coatings and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned carriers.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, tragacanth and mixtures thereof.

Compositions for rectal or vaginal administration are preferably suppositories which can be prepared by mixing the compounds of this invention with suitable nonirritating carriers or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multi-lamellar hydrated liquid crystals which are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients and the like. The

preferred lipids are natural and synthetic phospholipids and phosphatidyl cholines (lecithins) used separately or together.

Methods to form liposomes are known in the art. See, for example, Prescott, Ed., Methods in Cell Biology, Volume XIV, Academic Press, New York, N.Y. (1976), p. 33 et seq.

Dosage forms for topical administration of a compound of the present invention include powders, sprays, ointments and inhalants. The active compound may be mixed under sterile conditions with a pharmaceutically acceptable carrier and any needed preservatives, buffers or propellants which may be required. Ophthalmic formulations, eye ointments, powders and solutions are also contemplated as being within the scope of this invention.

The compounds of the present invention can be used in the form of pharmaceutically acceptable salts derived from inorganic or organic acids. The phrase "pharmaceutically acceptable salt" means those salts which are, within the scope of sound medical judgment, suitable for use in contact with the tissues of humans and lower animals without undue toxicity, irritation, allergic response and the like and are commensurate with a reasonable benefit/risk ratio.

For example, S. M. Berge et al. describe pharmaceutically acceptable salts in detail in (J. Pharmaceutical Sciences, 1977, 66: 1 et seq.). The salts can be prepared in situ during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethansulfonate (isothionate), lactate, malate, maleate, methanesulfonate, nicotinate, 2-naphthalenesulfonate, oxalate, palmitoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quaternized with such agents as lower alkyl halides such as, but not limited to, methyl, ethyl, propyl, and butyl chlorides, bromides and iodides; dialkyl sulfates like dimethyl, diethyl, dibutyl and diamyl sulfates; long chain halides such as, but not limited to, decyl, lauryl, myristyl and stearyl chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulfuric acid, and phosphoric acid and such organic acids as acetic acid, fumaric acid, maleic acid, 4-methylbenzenesulfonic acid, succinic acid and citric acid.

Basic addition salts can be prepared in situ during the final isolation and purification of compounds of this invention by reacting a carboxylic acid-containing moiety with a suitable base such as, but not limited to, the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as, but not limited to, lithium, sodium, potassium, calcium, magnesium and aluminum salts and the like and nontoxic quaternary ammonia and amine cations including ammonium, tetramethylammonium, tetraethylammonium, methylammonium, dimethylammonium, trimethylammonium, triethylammonium, diethylammonium, ethylammonium and the like. Other representative organic amines useful for the formation of base addition salts include ethylenediamine, ethanolamine, diethanolamine, piperidine, piperazine and the like.

The compounds of the present invention can exist in unsolvated as well as solvated forms, including hydrated forms, such as hemi-hydrates. In general, the solvated forms, with pharmaceutically acceptable solvents such as water and ethanol among others are equivalent to the unsolvated forms for the purposes of the invention.

The following examples serve to explain the invention without limiting it.

## Examples

### Abbreviations

| | |
|---|---|
| EtOAc | ethyl acetate |
| DMF | dimethylformamide |
| DMSO | dimethylsulfoxide |
| THF | tetrahydrofuran |
| MeOH | methanol |
| $Et_2O$ | diethylether |
| MTBE | methyl-tert-butylether |
| DCM | dichloromethane |
| DCE | dichloroethane |
| ACN | acetonitrile |
| TFA | trifluoroacetic acid |

| | |
|---|---|
| HOBT | 1-hydroxybenzotriazole |
| EDCI | 1-ethyl-3-(3-dimethylaminopropyl)carbodiimide |
| Boc/boc/BOC | tert-butoxycarbonyl |
| DBU | 1,8-diazabicyclo[5.4.0]undec-7-en |
| DIAD | diisopropyl azodicarboxylate |
| LAH | lithium aluminium hydride |
| DAST | diethylaminosulfur trifluoride |
| KOtBu | potassium tert-butanolate |
| TLC | thin layer chromatography |
| sat | saturated |
| aq | aqueous |
| RT | room temperature (20-25°C) |
| h | hour(s) |
| min | minute(s) |

The compounds were either characterized via proton-NMR in deuterium oxide, $d_6$-dimethylsulfoxide, d-chloroform or $d_4$-methanol on a 400 MHz, 500 MHz or 600 MHz NMR instrument (Bruker AVANCE), or by $^{13}$C-NMR at 125 MHz, or by $^{19}$F-NMR at 470 MHz, or by mass spectrometry, generally recorded via HPLC-MS in a fast gradient on C18-material (electrospray-ionisation (ESI) mode).

The magnetic nuclear resonance spectral properties (NMR) refer to the chemical shifts ($\delta$) expressed in parts per million (ppm). The relative area of the shifts in the $^1$H-NMR spectrum corresponds to the number of hydrogen atoms for a particular functional type in the molecule. The nature of the shift, as regards multiplicity, is indicated as singlet (s), broad singlet (br s), doublet (d), broad doublet (br d), triplet (t), broad triplet (br t), quartet (q), quintet (quint.), multiplet (m), doublet of doublets (dd), doublet of doublets of doublets (ddd), triplet of doublets (td), doublet of triplets of doublets (dtd), doublet of triplets of triplets (dtt), quartet of doublets of doublets (qdd) etc..

I. Synthetic examples

I.1 Synthesis of intermediate compounds

i-1) (R)-2-Amino-4-methoxy-4-oxobutanoic acid

73.7 ml of acetyl chloride (1.4 eq.) was added dropwise to methanol (250 ml) at 0 °C. The mixture was added dropwise to a suspension of 100 g (751 mmol) (R)-2-aminosuccinic acid in MeOH (250 ml) at 0 °C. After stirring for 3 h, the mixture was warmed to room temperature and stirred overnight. Five additional vials were set up as described above. All six reaction mixtures were combined and concentrated under reduce pressure. The residue was treated with MTBE and the precipitate that formed was filtered and dried under reduce pressure. (R)-2-amino-4-methoxy-4-oxobutanoic acid was obtained as a white solid (600 g, 90 %).
$^1$H NMR (400 MHz, Deuterium oxide): $\delta$ 4.33 (t, J=5.5 Hz, 1H), 3.81 (s, 1H), 3.72 (s, 3H), 3.10 (d, J=4.9 Hz, 2H).

i-2) (R)-2-((tert-Butoxycarbonyl)amino)-4-methoxy-4-oxobutanoic acid

The mixture of 100 g (545 mmol) (R)-2-amino-4-methoxy-4-oxobutanoic acid of example i-1) in water and dioxane (2000 ml, v/v 1:1) was adjusted to pH 9 with solid $Na_2CO_3$ under cooling with ice-water. Then, 238 g di-tert-butyl dicarbonate (2.0 eq.) was added portionwise to the above solution. The mixture was stirred at 0 °C for 1 h and at room temperature overnight. Three additional vials were set up as described above. All four reaction mixtures were combined and concentrated under reduce pressure. The residue was extracted with EtOAc, the aqueous layer was acidified to pH 2 with aqueous HCl (2 M) and extracted with EtOAc several times again. The organic phase was washed with saturated sodium chloride solution, dried over $Na_2SO_4$ and concentrated under reduce pressure. (R)-2-((tert-Butoxycarbonyl)amino)-4-methoxy-4-oxobutanoic acid was obtained as yellow oil (500 g, 93 %).
$^1$H NMR (400 MHz, DMSO-d6): $\delta$ 12.71 (br. s., 1H), 7.13 (d, J=8.4 Hz, 1H), 4.34 - 4.25 (m, 1H), 3.61 - 3.56 (m, 3H), 2.79 - 2.56 (m, 2H), 1.37 (s, 9H).

i-3) (R)-Methyl 4-(benzyl(2-ethoxy-2-oxoethyl)amino)-3-((tert-butoxycarbonyl)amino)-4-oxobutanoate

To a solution of 100 g (404 mmol) (R)-2-((tert-Butoxycarbonyl)amino)-4-methoxy-4-oxobutanoic acid of example i-2) in DCM (2000 ml) was added 54.7 g HOBT (1.0 eq.) and 77 g EDCI (1.0 eq.) at -30 °C and stirred for 1 h. A solution of 78 g ethyl 2-(benzylamino) acetate (1.0 eq.) and 82 g 4-methylmorpholine (2.0 eq.) in DCM (100 ml) was added

dropwise. The mixture was stirred at room temperature overnight. Four additional vials were set up as described above. All five reaction mixtures were combined and extracted with water. The organic layer was concentrated under reduce pressure, the residue was re-dissolved in EtOAc and treated with aqueous HCl (2 M). The organic layer was washed with saturated sodium chloride solution, dried over $Na_2SO_4$ and concentrated under reduce pressure. (R)-Methyl 4-(benzyl(2-ethoxy-2-oxoethyl)amino)-3-((tert-butoxycarbonyl)amino)-4-oxobutanoate was obtained as yellow oil (600 g, 70 %).

i-4) (R)-Methyl 2-(4-benzyl-3,6-dioxopiperazin-2-yl)acetate

To a solution of 150 g (355 mmol) (R)-methyl 4-(benzyl(2-ethoxy-2-oxoethyl)amino)-3-((tert-butoxycarbonyl)amino)-4-oxobutanoate of example i-3) in DCM (1000 ml) was added TFA (200 ml) dropwise at ice bath temperature. The mixture was stirred at room temperature for 1 h and concentrated under reduced pressure. The residual TFA salt was dissolved in MeOH (1000 ml) and adjusted to pH 8 with triethylamine at ice bath, then stirred at room temperature overnight. Three additional vials were set up as described above. All four reaction mixtures were combined and concentrated under reduce pressure. The residue was dissolved in DCM and washed with water three times. The organic phase was washed with saturated sodium chloride solution, dried over $Na_2SO_4$ and concentrated under reduce pressure. The resulting residue was treated with water (1000 ml) and MTBE (500 ml). The precipitate that formed was filtered and dried under reduce pressure. (R)-Methyl 2-(4-benzyl-3,6-dioxopiperazin-2-yl)acetate was obtained as white solid (230 g, 65 %).
$^1$H NMR (400 MHz, Chloroform-d): $\delta$ 7.40 - 7.25 (m, 5H), 6.68 (br. s., 1H), 4.61 (s, 2H), 4.43 (d, J=7.7 Hz, 1H), 3.96 - 3.81 (m, 2H), 3.72 (s, 3H), 3.12 (dd, J=3.3, 17.6 Hz, 1H), 2.85 (dd, J=8.8, 17.4 Hz, 1H).

i-5) (R)-2-(4-Benzylpiperazin-2-yl)ethanol

To a solution of 50 g (181 mmol) (R)-methyl 2-(4-benzyl-3,6-dioxopiperazin-2-yl)acetate of example i-4) in THF (1500 ml) was added 41.2 g LAH (6.0 eq.) in portions at ice bath within 2 h and stirred under reflux overnight. After cooling to 0 °C, the mixture was quenched by slow addition of aqueous $NH_4Cl$ solution (10 %, 50 mL). Three additional vials were set up as described above. All four reaction mixtures were combined and filtered. The filtrate was concentrated under reduce pressure. The residue was dissolved in DCM, dried over $Na_2SO_4$ and concentrated under reduce pressure. Crude (R)-2-(4-benzylpiperazin-2-yl)ethanol was obtained as yellow oil (100 g, 63 %).
$^1$H NMR (400 MHz, Chloroform-d): $\delta$ 7.36 - 7.23 (m, 5H), 3.80 (t, J=5.4 Hz, 2H), 3.53 - 3.45 (m, 2H), 3.06 - 2.92 (m, 2H), 2.91 - 2.81 (m, 1H), 2.74 (d, J=11.2 Hz, 2H), 2.00 (dt, J=3.0, 11.1 Hz, 1H), 1.83 (t, J=10.5 Hz, 1H), 1.62 - 1.52 (m, 2H).

i-6) (R)-tert-Butyl 4-benzyl-2-(2-hydroxyethyl)piperazine-1-carboxylate

To a solution of 100 g (454 mmol) (R)-2-(4-benzylpiperazin-2-yl)ethanol of example i-5) in DCM (1000 ml) was added dropwise a mixture of 109 g di-tert-butyl dicarbonate (1.1 eq.) in DCM (100 ml) at ice bath. The mixture was stirred at room temperature overnight. Then, the mixture was treated with water and the organic phase was concentrated under reduce pressure. The residue was purified by flash chromatography on silica with petrol ether/ethyl acetate. (R)-tert-butyl 4-benzyl-2-(2-hydroxyethyl)piperazine-1-carboxylate was obtained as yellow oil (100 g, 69 %).
$^1$H NMR (400 MHz, Chloroform-d): $\delta$ 7.36 - 7.24 (m, 5H), 4.29 (br. s., 1H), 4.04 (br. s., 1H), 3.83 (d, J=12.1 Hz, 1H), 3.63 (d, J=11.5 Hz, 1H), 3.49 (br. s., 2H), 3.38 (d, J=10.1 Hz, 1H), 3.02 (t, J=11.6 Hz, 1H), 2.71 (d, J=11.2 Hz, 2H), 2.26 (dd, J=4.1, 11.4 Hz, 2H), 2.02 (dt, J=3.4, 11.7 Hz, 1H), 1.64 (d, J=8.2 Hz, 1H), 1.47 (s, 9H).
LCMS: m/z= 321.1 [M+H].

i-7) (R)-tert-Butyl 4-benzyl-2-(2-oxoethyl)piperazine-1-carboxylate

To a solution of 9.6 ml oxalyl chloride (1.4 eq.) in DCM (250 ml) was added a solution of 14 ml DMSO (1.0 eq.) in DCM (100 ml) at -60 °C. After stirring for 1 h, a solution of 25 g (78 mmol) (R)-tert-butyl 4-benzyl-2-(2-hydroxyethyl)piperazine-1-carboxylate of example i-6) in DCM (250 ml) was added dropwise. Stirring was continued for 1 h at -60°C and then 54 ml triethylamine (5.0 eq.) was added and the reaction mixture was stirred for 1 h. The mixture was stirred at room temperature overnight. One additional vial was set up as described above. All two reaction mixtures were combined and washed with water. The organic phase was washed with saturated sodium chloride solution and concentrated under reduced pressure. The residue was purified by flash chromatography on silica with petrol ether/ethyl acetate. (R)-tert-Butyl 4-benzyl-2-(2-oxoethyl)piperazine-1-carboxylate was obtained as yellow oil (35 g, 72 %).
1H NMR (400 MHz, Chloroform-d): $\delta$ 9.75 (d, J=1.8 Hz, 1H), 7.35 - 7.22 (m, 5H), 4.62 (br. s., 1H), 3.89 (br. s., 1H), 3.56 - 3.40 (m, 2H), 3.13 - 3.00 (m, 1H), 2.84 (br. s., 2H), 2.81 - 2.65 (m, 2H), 2.21 (d, J=11.5 Hz, 1H), 2.05 (t, J=11.6 Hz, 1H), 1.45 (s, 9H).

i-8) (R)-tert-Butyl 4-benzyl-2-((S)-2-hydroxy-3-methoxypropyl)piperazine-1-carboxylate and (R)-tert-butyl 4-benzyl-2-((R)-2-hydroxy-3-methoxypropyl)piperazine-1-carboxylate

25 g Magnesium (20.0 eq.) and 2.79 g $HgCl_2$ (0.2 eq.) were placed in a dry 1 liter three-necked round-bottomed flask under nitrogen atmosphere. 100 ml of anhydrous THF and 1,2-2.2 ml dibromoethane (0.5 eq.) were added into the reaction flask and the mixture was stirred at room temperature for 5 min. The dropping funnel was charged with a solution of 83 g $CH_3OCH_2Cl$ (19.5 eq.) in THF (1000 ml). 75 ml of the above solution was added at once with consecutive stirring until a mild exothermic reaction began. The mixture was then cooled to -25 °C and the remaining solution was added dropwise within 20 min. The mixture was stirred at -10 °C for 12 h. Then, the suspension was cooled down to -78 °C and a solution of 16.38 g (51.4 mmol) (R)-tert-butyl 4-benzyl-2-(2-oxoethyl)piperazine-1-carboxylate of example i-7) in anhydrous THF (160 ml) was added dropwise to the suspension. The mixture was stirred at the same temperature for another 2 h. One additional vial was set up as described above. All two reaction mixtures were combined and quenched with aqueous $NH_4Cl$ solution (10 %) in ice bath. The mixture was extracted with EtOAc and the organic phase was washed with saturated sodium chloride solution, dried over $Na_2SO_4$ and concentrated under reduced pressure. The crude product was purified by Prep-HPLC. (R)-tert-Butyl 4-benzyl-2-((S)-2-hydroxy-3-methoxypropyl)piperazine-1-carboxylate A (18 g, 49 %) and (R)-tert-butyl 4-benzyl-2-((R)-2-hydroxy-3-methoxypropyl)piperazine-1-carboxylate B (8 g, 22 %) were obtained as yellow oil.

Prep-HPLC conditions:

Instrument: Shimadzu LC-8A preparative HPLC
Column: Luna (2) C18 250*50 mm i.d. 10u
Mobile phase: A for $H_2O$ (10 mM $NH_4HCO_3$) and B for $CH_3CN$
Gradient: B from 52 % to 52 % in 20 min
Flow rate: 80 ml/min
Wavelength: 220 &254 nm
Injection amount: 1.0 g per injection

**Isomer A (R,S)**

$^1$H NMR (400 MHz, DMSO-d6): δ 7.36 - 7.20 (m, 5H), 4.54 (d, J=4.6 Hz, 1H), 4.13 (br. s., 1H), 3.74 (d, J=12.8 Hz, 1H), 3.54 (d, J=13.5 Hz, 1H), 3.44 - 3.33 (m, 2H), 3.24 (s, 3H), 3.21 (d, J=5.1 Hz, 2H), 2.99 (br. s., 1H), 2.78 - 2.64 (m, 2H), 1.98 - 1.86 (m, 3H), 1.51 - 1.42 (m, 1H), 1.38 (s, 9H)
LCMS: m/z = 365.2 [M+H].

**Isomer B (R,R)**

$^1$H NMR (400 MHz, DMSO-d6): δ 7.36 - 7.22 (m, 5H), 4.40 (d, J=4.9 Hz, 1H), 4.19 (br. s., 1H), 3.74 (d, J=13.0 Hz, 1H), 3.50 - 3.37 (m, 3H), 3.23 (s, 3H), 3.22 - 3.16 (m, 2H), 2.91 (br. s., 1H), 2.68 (dd, J=11.2, 19.0 Hz, 2H), 2.06 - 1.99 (m, 1H), 1.95 (br. s., 1H), 1.86 (dt, J=3.2, 11.6 Hz, 1H), 1.54 - 1.45 (m, 1H), 1.39 (s, 9H)
LCMS: m/z = 365.2 [M+1].

i-9) 6-Bromo-2-chloro-3-fluorophenol

4.12 g (28.1 mmol) of 2-Chloro-3-fluorophenol was dissolved in chloroform (120 ml) and 8.0 ml diisopropylamine (2.0 eq.) was added. The solution was cooled down to -50 °C and 5.0 g *N*-bromosuccinimide (1.0 eq.) was added portionwise and stirred for 2 h at this temperature. The crude mixture was diluted with DCM and washed twice with 1 M aqueous hydrochloric acid and saturated sodium chloride solution. The organic phase was dried with $Na_2SO_4$ and evaporated to dryness while not exceeding 30 °C. 6-Bromo-2-chloro-3-fluorophenol was obtained as colorless oil (6.12 g, 97 %) which crystallizes upon standing.
LC-MS: No ionization.
$^1$H NMR (600 MHz, Chloroform-d): δ 5.98 (d, J = 0.9 Hz, 1H, OH), 6.71 (dd, J = 9.0, 8.2 Hz, 1H), 7.38 (dd, J = 9.0, 5.6 Hz, 1H).

i-10) 1-Bromo-3-(difluoromethyl)-2-fluorobenzene

494 mg (2.43 mmol) 3-Bromo-2-fluorobenzaldehyde were dissolved in DCM (5 ml) and 0.67 ml Deoxofluor (1.5

eq.) were added dropwise via syringe at room temperature. The solution was stirred for 3 days and quenched by addition of saturated sodium hydrogencarbonate solution. The aqueous layer was extracted twice with DCM and combined extracts were washed 5 M potassium hydroxide and saturated sodium chloride solution. The organic phase was dried with $MgSO_4$ and evaporated to dryness while not exceeding 45 °C at 15 mbar. Crude 1-bromo-3-(difluoromethyl)-2-fluorobenzene was obtained as yellowish oil (835 mg, purity 56%, 78 %) containing residual bis(2-methoxyethyl)amine and was used without further purification.
LC-MS: No ionization.
$^1$H NMR (500 MHz, DMSO-d6): δ 7.25 (t, J = 54.1 Hz, 1H), 7.33 (t, J = 7.9 Hz, 1H), 7.66 (ddd, J = 7.7, 6.5, 1.4 Hz, 1H), 7.93 (tq, J = 6.7, 1.2 Hz, 1H).
$^{19}$F NMR (471 MHz, DMSO-d6): δ -113.71 (dd, J = 54.3, 5.4 Hz, 2F), -112.67 (q, J = 5.9 Hz, 1F).

i-11) 2-(Benzyloxy)-5-fluorobenzaldehyde

To a stirred solution of 27 g (193 mmol) 5-fluoro-2-hydroxybenzaldehyde in DMF (1000 ml), 40.8 g $Na_2CO_3$ (2.0 eq.) and 36.3 g benzyl bromide (1.1 eq.) were added. The mixture was stirred at 80 °C for 1 h. Then, the reaction mixture was poured into water and extracted with MTBE. The organic layer was washed with saturated sodium chloride solution, dried over $Na_2SO_4$ and evaporated to give 2-(benzyloxy)-5-fluorobenzaldehyde (43 g, 97 %) as yellow oil.
$^1$H NMR (400 MHz, Chloroform-d): δ 10.50 (d, J=3.1 Hz, 1H), 7.54 (dd, J=3.3, 8.2 Hz, 1H), 7.46 - 7.35 (m, 5H), 7.28 - 7.21 (m, 1H), 7.03 (dd, J=3.7, 9.0 Hz, 1H), 5.19 (s, 2H).

i-12) 1-(Benzyloxy)-2-(difluoromethyl)-4-fluorobenzene

To a solution of 43 g (187 mmol) 2-(benzyloxy)-5-fluorobenzaldehyde in DCM (800 mL), 24.7 ml DAST (1.0 eq.) was added under nitrogen atmosphere. The reaction mixture was stirred at 27 °C for 12 h. Then, the reaction mixture was quenched with saturated sodium carbonate solution to pH 8 and the organic phase was concentrated in vacuo. The residue was purified by flash chromatography on silica with petrol ether/ethyl acetate. 1-(Benzyloxy)-2-(difluoromethyl)-4-fluorobenzene was obtained as colorless oil (26.5 g, 56 %).
$^1$H NMR (400 MHz, Chloroform-d): δ 7.45 - 7.28 (m, 5H), 7.13 - 6.82 (m, 3H), 5.11 (s, 2H).

i-13) 2-(Difluoromethyl)-4-fluorophenol

To a solution of 26.5 g (111 mmol) 1-(benzyloxy)-2-(difluoromethyl)-4-fluorobenzene in MeOH (1000 ml), 8 g Pd/C (50 %) was added to the solution. The reaction mixture was stirred under hydrogen atmosphere at 27 °C for 3 h. The catalyst was filtered off and the filtrate concentrated to give 2-(difluoromethyl)-4-fluorophenol (16 g, 89 %) as colorless oil.
$^1$H NMR (400 MHz, Chloroform-d): δ 7.17 (dd, J=3.1, 8.4 Hz, 1H), 7.08 - 7.01 (m, 1H), 7.00 - 6.70 (m, 1H), 5.44 (br. s., 1H).

i-14) 2-Bromo-6-(difluoromethyl)-4-fluorophenol

To a stirred solution of 16 g (99 mmol) 2-(difluoromethyl)-4-fluorophenol in chloroform (740 ml) under nitrogen atmosphere, 31.6 g pyridinium tribromide (1.0 eq.) was added at 40 °C and the solution held at this temperature for 12 h until full conversion. The reaction mixture was diluted with EtOAc, washed three times each with water, 10 % aqueous citric acid, 0.1 M sodium thiosulfate solution and saturated sodium chloride solution. The organic phase was evaporated to yield 2-bromo-6-(difluoromethyl)-4-fluorophenol (15 g, purity 89 %, 63 %) as oil.
$^1$H NMR (400 MHz, Chloroform-d): δ 7.17 (dd, J=3.1, 8.4 Hz, 1H), 7.08 - 7.01 (m, 1H), 7.00 - 6.70 (m, 1H), 5.44 (br. s., 1H).
LC/MS: No ionization.

i-15) 2-Bromo-6-chloro-3-fluorophenol

To a solution of 5.0 g (33.4 mmol) 2-chloro-5-fluorophenol and 9.5 ml diisopropylamine (2.0 eq.) in DCM (150 ml) was added 6.0 g *N*-bromosuccinimide (1.0 eq.) suspended in DCM (50 ml) portionwise at 0-5 °C. The resulting mixture was stirred for additional 5 h under ice cooling. The crude mixture was diluted with DCM and washed with 1 M aqueous hydrochloric acid and twice with saturated sodium chloride solution. The organic phase was dried with $Na_2SO_4$ and concentrated in vacuo while not exceeding 35 °C. The residue was purified by flash chromatography on silica with heptane/ethyl acetate. 2-Bromo-6-chloro-3-fluorophenol was obtained as oil in two fractions of different purity (A: 3.02 g, purity 90 %, 36 %; B: 662 mg, 9 %).
LC-MS: No ionization.
$^1$H NMR (600 MHz, Chloroform-d): δ 6.02 (d, J = 1.1 Hz, 1H, OH), 6.73 (dd, J = 9.0, 7.7 Hz, 1H), 7.28 (dd, J = 8.9, 5.5 Hz, 1H).

i-16) Chloro-4,5-difluorophenol

To a solution of 5.18 g (39.8 mmol) 3,4-difluorophenol in DMF (75 ml) was added 6.38 g *N*-chlorosuccinimide (1.2 eq.) at room temperature and the reaction mixture was stirred overnight. The crude mixture was diluted with ethyl acetate and washed twice with 5 % aqueous citric acid, saturated sodium bicarbonate and saturated sodium chloride solution. The organic phase was dried with $MgSO_4$ and evaporated to dryness. 2-Chloro-4,5-difluorophenol was obtained as yellowish resin (6.47 g, 99 %).
LC-MS: No ionization.
$^1$H NMR (500 MHz, Chloroform-d): $\delta$ 5.50 (s, 1H), 6.87 (dd, J = 11.0, 7.2 Hz, 1H), 7.18 (dd, J = 9.5, 7.9 Hz, 1H).

i-17) 2-Bromo-6-chloro-3,4-difluorophenol

6.74 g (39.3 mmol) 2-Chloro-4,5-difluorophenol and 11.1 ml diisopropylamine (2.0 eq.) were dissolved in chloroform (150 ml) and cooled down to -60 °C. At this temperature, 7.0 g *N*-bromosuccinimde (1.0 eq.) was added in portions and the mixture was stirred for further 2 h. The crude mixture was allowed to reach room temperature, diluted with DCM and washed twice with 1 M aqueous hydrochloric acid and saturated sodium chloride solution. The organic phase was dried with $Na_2SO_4$ and concentrated in vacuo while not exceeding 30 °C. The oily residue was purified by flash chromatography on silica with petrol ether/ethyl ether. 2-Bromo-6-chloro-3,4-difluorophenol was obtained as slowly crystallizing orange oil (3.24 g, 34 %).
LC-MS: No ionization.
$^1$H NMR (500 MHz, Chloroform-d): $\delta$ 5.81 (d, J = 0.9 Hz, 1H, OH), 7.24 (dd, J = 9.4, 7.7 Hz, 1H).
$^{19}$F NMR (471 MHz, Chloroform-d): $\delta$ -143.00 (dd, J = 22.4, 9.5 Hz, IF), -127.59 (dd, J = 22.3, 7.8 Hz, 1F).

i-18) 2-Bromo-3,4,6-trifluorophenol

To a solution of 4.50 g (30.4 mmol) 2,4,5-trifluorophenol and 8.6 ml diisopropylamine (2.0 eq.) in DCM (150 ml) was added 5.46 g *N*-bromosuccinimide (1.0 eq.) portionwise at 0-5 °C and stirred under ice cooling for 100 min. The crude mixture was diluted with DCM and washed with 1 M aqueous hydrochloric acid and twice with saturated sodium chloride solution. The organic phase was dried with $Na_2SO_4$ and concentrated in vacuo while not exceeding 35°C. The residue was purified by flash chromatography on silica with heptane/ethyl acetate. 2-Bromo-3,4,6-trifluorophenol was obtained as oil in three fractions of different purity (A: 560 mg, purity 95 %, 8 %; B: 396 mg, purity 85 %, 5 %; C: 905 mg, purity 70 %, 9 %).
LC-MS: No ionization.
$^1$H NMR (600 MHz, Chloroform-d): $\delta$ 5.45 (d, J = 2.6 Hz, 1H, OH), 7.04 (td, J = 9.9, 7.2 Hz, 1H).

i-19) 2-Bromo-4-fluoro-6-(trifluoromethyl)phenol

1.21 g (6.72 mmol) 4-Fluoro-2-(trifluoromethyl)phenol was dissolved in DMF (20 ml) and 1.44 g *N*-bromosuccinimde (1.2 eq.) was added in portions at room temperature and the mixture was stirred for further 2 h. The crude mixture was diluted with ethyl acetate and washed twice with 5 % aqueous citric acid and saturated sodium chloride solution. The organic phase was dried with $Na_2SO_4$ and evaporated to dryness. 2-Bromo-4-fluoro-6-(trifluoromethyl)phenol was obtained as orange oil (1.57 g, 90 %).
LC-MS: No ionization.
$^1$H NMR (600 MHz, Chloroform-d): $\delta$ 5.84 (s, 1H, OH), 7.29 (dd, J = 8.1, 3.0 Hz, 1H), 7.44 (dd, J = 7.1, 3.0 Hz, 1H).
$^{19}$F NMR (471 MHz, Chloroform-d): $\delta$ -120.21 (t, J = 7.5 Hz, IF), -63.13 (s, 3F).

i-20) 2-(Benzyloxy)-4,5-difluorobenzaldehyde

1.16 g (7.32 mmol) 4,5-Difluoro-2-hydroxybenzaldehyde was dissolved in DMF (10 ml) and 3.00 g potassium carbonate (3.0 eq.), 120 mg potassium iodide (0.1 eq.) were suspended therein. Then, 0.87 ml benzyl bromide (1.0 eq.) was added dropwise under Ar atmosphere at room temperature. The reaction mixture was stirred overnight and portioned between water and ethyl acetate. The organic phase was separated, washed twice with water, dried with $MgSO_4$ and evaporated to dryness. 2-(Benzyloxy)-4,5-difluorobenzaldehyde was obtained as slowly crystallizing yellowish oil (1.83 g, 100 %).
LC-MS: m/z = 271.1 [M+Na].

i-21) 1-(Benzyloxy)-2-(difluoromethyl)-4,5-difluorobenzene

1.83 g (7.38 mmol) 2-(Benzyloxy)-4,5-difluorobenzaldehyde was dissolved in DCE (5 ml) under Ar atmosphere and 6.25 ml Deoxofluor (50% solution in toluene, 2.0 eq.) was added at room temperature. The reaction mixture was heated to 60 °C for 4 h. The cooled mixture was diluted with DCM and washed with saturated sodium bicarbonate solution. The organic phase was dried with $MgSO_4$ and evaporated to dryness. The residue was purified by flash chromatography on silica with petrol ether/ethyl ether. 1-(Benzyloxy)-2-(difluoromethyl)-4,5-difluorobenzene was obtained as colorless oil (1.64 g, 82 %).
LC-MS: No ionization.

i-22) Unstable 2-(difluoromethyl)-4,5-difluorophenol

1.03 g (3.80 mmol) 1-(Benzyloxy)-2-(difluoromethyl)-4,5-difluorobenzene was dissolved in EtOAc (20 ml) and 250 mg Palladium on charcoal (10%) was suspended under argon atmosphere therein. The reaction mixture was hydrogenated until hydrogen uptake at 1 atm was completed. The crude mixture was purged with nitrogen, the catalyst was filtered off and the filter cake was rinsed with ethyl acetate. The filtrate was washed with saturated sodium bicarbonate solution, dried with $MgSO_4$ and evaporated to dryness whereby the oily residue slowly turned brownish. The bath temperature should not exceed 40 °C. Unstable 2-(difluoromethyl)-4,5-difluorophenol was used immediately without further purification in the next step (617 mg, 90 %).
LC-MS: No ionization.

i-23) 2-Bromo-6-(difluoromethyl)-3,4-difluorophenol

To a solution of 298 mg (1.66 mmol) 2-(difluoromethyl)-4,5-difluorophenol in DMF (20 ml) was added 294 mg *N*-bromosuccinimide (1.0 eq.) at room temperature and the mixture was stirred overnight. The crude mixture was diluted with ethyl acetate and washed twice with 5 % aqueous citric acid and saturated sodium chloride solution. The organic phase was dried with $Na_2SO_4$ and evaporated to dryness. 2-Bromo-6-(difluoromethyl)-3,4-difluorophenol was obtained as yellowish oil (375 mg, 88 %).
LC-MS: No ionization.
$^1$H NMR (500 MHz, Chloroform-d): δ 5.76 (s, 1H, OH), 6.91 (td, J = 55.0, 1.1 Hz, 1H), 7.41 (dd, J = 9.8, 8.3 Hz, 1H).
$^{19}$F NMR (471 MHz, Chloroform-d): δ -142.76 (dd, J = 21.8, 9.9 Hz, IF), -123.23 - - 123.09 (m, IF), -114.76 (d, J = 54.7 Hz, 2F).

i-24) 3-Bromo-4,5-difluoro-2-hydroxybenzonitrile

To a solution of 747 mg (4.82 mmol) 4,5-difluoro-2-hydroxybenzonitrile in DMF (20 ml) was added 857 mg *N*-bromosuccinimide (1.0 eq.) at room temperature and the mixture was stirred overnight. The crude mixture was diluted with ethyl acetate and washed twice with 5 % aqueous citric acid and saturated sodium chloride solution. The organic phase was dried with $MgSO_4$ and evaporated to dryness. 3-Bromo-4,5-difluoro-2-hydroxybenzonitrile was obtained as yellowish oil (984 mg, 87 %).
LC-MS: m/z = 234.0/236.0 [M+H]. Weak ionization.
$^1$H NMR (500 MHz, Chloroform-d): δ 7.40 (dd, J = 9.1, 8.0 Hz, 1H).
$^{19}$F NMR (471 MHz, Chloroform-d): δ -141.38 (dd, J = 22.7, 8.7 Hz, IF), -115.91 (dd, J = 22.2, 7.9 Hz, 1F).

i-25) 6-Bromo-2-chloro-3,4-difluorophenol

To a solution of 400 mg (2.43 mmol) 2-chloro-3,4-difluorophenol and 0.69 ml diisopropylamine (2.0 eq.) in DCM (12 ml) was added 437 mg *N*-bromosuccinimide (1.0 eq.) portionwise at -5 °C and the mixture was stirred for 2.5 h. The crude mixture was diluted with DCM and washed with 1 M aqueous hydrochloric acid and twice with saturated sodium chloride solution. The organic phase was dried with $Na_2SO_4$ and evaporated to dryness at 35 °C. 6-Bromo-2-chloro-3,4-difluorophenol was obtained as oil (568 mg, 96 %).
LC-MS: No ionization.
$^1$H NMR (500 MHz, Chloroform-d): δ 5.77 (s, 1H), 7.33 (dd, J = 9.1, 7.7 Hz, 1H).
$^{19}$F NMR (471 MHz, Chloroform-d): δ -143.19 (dd, J = 21.4, 8.8 Hz, IF), -134.76 (dd, J = 21.2, 7.7 Hz, 1F).

i-26) 2-Bromo-6-chloro-4-methoxyphenol

To a solution of 3.52 g (22.2 mmol) 2-chloro-4-methoxyphenol and 6.27 ml diisopropylamine (2.0 eq.) in DCM

(15 ml) was added 3.98 g *N*-bromosuccinimide (1.0 eq.) portionwise at 0-5°C and the resulting mixture was held at this temperature for approximately 3 h. The crude mixture was diluted with DCM and washed with 1 M aqueous hydrochloric acid and twice with saturated sodium chloride solution. The organic phase was dried with $Na_2SO_4$ and evaporated to dryness while not exceeding 35 °C. The residue was purified by flash chromatography on silica with heptane/ethyl acetate. 2-Bromo-6-chloro-4-methoxyphenol was obtained as oil (217 mg, 4 %).
LC-MS: No ionization.
$^1$H NMR (500 MHz, Methanol-d4): δ 3.72 (s, 3H), 6.92 (d, J = 2.9 Hz, 1H), 7.03 (d, J = 2.9 Hz, 1H).

i-27) 2-Bromo-6-chloro-4-(trifluoromethyl)phenol

550 mg (2.80 mmol) 2-Chloro-4-(trifluoromethyl)phenol was dissolved in DMF (30 ml) and 604 mg *N*-bromosuccinimide (1.2 eq.) was added at room temperature and the mixture was stirred overnight. The crude mixture was diluted with ethyl acetate and washed three times with 10 % aqueous citric acid and twice with saturated sodium chloride solution. The organic phase was dried with $MgSO_4$ and concentrated in vacuo. The residue was purified by flash chromatography on silica with DCM/methanol. Remaining DMF was removed by dissolving the purified phenol in alkaline water and by continuous extraction with DCM. Then, the water phase was acidified and the product re-extracted with DCM. The organic phase was dried with $MgSO_4$ and concentrated in vacuo. 2-Bromo-6-chloro-4-(trifluoromethyl)phenol was obtained as oil (337 mg, 44 %).
LC-MS: No ionization.
$^1$H NMR (600 MHz, DMSO-d6): δ 7.84 (d, J = 2.1 Hz, 1H), 7.90 (d, J = 2.2 Hz, 1H).

i-28) 4,5-Difluoro-2-iodophenol

To a mixture of 5.51 g (42.4 mmol) 3,4-difluorophenol, 10.75 g iodine (1.0 eq.) and 7.03 g KI (1.0 eq.) in water (200 ml) was added 4.75 g KOH (2.0 eq.) dissolved in water (200 ml) over a period of 45 min under ice-cooling. After completed addition, the reaction mixture was stirred for 3 h at room temperature. The crude mixture was neutralized with solid $NH_4Cl$ and saturated sodium thiosulfate solution was added in order to remove remaining iodine. The solution was extracted twice with diethyl ether and the combined organic phases were dried with $MgSO_4$. The solvent was removed under reduced pressure. The brownish oily residue was purified by flash chromatography on silica with heptane/diethyl ether. 4,5-Difluoro-2-iodophenol was obtained as colorless oil (4.91 g, 45 %).
LC-MS: No ionization.
$^1$H NMR (600 MHz, DMSO-d6): δ 6.85 (dd, J = 12.3, 7.2 Hz, 1H), 7.68 - 7.90 (m, 1H), 10.73 (s, 1H).

i-29) 1-(Benzyloxy)-4,5-difluoro-2-iodobenzene

4.91 g (19.16 mmol) 4,5-Difluoro-2-iodophenol was dissolved in DMF (100 ml) and 7.95 g $K_2CO_3$ (3.0 eq.) as well as 318 mg KI (0.1 eq.) were added. Then, 2.3 ml benzyl bromide (1.0 eq.) was added dropwise at room temperature and the mixture was stirred overnight. The crude mixture was partitioned between ethyl acetate and water. The organic phase was separated, washed twice with water, dried with $MgSO_4$ and evaporated to dryness. 1-(Benzyloxy)-4,5-difluoro-2-iodobenzene was obtained as yellowish oil (6.66 g, 100 %).
LC-MS: No ionization.
1H NMR (500 MHz, DMSO-d6): δ 5.18 (s, 2H), 7.28 - 7.38 (m, 2H), 7.40 - 7.46 (m, 2H), 7.47 - 7.53 (m, 2H), 7.93 (dd, J = 9.9, 9.1 Hz, 1H).
$^{19}$F NMR (471 MHz, DMSO-d6): δ -146.66 (dt, J = 22.3, 8.4 Hz, IF), -135.41 (dt, J = 22.5, 11.4 Hz, 1F).

i-30) 1-(Benzyloxy)-4,5-difluoro-2-(trifluoromethyl)benzene

2.52 g (7.28 mmol) 1-(Benzyloxy)-4,5-difluoro-2-iodobenzene, 9.10 g 2,2-difluoro-2-(triphenylphosphonio)acetate (3.5 eq.), 9.50 g tetrakis(acetonitrile)copper(I)hexafluorophosphate (3.5 eq.) and 4.4 ml DBU (4.0 eq.) were dissolved in DMF (15 ml) at room temperature under argon atmosphere. The reaction was heated up to 55 °C for 6 h. The crude mixture was partitioned between diethyl ether and 1 M aqueous hydrochloric acid. The organic phase was separated, washed with 1 M aqueous hydrochloric acid followed by water and saturated sodium chloride solution. The etheric phase was dried with $MgSO_4$ and concentrated in vacuo while the temperature did not exceed 25 °C. The residue was purified by flash chromatography on silica with petrol ether/diethyl ether. 1-(Benzyloxy)-4,5-difluoro-2-(trifluoromethyl)benzene was obtained as colorless oil (1.69 g, 81 %).
LC-MS: No ionization.
$^1$H NMR (500 MHz, DMSO-d6): δ 5.27 (s, 2H), 7.29 - 7.49 (m, 5H), 7.60 (dd, J = 12.6, 6.5 Hz, 1H), 7.85 (dd, J = 10.6, 8.8 Hz, 1H).

$^{19}F$ NMR (471 MHz, DMSO-d6): δ -147.32 (ddd, J = 23.7, 10.8, 6.9 Hz, IF), -128.90 (dt, J = 22.4, 11.2 Hz, IF), -60.52 (s, 3F).

i-31) 4,5-Difluoro-2-(trifluoromethyl)phenol

1.62 g (5.62 mmol) 1-(Benzyloxy)-4,5-difluoro-2-(trifluoromethyl)benzene was dissolved in EtOAc (50 ml) and 250 mg Palladium on charcoal (10%) was suspended under nitrogen atmosphere therein. The reaction mixture was hydrogenated until hydrogen uptake at 1 atm was completed. The crude mixture was purged with nitrogen, the catalyst was filtered off and the filter cake was rinsed with ethyl acetate. The filtrate was evaporated to dryness while temperature should not exceed 40 °C. Crude 4,5-difluoro-2-(trifluoromethyl)phenol was used immediately without further purification in the next step (1.19 g, 100 %).
LC-MS: No ionization.

i-32) 2-Bromo-3,4-difluoro-6-(trifluoromethyl)phenol

1.19 g (6.00 mmol) 4,5-Difluoro-2-(trifluoromethyl)phenol was dissolved in DMF (50 ml) and 1.17 g *N*-bromosuccinimide (1.1 eq.) was added at room temperature and the mixture was stirred overnight. The crude mixture was diluted with ethyl acetate and washed twice with 5 % aqueous citric acid and twice with saturated sodium chloride solution. The organic phase was dried with $Na_2SO_4$ and concentrated in vacuo. 2-Bromo-3,4-difluoro-6-(trifluoromethyl)phenol was obtained as yellowish oil (1.60 g, 96 %).
LC-MS: No ionization.
$^{1}H$ NMR (500 MHz, DMSO-d6): δ 7.83 (dd, J = 10.5, 8.7 Hz, 1H), 10.79 (s, 1H).
$^{19}F$ NMR (471 MHz, DMSO-d6): δ -144.57 (dd, J = 24.4, 10.8 Hz, IF), -120.76 (dd, J = 24.2, 8.6 Hz, IF), -60.69 (s, 3F).

i-33) (R)-tert-butyl 4-benzyl-2-((S)-2-(2-bromo-4-chlorophenoxy)-3-methoxypropyl)-piperazine-1-carboxylate

A solution of 250 mg (0.686 mmol) (R)-tert-butyl 4-benzyl-2-((S)-2-hydroxy-3-methoxypropyl)piperazine-1-carboxylate and 0.1 ml 2-bromo-4-chloro-1-fluorobenzene (1.2 eq.) in DMSO (5 ml) under nitrogen atmosphere was stirred at room temperature for 2 h. The reaction mixture was poured onto brine and extracted twice with EtOAc. Combined extracts were washed with water three times and with saturated sodium chloride solution, dried over $MgSO_4$ and evaporated to dryness. The residue was purified by flash chromatography on silica with dichloromethane/methanol. (R)-tert-butyl 4-benzyl-2-((S)-2-(2-bromo-4-chlorophenoxy)-3-methoxypropyl)piperazine-1-carboxylate was obtained as oil (192 mg, 51 %).

1.2 Synthesis of the target compounds

f-1) General synthetic procedure exemplified by the synthesis of (4aR,6S)-8-(difluoromethyl)-10-fluoro-6-(methoxymethyl)-2,3,4,4a,5,6-hexahydro-1H-pyrazino[2,1-d][1,5]benzoxazepine hydrochloride (compound of example 9)

f-1.1) (R)-tert-butyl 4-benzyl-2-((S)-2-(2-bromo-6-(difluoromethyl)-4-fluorophenoxy)-3-methoxypropyl)piperazine-1-carboxylate

5.55 g (15.23 mmol) of (R)-tert-butyl 4-benzyl-2-((R)-2-hydroxy-3-methoxypropyl)-piperazine-1-carboxylate (isomer B of example i-8)), 4.40 g 2-bromo-6-(difluoromethyl)-4-fluorophenol of example i-14) (1.2 eq.) and 8.39 g triphenylphosphine (2.1 eq.) were dissolved in toluene under nitrogen atmosphere and cooled with ice-water. Then, 17 ml DIAD (2.08 eq.) was added to the reaction mixture dropwise. The reaction mixture was allowed to reach room temperature and stirring was continued overnight. Toluene was evaporated and the remaining residue taken up with ethyl acetate. The organic phase was washed twice with water and saturated sodium chloride solution, dried over $MgSO_4$ and evaporated. The residue was purified by flash chromatography on silica with cyclohexane/ethyl acetate. (R)-tert-butyl 4-benzyl-2-((S)-2-(2-bromo-6-(difluoromethyl)-4-fluorophenoxy)-3-methoxypropyl)-piperazine-1-carboxylate was obtained as yellowish oil (8.95 g, 80 %).

f-1.2) (R)-1-benzyl-3-((S)-2-(2-bromo-6-(difluoromethyl)-4-fluorophenoxy)-3-methoxypropyl)piperazine

To a solution of 7.19 g (12.24 mmol) (R)-tert-butyl 4-benzyl-2-((S)-2-(2-bromo-6-(difluoromethyl)-4-fluorophenoxy)-3-methoxypropyl)piperazine-1-carboxylate of example f-1.1) in dichloromethane (70 ml) was slowly added 22 ml hydrochloride acid solution (5-6 M in isopropanol, ca. 9.9 eq.) and the solution was stirred at room temperature overnight. The reaction mixture was concentrated in vacuo, the residue dissolved in water and washed with MTBE twice. The MTBE phase was back-extracted with water once. The pH value of the combined aqueous phase was adjusted to pH

8 by careful addition of solid sodium hydrogencarbonate and then extracted with DCM four times. The combined extracts were dried with $MgSO_4$ and evaporated to dryness. Crude (R)-1-benzyl-3-((S)-2-(2-bromo-6-(difluoromethyl)-4-fluorophenoxy)-3-methoxypropyl)piperazine was obtained as oil (5.45 g, 91 %) and used without further purification in the next step.

f-1.3) (4aR,6S)-3-Benzyl-8-(difluoromethyl)-10-fluoro-6-(methoxymethyl)-2,3,4,4a,5,6-hexahydro-1H-benzo [b]pyrazino[1,2-d][1,4]oxazepine

5.45 g (11.18 mmol) of (R)-1-benzyl-3-((S)-2-(2-bromo-6-(difluoromethyl)-4-fluoro-phenoxy)-3-methoxypropyl)piperazine of example f-1.2) was dissolved in degassed toluene (70 ml) under argon atmosphere and added to a mixture of 780 mg tris-(dibenzylideneacetone)-dipalladium(O) (0.076 eq.), 710 mg 2,2-bis(diphenylphosphino)-1,1-binaphthyl (0.10 eq.) and 1.65 g sodium tert-butoxide (1.54 eq) held under argon atmosphere at room temperature. The solution was stirred for 5 minutes and then heated to 110 °C for 1.5 h. The temperature was allowed to reach room temperature and charcoal was added to the reaction mixture. The resulting suspension was filtered through celite, the residue was washed with toluene and dichloromethane subsequently and the filtrate was evaporated to dryness. The residue was purified by flash chromatography on silica with cyclohexane/ethyl acetate. (4aR,6S)-3-Benzyl-8-(difluoromethyl)-10-fluoro-6-(methoxymethyl)-2,3,4,4a,5,6-hexahydro-1H-benzo[b]pyrazino[1,2-d][1,4]oxazepine was obtained as oil in good purity (4.60 g, 100 %).

f-1.4) (4aR,6S)-Ethyl 8-(difluoromethyl)-10-fluoro-6-(methoxymethyl)-4,4a,5,6-tetra-hydro-1H-benzo [b]pyrazino[1,2-d][1,4]oxazepine-3 (2H)-carboxylate

To a solution of 4.59 g (11.29 mmol) (4aR,6S)-3-benzyl-8-(difluoromethyl)-10-fluoro-6-(methoxymethyl)-2,3,4,4a,5,6-hexahydro-1H-benzo[b]pyrazino[1,2-d][1,4]oxazepine of example f-1.3) in toluene (90 ml) was added 9.49 g sodium hydrogencarbonate (10.0 eq.) and 8.6 ml ethyl chloroformate (8.0 eq.). The resulting suspension was heated to 95 °C and stirred for 2 h upon completion. The suspension was cooled to room temperature, diluted with water, the organic phase separated and the aqueous phase was extracted with dichloromethane twice. The combined extracts were washed with aqueous potassium hydroxide (1 M), dried over $MgSO_4$ and evaporated to dryness. The residue was purified by flash chromatography on silica with cyclohexane/ethyl acetate. (4aR,6S)-Ethyl 8-(difluoromethyl)-10-fluoro-6-(methoxymethyl)-4,4a,5,6-tetrahydro-1H-benzo[b]pyrazino[1,2-d][1,4]oxazepine-3(2H)-carboxylate was obtained as oil (3.60 g, 82 %).

f-1.5) (4aR,6S)-8-(Difluoromethyl)-10-fluoro-6-(methoxymethyl)-2,3,4,4a,5,6-hexahydro-1H-benzo[b]pyrazino[1,2-d][1,4]oxazepine

3.60 g (9.27 mmol) of (4aR,6S)-ethyl 8-(difluoromethyl)-10-fluoro-6-(methoxymethyl)-4,4a,5,6-tetrahydro-1H-benzo[b]pyrazino[1,2-d][1,4]oxazepine-3(2H)-carboxylate of example f-1.4) was dissolved in 50 ml potassium hydroxide solution (0.6 M in MeOH, 3.24 eq.), diluted with water (9 ml) and heated at reflux for 3 h. Given the observed incomplete conversion, the reaction mixture was split into portions of 16-17 ml each and heated to 140 °C for 75 minutes in a microwave oven (CEM). Methanol was evaporated and the residue partitioned between dichloromethane and water. The aqueous layer was separated and the pH adjusted to pH 8 by adding aqueous hydrochloride acid (1 M) slowly. The aqueous layer was extracted with dichloromethane another three times, the combined organic extracts were dried over $MgSO_4$ and concentrated in vacuo. The residue was purified by flash chromatography on silica with dichloromethane/methanol. (4aR,6S)-8-(Difluoromethyl)-10-fluoro-6-(methoxymethyl)-2,3,4,4a,5,6-hexahydro-1H-benzo[b]pyrazino[1,2-d][1,4]oxazepine was obtained as oily residue (2.70 g, 92 %). The corresponding methyl carbamate as minor byproduct could be isolated as well (230 mg, 7 %). Its cleavage is achieved by the very same procedure.

f-1.6) (4aR,6S)-8-(Difluoromethyl)-10-fluoro-6-(methoxymethyl)-2,3,4,4a,5,6-hexahydro-1H-benzo[b]pyrazino[1,2-d][1,4]oxazepinehydrochloride

6.2 g (19.6 mmol) of (4aR,6S)-8-(difluoromethyl)-10-fluoro-6-(methoxymethyl)-2,3,4,4a,5,6-hexahydro-1H-benzo[b]pyrazino[1,2-d][1,4]oxazepine of example f-1.5) were dissolved in $CH_2Cl_2$ (18 ml) and diluted with $Et_2O$ (100 ml). The solution became turbid and the solubility was adjusted by addition of MeOH (ca. 5 ml) following additional quantities of $Et_2O$ (40 ml). The clear solution was purged with nitrogen and cooled with an ice-water bath during dropwise addition of 10 ml etheric hydrochloric solution (2 M, 1.02 eq.). The suspension was further diluted with diethyl ether, the precipitate was sucked off and washed with a small volume of ether. (4aR,6S)-8-(Difluoromethyl)-10-fluoro-6-(methoxymethyl)-2,3,4,4a,5,6-hexahydro-1H-benzo[b]pyrazino[1,2-d][1,4]oxazepine hydrochloride was obtained as white solid (6.46 g, 93 %) after drying at 40 °C.

f-2) Variations of the method of f-1)

f-2.1) Synthesis of (4aR,6S)-8-Chloro-10,11-difluoro-6-(methoxymethyl)-2,3,4,4a,5,6-hexahydro-1H-pyrazino[2,1-d][1,5]benzoxazepine (free base of compound of example 16) via debenzylation of the N-benzyl protected analogue

1.50 g (3.67 mmol) of (4aR,6S)-3-benzyl-8-chloro-10,11-difluoro-6-(methoxymethyl)-2,3,4,4a,5,6-hexahydro-1H-benzo[b]pyrazino[1,2-d][1,4]oxazepine was dissolved in 1,2-dichloroethane (30 ml) under nitrogen atmosphere and 1.2 ml 1-chloroethyl chloroformate (3.0 eq.) was added. The reaction mixture was stirred overnight at room temperature. In some cases, heating was required in order to drive the reaction to completion. The solvent and excess reagent was evaporated and the crude formate dissolved in MeOH (10 ml). The solution was refluxed for 2 h and al volatiles were removed in vacuo. The crude hydrochloride was dissolved in water and washed with MTBE several times. Then, the water phase was neutralized to pH 8 with saturated sodium hydrogencarbonate solution and extracted with dichloromethane four times. The combined extracts were dried over $MgSO_4$ and evaporated to dryness. The residue could be purified by flash chromatography on silica with dichloromethane/methanol if necessary. Crude (4aR,6S)-8-chloro-10,11-difluoro-6-(methoxymethyl)-2,3,4,4a,5,6-hexahydro-1H-benzo[b]pyrazino[1,2-d][1,4]oxazepine was used in the salt forming step according to the general procedure (1.06 g, 81 % of hydrochloride).

f-2.2) Synthesis of (4aR,6S)-8-(Difluoromethyl)-10,11-difluoro-6-(methoxymethyl)-2,3,4,4a,5,6-hexahydro-1H-benzo[b]pyrazino[1,2-d][1,4]oxazepine (free base of compound of example 28) via debenzylation of the N-benzyl protected analogue with hydrogen

492 mg (1.16 mmol) of (4aR,6S)-3-benzyl-8-(difluoromethyl)-10,11-difluoro-6-(methoxymethyl)-2,3,4,4a,5,6-hexahydro-1H-benzo[b]pyrazino[1,2-d][1,4]oxazepine was dissolved in EtOAc (45 ml) and AcOH (5 ml) as well as 200 mg Pd/C (10 %) was added to mixture under nitrogen atmosphere. Then, the flask was set purged with hydrogen and stirring was continued at room temperature for 19 h. Given only partial conversion, additional 200 mg of Pd-catalyst was added after backfilling with nitrogen. The flask was purged with hydrogen again and the reaction mixture was heated at 50 °C for 4 h (still incomplete conversion). The reaction mixture was purged with nitrogen, the catalyst was filtered off and the filter cake was washed with EtOAc. The collected filtrate was washed with saturated sodium hydrogencarbonate three times and with saturated sodium chloride solution. The organic phase was dried over $MgSO_4$ and evaporated. The residue was purified by flash chromatography on silica with dichloromethane/methanol. (4aR,6S)-8-(Difluoromethyl)-10,11-difluoro-6-(methoxymethyl)-2,3,4,4a,5,6-hexahydro-1H-benzo[b]pyrazino[1,2-d][1,4]oxazepine was obtained as oil (238 mg, 61 %) and used in the salt forming step according to the general procedure (175 mg, 66 % of hydrochloride).

f-2.3) Synthesis of (4aR,6S)-8-Chlor0-11-fluoro-6-(methoxymethyl)-2,3,4,4a,5,6-hexahydro-1H-benzo[b]pyrazino[1,2-d][1,4]oxazepine fumarate (compound of example 15) from the free base

175 mg (0.58 mmol) of (4aR,6S)-8-chloro-11-fluoro-6-(methoxymethyl)-2,3,4,4a,5,6-hexahydro-1H-benzo[b]pyrazino[1,2-d][1,4]oxazepine were dissolved in ethanol (8 ml), 67.5 mg fumaric acid (1.0 eq.) was added and the slurry was stirred at room temperature for 2h. The mixture was evaporated to dryness. (4aR,6S)-8-Chloro-11-fluoro-6-(methoxymethyl)-2,3,4,4a,5,6-hexahydro-1H-benzo [b]pyrazino[1,2-d][1,4]oxazepine fumarate was obtained as solid (or foam) (240 mg, 97 %).

f-2.4) Synthesis of (4aR,6S)-10-Ethynyl-8-fluoro-6-(methoxymethyl)-2,3,4,4a,5,6-hexa-hydro-1H-benzo[b]pyrazino[1,2-d][1,4]oxazepine tartrate (compound of example 38) from the free base

7 mg (24 μmol) of (4aR,6S)-10-ethynyl-8-fluoro-6-(methoxymethyl)-2,3,4,4a,5,6-hexahydro-1H-benzo[b]pyrazino[1,2-d][1,4]oxazepine were dissolved in ethanol (2 ml) and 3.6 mg (L)-tartaric acid (1.0 eq.) was added. The mixture was stirred for 1 h at room temperature and the solvent was evaporated. The residue was dissolved in dichloromethane/diethyl ether and evaporated to dryness again. (4aR,6S)-10-Ethynyl-8-fluoro-6-(methoxymethyl)-2,3,4,4a,5,6-hexahydro-1H-benzo[b]pyrazino[1,2-d][1,4]oxazepine tartrate was obtained as foam (10 mg, 94 %).

f-2.5) Synthesis of ((4aR,6S)-8-chloro-2,3,4,4a,5,6-hexahydro-1H-benzo[b]pyrazino-[1,2-d][1,4]oxazepin-6-yl)methanol (free base of the compound of example 14) via demethylation of the compound of example 1

To an ice-cooled solution of 500 mg (1.57 mmol) (4aR,6S)-8-chloro-6-(methoxymethyl)-2,3,4,4a,5,6-hexahydro-1H-benzo [b]pyrazino[1,2-d][1,4]oxazepine hydrochloride in dichloromethane (10 ml) under nitrogen atmosphere was added 4.7 ml boron tribromide solution (1 M in DCM, 3.0 eq.). The reaction mixture was stirred for 3 h at 0 °C and quenched by careful addition of cold, saturated sodium hydrogencarbonate solution. The organic phase was separated

and the aqueous phase of pH 8-9 further extracted with dichloromethane several times. The combined extracts were dried over $MgSO_4$ and evaporated to dryness. Crude ((4aR,6S)-8-chloro-2,3,4,4a,5,6-hexahydro-1H-benzo[b]pyrazino[1,2-d][1,4]oxazepin-6-yl)methanol was obtained as oily residue (515 mg, 80 %) and used in the next step without further purification.

f-2.6) Boc-protection of ((4aR,6S)-8-chloro-2,3,4,4a,5,6-hexahydro-1H-benzo[b]pyrazino[1,2-d][1,4]oxazepin-6-yl)methanol (free base of the compound of example 1)

To an ice-cooled suspension of (crude) 515 mg (1.53 mmol) ((4aR,6S)-8-chloro-2,3,4,4a,5,6-hexahydro-1H-benzo[b]pyrazino[1,2-d][1,4]oxazepin-6-yl)methanol and 350 mg sodium carbonate (2.0 eq.) in methanol (20 ml) were added 570 mg Boc-anhydride (1.7 eq.). Stirring was continued for 1 h at 0°C and the reaction mixture was allowed to reach room temperature overnight. The reaction mixture was concentrated in vacuo and the residue partitioned between water and EtOAc. The aqueous phase was extracted with EtOAc, the combined extracts were washed with brine, dried over $MgSO_4$ and evaporated to dryness. The residue could be purified by flash chromatography on silica with cyclohexane/ethyl acetate if necessary. (4aR,6S)-tert-Butyl 8-chloro-6-(hydroxymethyl)-4,4a,5,6-tetrahydro-1H-benzo[b]pyrazino[1,2-d][1,4]oxazepine-3(2H)-carboxylate was obtained as oil (538 mg, 95 %) with sufficient purity.

f-2.7) Synthesis of 8-chloro-6-(D3-methoxymethyl)-4,4a,5,6-tetrahydro-1H-benzo[b]-pyrazino[1,2-d][1,4]oxazepine-3(2H)-carboxylate (free base of the Boc-protected compound of example 19)

60 mg (0.155 mmol) of (4aR,6S)-tert-butyl 8-chloro-10-fluoro-6-(hydroxymethyl)-4,4a,5,6-tetrahydro-1H-benzo[b]pyrazino[1,2-d][1,4]oxazepine-3(2H)-carboxylate (free base of the Boc-protected compound of example 17) was dissolved in DMF (3 ml) under nitrogen atmosphere and 26 mg sodium hydride (4.2 eq.) was added under ice-water cooling. Stirring was continued for 1 h and 10 $\mu$l iodomethane-D3 (1.5 eq.) were added. The mixture was allowed to reach room temperature and stirred overnight. The reaction mixture was poured onto water and extracted with ethyl acetate. Combined extracts were washed with water twice and with saturated sodium chloride solution, dried over $MgSO_4$ and concentrated in vacuo. Crude (4aR,6S)-tert-butyl 8-chloro-6-(D3-methoxymethyl)-4,4a,5,6-tetrahydro-1H-benzo[b]pyrazino[1,2-d][1,4]oxazepine-3(2H)-carboxylate was obtained as oil (50 mg, 80 %) and used in the next step without further purification.

f-2.8) Synthesis of 8-chloro-6-((difluoromethoxy)methyl)-10-fluoro-4,4a,5,6-tetrahydro-1H-benzo[b]pyrazino[1,2-d][1,4]oxazepine-3(2H)-carboxylate (free base of the Boc-protected compound of example 24)

To a solution of 200 mg (0.517 mmol) (4aR,6S)-tert-butyl 8-chloro-10-fluoro-6-(hydroxymethyl)-4,4a,5,6-tetrahydro-1H-benzo[b]pyrazino[1,2-d][1,4]oxazepine-3(2H)-carboxylate (free base of the Boc-protected compound of example 17) and 12 mg copper(I) iodide (0.12 eq.) in acetonitrile (4 ml) was added 45 mg 2,2-difluoro-2-(fluorosulfonyl) acetic acid (0.48 eq.) dropwise at 45°C. After the addition, the mixture was stirred at 45°C for 2 h. The mixture was adjusted to pH 7 by addition of saturated sodium hydrogencarbonate solution and concentrated to dryness. The crude product was purified by prep-TLC with petroleum ether/ethyl acetate. (4aR,6S)-tert-Butyl 8-chloro-6-((difluoromethoxy)methyl)-10-fluoro-4,4a,5,6-tetrahydro-1H-benzo[b]pyrazino[1,2-d][1,4]oxazepine-3(2H)-carboxylate was obtained as yellow solid (35 mg, 32 %).

f-2.9) Synthesis of (4aR,6S)-8-Chloro-10-fluoro-6-(D3-methoxymethyl)-2,3,4,4a,5,6-hexahydro-1H-benzo[b]pyrazino[1,2-d][1,4]oxazepine (free base of the compound of example 19) via Boc-deprotection

50 mg (0.124 mmol) of (4aR,6S)-tert-butyl 8-chloro-10-fluoro-6-(D3-methoxymethyl)-4,4a,5,6-tetrahydro-1H-benzo[b]pyrazino[1,2-d][1,4]oxazepine-3(2H)-carboxylate was dissolved in dichloromethane (3 ml) and hydrochloric acid solution (5-6 M in isopropanol, 10.0 eq.) was added and the mixture stirred at room temperature overnight. The reaction mixture was concentrated in vacuo and the residue was portioned between dichloromethane and saturated sodium hydrogencarbonate solution. The organic phase was dried over $MgSO_4$ and evaporated to dryness. The residue was purified by flash chromatography on silica with dichloromethane/methanol. (4aR,6S)-8-Chloro-10-fluoro-6-(D3-methoxymethyl)-2,3,4,4a,5,6-hexahydro-1H-benzo[b]pyrazino[1,2-d][1,4]oxazepine was obtained as oil and used in the salt forming step according to the general procedure (25 mg, 59 % of the hydrochloride).

f-2.10) Synthesis of 8-ethynyl-10-fluoro-6-(methoxymethyl)-4,4a,5,6-tetrahydro-1H-benzo[b]pyrazino[1,2-d][1,4]oxazepine-3(2H)-carboxylate (free base of the boc-protected compound of example 36)

A microwave vial was charged with 91 mg (0.227 mmol) of (4aR,6S)-tert-butyl 8-chloro-10-fluoro-6-(methoxymethyl)-4,4a,5,6-tetrahydro-1H-benzo[b]pyrazino[1,2-d][1,4]oxazepine-3(2H)-carboxylate, 6.9 mg Bis(acetonitrile)dichloropalladium(II) (0.027 eq.), 29 mg 2-dicyclohexylphosphino-2',4',6'-triisopropylbiphenyl (0.061 eq.) and 260 mg cesium carbonate (3.5 eq.) in acetonitrile (6 ml) under argon atmosphere at room temperature. The mixture was stirred for 20 min, before 0.5 ml trimethylsilylacetylene (16.6 eq.) were added. The sealed via was heated to 90 °C for 5.5 h in a microwave oven. The cooled reaction mixture was portioned between water and MTBE. The organic phase was dried over $MgSO_4$ and evaporated to dryness. The residue was purified by flash chromatography on silica with cyclohexane/ethyl acetate.

(4aR,6S)-tert-Butyl 10-fluoro-6-(methoxymethyl)-8-((trimethylsilyl)ethynyl)-4,4a,5,6-tetrahydro- 1H-benzo[b]pyrazino[1,2-d] [1,4]oxazepine-3(2H)-carboxylate was obtained as oil (83 mg, 79 %).

83 mg (0.179 mmol) of (4aR,6S)-tert-butyl 10-fluoro-6-(methoxymethyl)-8-((trimethylsilyl)ethynyl)-4,4a,5,6-tetrahydro-1H-benzo[b]pyrazino[1,2-d][1,4]oxazepine-3(2H)-carboxylate were dissolved in methanol (3 ml) and 0.18 ml aqueous sodium hydroxide solution (1 M, 1.0 eq.) was added. The reaction mixture was stirred at room temperature for 30 min. Then, the solvent was evaporated and crude (4aR,6S)-tert-butyl 8-ethynyl-10-fluoro-6-(methoxymethyl)-4,4a,5,6-tetrahydro-1H-benzo[b]pyrazino[1,2-d][1,4]oxazepine-3(2H)-carboxylate was used in the deprotection, salt forming step according to both general procedures (50 mg, 85 % of hydrochloride).

f-2.11) Synthesis of (4aR,6S)-tert-Butyl 8-cyano-6-(methoxymethyl)-4,4a,5,6-tetra-hydro-1H-benzo[b]pyrazino[1,2-d][1,4]oxazepine-3(2H)-carboxylate (free base of the boc-protected compound of example 12)

150 mg (0.392 mmol) of (4aR,6S)-tert-butyl 8-chloro-6-(methoxymethyl)-4,4a,5,6-tetrahydro-1H-benzo[b]pyrazino-[1,2-d][1,4]oxazepine-3(2H)-carboxylate was dissolved DMA (0.8 ml) under argon atmosphere and 5.3 mg potassium hydrogensulfate (0.1 eq.), 37.4 mg dicyclohexyl(2',4',6'-triisopropyl-[1,1'-biphenyl]-2-yl)phosphine (0.2 eq.), 0.5 mg zinc dust (0.02 eq.) and 28 mg dicyanozinc (0.6 eq.) were successively added. The reaction mixture was stirred for 10 minutes at room temperature, before 8.8 mg diacetoxypalladium(II) (0.1 eq.) were added. The mixture was heated to 120 °C for 3 h, cooled down and evaporated. The residue was purified by flash chromatography on silica with dichloromethane/methanol. (4aR,6S)-tert-Butyl 8-cyano-6-(methoxymethyl)-4,4a,5,6-tetrahydro-1H-benzo[b]pyrazino[1,2-d][1,4]oxazepine-3(2H)-carboxylate was obtained as oil (103 mg, 70 %).

f-2.12) Synthesis of (4aR,6S)-tert-Butyl 8-cyclopropyl-6-(methoxymethyl)-4,4a,5,6-tetrahydro-1H-benzo[b]pyrazino[1,2-d][1,4]oxazepine-3(2H)-carboxylate (free base of the boc-protected compound of example 50)

A microwave vial was charged with 100 mg (0.261 mmol) of (4aR,6S)-tert-butyl 8-chloro-6-(methoxymethyl)-4,4a,5,6-tetrahydro-1H-benzo[b]pyrazino[1,2-d][1,4]oxazepine-3(2H)-carboxylate, 135 mg cyclopropyl boronic acid (6.0 eq.), 444 mg potassium phosphate (8.0 eq.) and 21.4 mg dicyclohexyl(2',6'-dimethoxy-[1,1'-biphenyl]-2-yl)phosphine (0.2 eq.) in toluene (3 ml) and water (1 ml) under argon atmosphere at room temperature. The mixture was stirred for 10 min, then 5.9 mg diacetoxypalladium(II) (0.1 eq.) was finally added. The reaction mixture was sealed and heated to 110 °C for 2 h in a microwave oven. The cooled mixture was diluted with saturated ammonium chloride solution and extracted with ethyl acetate. The organic phase was washed with saturated sodium hydrogencarbonate solution twice, dried over $MgSO_4$ and concentrated in vacuo. The residue was purified by flash chromatography on silica with cyclohexane/ethyl acetate. (4aR,6S)-tert-Butyl 8-cyclopropyl-6-(methoxymethyl)-4,4a,5,6-tetrahydro-1H-benzo[b]pyrazino[1,2-d][1,4]oxazepine-3(2H)-carboxylate was obtained as oil (73 mg, 72 %).

f-2.13) Synthesis of (4aR,6S)-8-(difluoromethyl)-11-fluoro-10-methoxy-6-(methoxymethyl)-2,3,4,4a,5,6-hexahydro-1H-benzo[b]pyrazino[1,2-d][1,4]oxazepine (free base of the compound of example 31)

30 mg (74 μmol) (4aR,6S)-ethyl 8-(difluoromethyl)-10,11-difluoro-6-(methoxymethyl)-4,4a,5,6-tetrahydro-1H-benzo[b]pyrazino[1,2-d][1,4]oxazepine-3(2H)-carboxylate were dissolved in 1.5 ml potassium hydroxide solution (2 M in methanol, 40.6 eq.) and transferred to a microwave vial. The reaction mixture was sealed and heated to 110 °C for 2 h. The solvent was evaporated and the residue partitioned between ethyl acetate and water. The organic phase was washed with water and saturated sodium chloride solution, dried over $MgSO_4$ and concentrated in vacuo. Crude (4aR,6S)-8-(difluoromethyl)-ll-fluoro-10-methoxy-6-(methoxymethyl)-2,3,4,4a,5,6-hexahydro-1H-benzo[b]pyrazino[1,2-d][1,4]oxazepine was obtained as oil and used in the salt forming step according to the general procedure (19 mg, 67 % of hydrochloride).

f-2.14) Synthesis of (4aR,6S)-8-Chloro-10,11-difluoro-9-methoxy-6-(methoxymethyl)-2,3,4,4a,5,6-hexahydro-1H-benzo[b]pyrazino[1,2-d] [1,4]oxazepine (free base of the compound of example 57)

A solution of 26 mg (62 μmol) (4aR,6S)-ethyl 8-chloro-9,10,11-trifluoro-6-(methoxymethyl)-4,4a,5,6-tetrahydro-

1H-benzo[b]pyrazino[1,2-d][1,4]oxazepine-3(2H)-carboxylate in dissolved in 4 ml potassium hydroxide solution (2 M in methanol, 128 eq.) was refluxed at 80 °C for 8 h. The cooled reaction mixture was portioned between saturated sodium chloride solution-water and dichloromethane. The organic phase was washed with water and saturated sodium chloride solution twice. The aqueous phase was back-extracted with dichloromethane, the combined extracts were dried with $Na_2SO_4$ and concentrated in vacuo. The residue was purified by flash chromatography on silica with dichloromethane/methanol. (4aR,6S)-8-Chloro-10,11-difluoro-9-methoxy-6-(methoxymethyl)-2,3,4,4a,5,6-hexahydro-1H-benzo[b]pyrazino[1,2-d][1,4]oxazepine was obtained in sufficient purity as oil (7 mg, 29 %) and used in the salt forming step according to the general procedure (7 mg, purity 85 %, 79 % of hydrochloride).

f-2.15) Synthesis of (4aR,6S)-10,11-Difluoro-6-(methoxymethyl)-2,3,4,4a,5,6-hexahydro-1H-benzo[b]pyrazino[1,2-d][1,4]oxazepine (free base of the compound of example 21)

30 mg (84 μmol) of (4aR,6S)-8-chloro-10,11-difluoro-6-(methoxymethyl)-2,3,4,4a,5,6-hexahydro-1H-benzo[b]pyrazino[1,2-d][1,4]oxazepine hydrochloride was dissolved in 10 ml methanol and hydrogenated in the H-Cube at room temperature under the following conditions in a first run: cartridge: CatCart 10 % Pd/C; pressure: 30 bar; flow rate: 1 ml/min. Given slow conversion, conditions were adjusted for a second, third and fourth run: temperature: 40 °C; pressure: 50 bar; flow rate: 1 ml/min. The solvent was evaporated and the residue was purified by flash chromatography on silica with dichloromethane/methanol. (4aR,6S)-10,11-Difluoro-6-(methoxymethyl)-2,3,4,4a,5,6-hexahydro-1H-benzo[b]pyrazino[1,2-d][1,4]oxazepine was obtained as oil and used in the salt forming step according to the general procedure (6 mg, 22 % of hydrochloride).

In the below examples the names of the synthesized target compounds are followed by a description of their structure. This description does however not include any information on their salt form; this information can be taken from the substances' names. Any discrepancy between name and structure is unintentional; in this case the name is decisive.

1.3 Compound Characterizations

Example 1

(4aR,6S)-8-Chloro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is Cl, $R^5$ is H, $R^6$ is H and $R^7$ is H)

$^1$H NMR (600 MHz, DMSO-d6): δ 1.69 (ddd, J = 15.4, 11.4, 10.2 Hz, 1H), 1.92 (ddd, J = 15.4, 3.7, 2.2 Hz, 1H), 2.63 - 2.74 (m, 1H), 3.12 - 3.25 (m, 3H), 3.26 (s, 3H), 3.36 - 3.43 (m, 2H), 3.53 (dd, J = 10.1, 5.2 Hz, 1H), 3.58 - 3.66 (m, 1H), 3.74 (tt, J = 10.7, 2.5 Hz, 1H), 4.46 - 4.54 (m, 1H), 6.96 - 7.06 (m, 3H), 9.18 (s, 2H).
LC-MS: m/z = 283.1/285.1 [M+H].

Example 2

(4aR,6R)-8-Chloro-6-(methoxymethyl)-2,3,4,4a,5,6-hexahydro-1H-pyrazino[2,1-d][1,5]benzoxazepine; 2,2,2-trifluoroacetic acid

(compound of formula Ia.3 wherein $R^1$ is methyl, $R^4$ is Cl, $R^5$ is H, $R^6$ is H and $R^7$ is H)

$^1$H NMR (500 MHz, DMSO-d6): δ 1.81 - 2.04 (m, 2H), 3.07 - 3.18 (m, 2H), 3.19 - 3.32 (m, 3H), 3.33 (s, 3H), 3.39 - 3.47 (m, 3H), 3.54 (dt, J = 9.9, 5.0 Hz, 1H), 4.63 (dq, J = 9.4, 4.8 Hz, 1H), 6.94 - 7.05 (m, 2H), 7.08 (dd, J = 7.6, 2.1 Hz, 1H), 9.06 (s, 2H). $^{19}$F NMR (471 MHz, DMSO-d6): δ -73.58 (s, TFA).
LC-MS: m/z = 283.1/285.1 [M+H].

Example 3

(4aR,6S)-6-(Methoxymethyl)-2,3,4,4a,5,6-hexahydro-1H-pyrazino[2,1-d][1,5]benzoxazepine; 2,2,2-trifluoroacetic acid

(compound of formula Ia.2 wherein $R^1$ is methyl, $R^4$ is H, $R^5$ is H, $R^6$ is H and $R^7$ is H)

$^1$H NMR (500 MHz, DMSO-d6): δ 1.45 - 1.66 (m, 1H), 1.85 (d, J = 15.4 Hz, 1H), 2.73 (t, J = 11.5 Hz, 1H), 3.07 - 3.27 (m, 5H), 3.29 (s, 3H), 3.57 - 3.77 (m, 3H), 4.36 (dd, J = 11.2, 6.0 Hz, 1H), 6.70 - 7.18 (m, 4H), 8.94 (s, 2H).
$^{19}$F NMR (500 MHz, DMSO-d6): δ -73.54 (s, TFA).
LC-MS: m/z = 249.2 [M+H].

Example 4

(4aR,6R)-6-(Methoxymethyl)-2,3,4,4a,5,6-hexahydro-1H-pyrazino[2,1-d][1,5]benzoxazepine; 2,2,2-trifluoroacetic acid

(compound of formula Ia.3 wherein $R^1$ is methyl, $R^4$ is H, $R^5$ is H, $R^6$ is H and $R^7$ is H)

$^1$H NMR (500 MHz, DMSO-d6): δ 1.76 - 2.02 (m, 2H), 3.08 - 3.64 (m, 9H), 4.57 (d, J = 9.3 Hz, 1H), 6.71 - 7.14 (m, 4H), 8.89 (s, 2H).
$^{19}$F NMR (500 MHz, DMSO-d6): δ -73.51 (s, TFA).
LC-MS: m/z = 249.2 [M+H].

Example 5

(4aR,6S)-8-Chloro-9-fluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is Cl, $R^5$ is F, $R^6$ is H and $R^7$ is H)
$^1$H NMR (500 MHz, DMSO-d6): δ 1.75 (dt, J = 15.6, 10.7 Hz, 1H), 1.92 (d, J = 15.4 Hz, 1H), 2.67 (q, J = 10.9 Hz, 1H), 3.05 - 3.23 (m, 3H), 3.34 - 3.67 (m, 9H), 3.75 (t, J = 10.5 Hz, 1H), 4.54 (dq, J = 8.9, 4.1 Hz, 1H), 6.97 - 7.16 (m, 2H), 9.41 - 9.84 (m, 2H). $^{19}$F NMR (471 MHz, DMSO-d6): δ -121.33 (dd, J = 6.4, 1.9 Hz).
LC-MS: m/z = 301.2/303.2 [M+H].

Example 6

(4aR,6S)-8-(Difluoromethyl)-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is $CHF_2$, $R^5$ is H, $R^6$ is H and $R^7$ is H)
$^1$H NMR (500 MHz, DMSO-d6): δ 1.67 (dt, J = 15.5, 10.8 Hz, 1H), 1.88 (dt, J = 15.3, 3.0 Hz, 1H), 2.70 (dd, J = 12.5, 10.7 Hz, 1H), 3.13 - 3.26 (m, 4H), 3.30 (s, 3H), 3.35 - 3.43 (m, 4H), 3.61 (dt, J = 14.4, 3.1 Hz, 1H), 3.74 (tt, J = 10.5, 2.5 Hz, 1H), 4.50 (ddt, J = 10.4, 7.1, 3.7 Hz, 1H), 7.09 (t, J = 55.4 Hz, 1H), 7.12 - 7.28 (m, 3H), 9.21 (s, 2H). $^{19}$F NMR (471 MHz, DMSO-d6): δ -114.44 (dd, J = 302.2, 55.5 Hz, IF), -107.51 (dd, J = 302.5, 55.9 Hz, IF).
LC-MS: m/z = 299.2 [M+H].

Example 7

(4aR,6S)-8-Chloro-10-fluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is Cl, $R^5$ is H, $R^6$ is F and $R^7$ is H)
$^1$H NMR (500 MHz, DMSO-d6): δ 1.70 (ddd, J = 15.4, 11.4, 10.2 Hz, 1H), 1.92 (ddd, J = 15.6, 4.2, 2.1 Hz, 1H), 2.67 (dd, J = 12.4, 11.2 Hz, 1H), 3.17 (pd, J = 12.4, 3.0 Hz, 3H), 3.25 (s, 3H), 3.37 (dd, J = 10.2, 4.7 Hz, 1H), 3.42 (ddd, J = 14.6, 11.8, 3.2 Hz, 1H), 3.49 (dd, J = 10.1, 5.2 Hz, 1H), 3.65 (dt, J = 14.7, 2.7 Hz, 1H), 3.80 (tt, J = 11.0, 2.5 Hz, 1H), 4.48 (dq, J = 11.5, 4.8 Hz, 1H), 6.87 - 7.00 (m, 2H), 9.30 (s, 2H).
$^{19}$F NMR (471 MHz, DMSO-d6): δ -116.94 (t, J = 9.2 Hz).
LC-MS: m/z = 301.1/303.1 [M+H].

Example 8

(4aR,6S)-10-Fluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is H, $R^5$ is H, $R^6$ is F and $R^7$ is H)
$^1$H NMR (500 MHz, DMSO-d6): δ 1.52 (dt, J = 15.3, 10.6 Hz, 1H), 1.85 (ddd, J = 15.4, 4.5, 1.8 Hz, 1H), 2.67 (t, J = 11.8 Hz, 1H), 3.07 - 3.27 (m, 3H), 3.29 (s, 3H), 3.38 (dd, J = 10.6, 7.1 Hz, 1H), 3.41 - 3.58 (m, 2H), 3.65 (dt, J = 14.2, 2.7 Hz, 1H), 3.76 - 3.85 (m, 1H), 4.35 (ddt, J = 11.5, 8.0, 4.4 Hz, 1H), 6.65 (td, J = 8.3, 3.0 Hz, 1H), 6.80 (dd, J = 8.7, 6.1 Hz, 1H), 6.93 (dd, J = 11.1, 3.0 Hz, 1H), 9.27 (s, 2H).
$^{19}$F NMR (471 MHz, DMSO-d6): δ -118.05 - -117.85 (m).
LC-MS: m/z = 267.2 [M+H].

Example 9

(4aR,6S)-8-(Difluoromethyl)-10-fluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is $CHF_2$, $R^5$ is H, $R^6$ is F and $R^7$ is H)
$^1$H NMR (500 MHz, DMSO-d6): δ 1.66 (ddd, J = 15.4, 11.5, 10.2 Hz, 1H), 1.88 (ddd, J = 15.6, 3.6, 2.0 Hz, 1H), 2.69 (dd, J = 12.6, 10.9 Hz, 1H), 3.12 - 3.26 (m, 3H), 3.29 (s, 3H), 3.32 - 3.49 (m, 3H), 3.65 (dt, J = 14.5, 2.8 Hz, 1H), 3.81 (tt, J = 11.0, 2.4 Hz, 1H), 4.51 (ddt, J = 10.7, 7.0, 3.5 Hz, 1H), 6.89 (dd, J = 8.3, 2.9 Hz, 1H), 7.06 (t, J = 55.4 Hz, 1H), 7.15 (dd, J = 10.8, 3.0 Hz, 1H), 9.33 (s, 2H).
$^{19}$F NMR (471 MHz, DMSO-d6): δ -116.13 (t, J = 9.7 Hz, IF), -115.17 (dd, J = 303.9, 54.9 Hz, IF), -107.77 (dd, J = 304.4, 55.6 Hz, IF).
LC-MS: m/z = 317.3 [M+H].

Example 10

(4aR,6S)-10-Chloro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is H, $R^5$ is H, $R^6$ is Cl and $R^7$ is H)
$^1$H NMR (500 MHz, DMSO-d6): δ 1.54 (ddd, J = 15.3, 11.3, 9.9 Hz, 1H), 1.86 (ddd, J = 15.3, 4.4, 1.9 Hz, 1H), 2.67 (q, J = 10.9 Hz, 1H), 3.08 - 3.28 (m, 4H), 3.29 (s, 3H), 3.35 - 3.45 (m, 2H), 3.66 (dt, J = 14.3, 2.7 Hz, 1H), 3.79 (tt, J = 10.8, 2.3 Hz, 1H), 4.39 (ddt, J = 11.3, 6.9, 4.2 Hz, 1H), 6.81 (d, J = 8.5 Hz, 1H), 6.88 (dd, J = 8.3, 2.5 Hz, 1H), 7.13 (d, J = 2.5 Hz, 1H), 9.12 - 9.48 (m, 2H).
LC-MS: m/z = 283.3/285.2 [M+H].

Example 11

(4aR,6S)-8,10-Difluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is F, $R^5$ is H, $R^6$ is F and $R^7$ is H)
$^1$H NMR (500 MHz, DMSO-d6): δ 1.68 (ddd, J = 15.3, 11.6, 10.0 Hz, 1H), 1.90 (ddd, J = 15.4, 4.5, 1.7 Hz, 1H), 2.67 (t, J = 12.0 Hz, 1H), 3.12 (dt, J = 13.3, 6.4 Hz, 1H), 3.21 (ddd, J = 12.1, 9.3, 2.6 Hz, 2H), 3.25 (s, 3H), 3.29 - 3.35 (m, 1H), 3.36 - 3.44 (m, 2H), 3.69 (dt, J = 14.2, 2.7 Hz, 1H), 3.85 (s, 1H), 4.44 (dq, J = 10.0, 4.6 Hz, 1H), 6.71 - 6.84 (m, 2H), 9.31 (s, 2H).
$^{19}$F NMR (471 MHz, DMSO-d6): δ -127.54 (d, J = 12.3 Hz, IF), -115.84 (t, J = 11.5 Hz, IF).
LC-MS: m/z = 285.3 [M+H].

Example 12

(4aR,6S)-6-(Methoxymethyl)-2,3,4,4a,5,6-hexahydro-1H-pyrazino[2,1-d][1,5]benzoxazepine-8-carbonitrile hydrochloride

(compound of formula Ia.2 wherein $R^1$ is methyl, $R^4$ is CN, $R^5$ is H, $R^6$ is H and $R^7$ is H)
$^1$H NMR (600 MHz, DMSO-d6): δ 1.80 (dt, J = 15.7, 11.0 Hz, 1H), 1.93 (dt, J = 15.6, 3.0 Hz, 1H), 2.67 (dd, J = 12.6, 11.1 Hz, 1H), 3.12 - 3.25 (m, 3H), 3.27 (s, 3H), 3.40 - 3.47 (m, 1H), 3.49 - 3.58 (m, 2H), 3.62 - 3.68 (m, 1H), 3.78 (tt, J = 10.9, 2.6 Hz, 1H), 4.62 (dq, J = 11.5, 3.9 Hz, 1H), 7.13 (t, J = 7.9 Hz, 1H), 7.27 (dd, J = 7.7, 1.5 Hz, 1H), 7.37 (dd, J = 8.1, 1.5 Hz, 1H), 9.17 (s, 2H).
LC-MS: m/z = 274.2 [M+H].

Example 13

(4aR,6S)-10-Fluoro-6-(methoxymethyl)-2,3,4,4a,5,6-hexahydro-1H-pyrazino[2,1-d][1,5]benzoxazepine-8-carbonitrile hydrochloride

(compound of formula Ia.2 wherein $R^1$ is methyl, $R^4$ is CN, $R^5$ is H, $R^6$ is F and $R^7$ is H)
$^1$H NMR (500 MHz, DMSO-d6): δ 1.83 (dt, J = 15.7, 11.0 Hz, 1H), 1.89 - 1.98 (m, 1H), 2.67 (t, J = 11.9 Hz, 1H), 3.10 - 3.23 (m, 3H), 3.26 (s, 3H), 3.42 - 3.53 (m, 2H), 3.56 (dd, J = 10.3, 3.9 Hz, 1H), 3.68 (dt, J = 14.7, 2.6 Hz, 1H), 3.85 (tt,

J = 11.1, 2.5 Hz, 1H), 4.59 (dq, J = 11.5, 3.9 Hz, 1H), 7.20 (dd, J = 7.7, 3.0 Hz, 1H), 7.30 (dd, J = 10.9, 3.0 Hz, 1H), 9.35 (s, 2H).
$^{19}$F NMR (471 MHz, DMSO-d6): δ -116.37 (t, J = 10.2, 8.3 Hz).
LC-MS: m/z = 292.2 [M+H].

Example 14

[(4aR,6S)-8-Chloro-2,3,4,4a,5,6-hexahydro-1H-pyrazino[2,1-d][1,5]benzoxazepin-6-yl]methanol hydrochloride

(compound of formula Ia.2 wherein $R^1$ is H, $R^4$ is Cl, $R^5$ is H, $R^6$ is H and $R^7$ is H)
$^1$H NMR (600 MHz, DMSO-d6): δ 1.64 (dt, J = 15.4, 10.5 Hz, 1H), 2.00 (dt, J = 15.3, 3.1 Hz, 1H), 2.74 (s, 1H), 3.13 - 3.26 (m, 3H), 3.30 - 3.42 (m, 2H), 3.58 - 3.64 (m, 1H), 3.67 (t, J = 10.4 Hz, 1H), 3.70 - 3.76 (m, 1H), 4.28 - 4.35 (m, 1H), 4.86 (t, J = 5.4 Hz, 1H), 6.95 - 7.05 (m, 3H), 8.95 (s, 2H).
LC-MS: m/z = 269.3/271.3 [M+H].

Example 15

(4aR,6S)-8-Chloro-11-fluoro-6-(methoxymethyl)-2,3,4,4a,5,6-hexahydro-1H-pyrazino[2,1-d][1,5]benzoxazepine; fumaric acid

(compound of formula Ia.2 wherein $R^1$ is methyl, $R^4$ is Cl, $R^5$ is H, $R^6$ is H and $R^7$ is F)
$^1$H NMR (500 MHz, Methanol-d4): δ 1.87 (ddd, J = 15.3, 4.6, 1.2 Hz, 1H), 1.95 - 2.13 (m, 1H), 2.97 (t, J = 11.2 Hz, 1H), 3.24 - 3.29 (m, 2H), 3.30 - 3.32 (m, 4H), 3.33 - 3.38 (m, 2H), 3.45 - 3.61 (m, 3H), 3.86 (t, J = 10.1 Hz, 1H), 4.52 (s, 1H), 6.69 (s, 2H, fumerate), 6.89 (dd, J = 10.8, 8.9 Hz, 1H), 7.07 (dd, J = 9.0, 5.4 Hz, 1H).
$^{19}$F NMR (471 MHz, Methanol-d4): δ -124.16 (s).
LC-MS: m/z = 301.3/303.3 [M+H].

Example 16

(4aR,6S)-8-Chloro-10,11-difluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is Cl, $R^5$ is H, $R^6$ is F and $R^7$ is F)
$^1$H NMR (600 MHz, DMSO-d6): δ 1.77 - 1.94 (m, 2H), 2.81 (t, J = 11.5 Hz, 1H), 3.05 (td, J = 12.0, 3.5 Hz, 1H), 3.22 (s, 3H), 3.24 - 3.33 (m, 3H), 3.37 - 3.48 (m, 3H), 3.84 (dd, J = 11.3, 6.9 Hz, 1H), 4.53 (dd, J = 9.7, 5.2 Hz, 1H), 7.32 (dd, J = 10.1, 7.9 Hz, 1H), 9.23 (s, 2H).
$^{19}$F NMR (471 MHz, DMSO-d6): δ -146.36 (d, J = 20.6 Hz, IF), -141.00 (dd, J = 22.7, 10.2 Hz, IF).
LC-MS: m/z = 319.2/321.2 [M+H].

Example 17

[(4aR,6S)-8-Chloro-10-fluoro-2,3,4,4a,5,6-hexahydro-1H-pyrazino[2,1-d][1,5]benzoxazepin-6-yl]methanol hydrochloride

(compound of formula Ia.2 wherein $R^1$ is H, $R^4$ is Cl, $R^5$ is H, $R^6$ is F and $R^7$ is H)
$^1$H NMR (500 MHz, DMSO-d6): δ 1.63 (dt, J = 15.6, 10.6 Hz, 1H), 1.99 (ddd, J = 15.6, 3.9, 2.3 Hz, 1H), 2.71 (dd, J = 12.5, 11.1 Hz, 1H), 3.19 (ddd, J = 20.0, 12.4, 3.0 Hz, 3H), 3.35 - 3.44 (m, 2H), 3.59 - 3.77 (m, 3H), 4.26 - 4.35 (m, 1H), 4.85 (t, J = 5.2 Hz, 1H), 6.90 - 6.98 (m, 2H), 9.01 (s, 2H).
$^{19}$F NMR (471 MHz, DMSO-d6): δ -118.36 - -115.09 (m).
LC-MS: m/z = 287.3/289.3 [M+H].

Example 18

(4aR,6S)-8,10,11-Trifluoro-6-(methoxymethyl)-2,3,4,4a,5,6-hexahydro-1H-pyrazino[2,1-d][1,5]benzoxazepine; fumaric acid

(compound of formula Ia.2 wherein $R^1$ is methyl, $R^4$ is F, $R^5$ is H, $R^6$ is F and $R^7$ is F)
$^1$H NMR (500 MHz, Methanol-d4): δ 1.82 - 1.99 (m, 2H), 2.95 (dd, J = 12.3, 11.0 Hz, 1H), 3.24 - 3.34 (m, 5H), 3.34 -

3.48 (m, 5H), 3.57 - 3.64 (m, 1H), 3.95 (t, J = 9.9 Hz, 1H), 4.51 (dq, J = 10.1, 4.9 Hz, 1H), 6.70 (s, 2H), 6.85 (td, J = 10.2, 7.4 Hz, 1H).
$^{19}$F NMR (471 MHz, Methanol-d4): δ -153.98 - -153.54 (m, IF), -142.24 (dd, J = 20.9, 10.5 Hz, IF), -134.30 (t, J = 10.0 Hz, IF).
LC-MS: m/z = 303.3 [M+H].

Example 19

(4aR,6S)-8-Chloro-10-fluoro-6-(trideuteriomethoxymethyl)-2,3,4,4a,5,6-hexahydro-1H-pyrazino[2,1-d][1,5]benzoxazepine hydrochloride

(compound of formula Ia.2 wherein $R^1$ is $CD_3$, $R^4$ is Cl, $R^5$ is H, $R^6$ is F and $R^7$ is H)
$^1$H NMR (500 MHz, DMSO-d6): δ 1.70 (dt, J = 15.5, 11.1 Hz, 1H), 1.91 (ddd, J = 15.5, 4.0, 2.0 Hz, 1H), 2.68 (t, J = 11.8 Hz, 1H), 3.10 - 3.24 (m, 3H), 3.34 - 3.45 (m, 2H), 3.50 (dd, J = 10.2, 5.2 Hz, 1H), 3.65 (dt, J = 14.8, 2.5 Hz, 1H), 3.73 - 3.84 (m, 1H), 4.48 (dq, J = 11.2, 4.7 Hz, 1H), 6.90 - 6.98 (m, 2H), 9.14 (s, 2H).
$^{19}$F NMR (471 MHz, DMSO-d6): δ -116.95 (t, J = 9.7 Hz).
LC-MS: m/z = 304.3/306.3 [M+H].

Example 20

[(4aR,6S)-8-Chloro-10,11-difluoro-2,3,4,4a,5,6-hexahydro-1H-pyrazino[2,1-d][1,5]benzoxazepin-6-yl]methanol hydrochloride

(compound of formula Ia.2 wherein $R^1$ is H, $R^4$ is Cl, $R^5$ is H, $R^6$ is F and $R^7$ is F)
$^1$H NMR (500 MHz, DMSO-d6): δ 1.76 - 1.90 (m, 1H), 1.94 (dd, J = 14.9, 4.4 Hz, 1H), 2.84 (t, J = 11.4 Hz, 1H), 3.00 - 3.12 (m, 1H), 3.17 (s, 1H), 3.21 - 3.41 (m, 3H), 3.41 - 3.49 (m, 1H), 3.65 (dt, J = 9.8, 4.6 Hz, 1H), 3.84 (t, J = 10.2 Hz, 1H), 4.28 - 4.42 (m, 1H), 4.83 (t, J = 5.3 Hz, 1H), 7.31 (dd, J = 10.1, 8.0 Hz, 1H), 9.36 (s, 2H).
$^{19}$F NMR (471 MHz, DMSO-d6): δ -146.31 (s, 1F), -141.13 (dd, J = 22.9, 10.1 Hz, IF). LC-MS: m/z = 305.2/307.2 [M+H].

Example 21

(4aR,6S)-10,11-Difluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is H, $R^5$ is H, $R^6$ is F and $R^7$ is F)
$^1$H NMR (500 MHz, DMSO-d6): δ 1.59 (q, J = 14.4, 12.9 Hz, 1H), 1.84 (dd, J = 15.1, 5.1 Hz, 1H), 2.80 (t, J = 11.5 Hz, 1H), 3.03 - 3.12 (m, 1H), 3.23 (td, J = 10.7, 3.3 Hz, 3H), 3.27 (s, 3H), 3.29 - 3.36 (m, 2H), 3.44 (dt, J = 14.1, 3.5 Hz, 1H), 3.86 (t, J = 9.9 Hz, 1H), 4.39 (ddt, J = 11.9, 8.3, 4.5 Hz, 1H), 6.70 (ddd, J = 9.1, 5.4, 2.0 Hz, 1H), 6.97 (q, J = 9.2 Hz, 1H), 9.13 (s, 2H).
$^{19}$F NMR (471 MHz, DMSO-d6): δ -145.90 (d, J = 21.3 Hz, IF), -141.95 (ddd, J = 21.5, 9.6, 4.8 Hz, IF).
LC-MS: m/z = 285.3 [M+H].

Example 22

(4aR,6S)-10-Fluoro-6-(methoxymethyl)-8-(trifluoromethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is $CF_3$, $R^5$ is H, $R^6$ is F and $R^7$ is H)
$^1$H NMR (600 MHz, DMSO-d6): δ 1.81 (dt, J = 15.8, 9.7 Hz, 1H), 2.02 - 2.09 (m, 1H), 2.83 (s, 1H), 3.15 - 3.25 (m, 3H), 3.29 (d, J = 2.3 Hz, 3H), 3.40 - 3.45 (m, 2H), 3.52 - 3.64 (m, 3H), 4.45 (dtd, J = 9.6, 5.5, 2.8 Hz, 1H), 7.10 (dd, J = 8.3, 3.0 Hz, 1H), 7.28 (dd, J = 10.5, 3.0 Hz, 1H), 9.11 - 9.29 (m, 2H).
$^{19}$F NMR (471 MHz, DMSO-d6): δ -116.92 (t, J = 9.4 Hz, IF), -59.76 (s, $CF_3$).
LC-MS: m/z = 335.3 [M+H].

Example 23:

[(4aR,6S)-8-(Difluoromethyl)-10-fluoro-2,3,4,4a,5,6-hexahydro-1H-pyrazino[2,1-d][1,5]benzoxazepin-6-yl]methanol hydrochloride

(compound of formula Ia.2 wherein $R^1$ is H, $R^4$ is $CHF_2$, $R^5$ is H, $R^6$ is F and $R^7$ is H)
$^1$H NMR (500 MHz, DMSO-$d_6$): δ 1.61 (dt, *J* = 15.4, 10.8 Hz, 1H), 1.88 (dt, *J* = 15.4, 2.8 Hz, 1H), 2.65 - 2.76 (m, 1H), 3.17 (s, 1H), 3.18 - 3.25 (m, 3H), 3.41 - 3.45 (m, 1H), 3.47 (dd, *J* = 11.8, 3.7 Hz, 1H), 3.65 (dt, *J* = 14.4, 2.8 Hz, 1H), 3.76 (tt, *J* = 10.7, 2.5 Hz, 1H), 4.33 (ddt, *J* = 10.7, 7.2, 3.7 Hz, 1H), 5.13 (s, 1H), 6.88 (dd, *J* = 8.2, 3.0 Hz, 1H), 7.13 (dd, *J* = 10.8, 3.0 Hz, 1H), 7.27 (t, *J* = 54.9 Hz, 1H), 9.17 (s, 2H).
$^{19}$F NMR (471 MHz, DMSO-$d_6$): δ -117.25 - -116.30 (m, IF), -107.68 (dd, *J* = 302.4, 55.5 Hz, 2F).
LC-MS: m/z = 303.2 [M+H].

Example 24

(4aR,6S)-8-Chloro-6-(difluoromethoxymethyl)-10-fluoro-2,3,4,4a,5,6-hexahydro-1H-pyrazino[2,1-d][1,5]benzoxazepine hydrochloride

(compound of formula Ia.2 wherein $R^1$ is $CHF_2$, $R^4$ is Cl, $R^5$ is H, $R^6$ is F and $R^7$ is H)
$^1$H NMR (500 MHz, DMSO-$d_6$): δ 1.73 (dt, *J* = 15.5, 10.8 Hz, 1H), 1.96 (ddd, *J* = 15.6, 3.9, 2.2 Hz, 1H), 2.68 (t, *J* = 11.8 Hz, 1H), 3.17 (dt, *J* = 12.1, 3.4 Hz, 3H), 3.45 (ddd, *J* = 14.6, 11.1, 3.7 Hz, 1H), 3.65 (dt, *J* = 13.8, 2.4 Hz, 1H), 3.83 (t, *J* = 10.7 Hz, 1H), 3.89 (dd, *J* = 10.7, 4.3 Hz, 1H), 3.95 (dd, *J* = 10.7, 5.7 Hz, 1H), 4.60 (dq, J = 11.3, 4.4 Hz, 1H), 6.71 (t, *J* = 75.9 Hz, 1H), 6.92 - 7.01 (m, 2H), 9.33 (s, 2H).
$^{19}$F NMR (471 MHz, DMSO-$d_6$): δ -116.64 - -116.54 (m, IF), -83.20 (d, *J* = 7.1 Hz, IF), -83.04 (d, *J* = 7.1 Hz, IF).
LC-MS: m/z = 337.1/339.1 [M+H].

Example 25

(4aR,6S)-10-Chloro-8-fluoro-6-(methoxymethyl)-2,3,4,4a,5,6-hexahydro-1H-pyrazino[2,1-d][1,5]benzoxazepine

(compound of formula Ia.2 wherein $R^1$ is methyl, $R^4$ is F, $R^5$ is H, $R^6$ is Cl and $R^7$ is H)
$^1$H NMR (500 MHz, DMSO-$d_6$): δ 1.68 (ddd, *J* = 15.5, 11.5, 10.0 Hz, 1H), 1.87 (ddd, *J* = 15.5, 4.3, 1.8 Hz, 1H), 2.60 (t, *J* = 11.8 Hz, 1H), 3.01 - 3.18 (m, 3H), 3.25 (s, 3H), 3.27 - 3.45 (m, 3H), 3.66 (dt, *J* = 14.1, 2.7 Hz, 1H), 3.78 (t, *J* = 10.5 Hz, 1H), 4.47 (dq, *J* = 9.9, 4.6 Hz, 1H), 6.88 - 6.99 (m, 2H), 8.75 (s, 1H).
$^{19}$F NMR (471 MHz, DMSO-$d_6$): δ -128.61 (d, *J* = 9.4 Hz).
LC-MS: m/z = 301.1/303.1 [M+H].

Example 26

(4aR,6S)-8-(Difluoromethyl)-10,11-difluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is $CHF_2$, $R^5$ is H, $R^6$ is F and $R^7$ is F)
$^1$H NMR (500 MHz, DMSO-$d_6$): δ 1.71 - 1.91 (m, 2H), 2.78 - 2.89 (m, 1H), 3.02 - 3.13 (m, 1H), 3.20 - 3.39 (m, 8H), 3.45 (dt, *J* = 14.7, 3.5 Hz, 1H), 3.87 (t, *J* = 9.5 Hz, 1H), 4.54 (s, 1H), 7.10 (t, *J* = 54.8 Hz, 1H), 7.27 (t, *J* = 9.2 Hz, 1H), 9.35 (s, 2H). $^{19}$F NMR (471 MHz, DMSO-$d_6$): δ -140.89 (br. s, 2F), -140.11 - -139.85 (m, 2F).
LC-MS: m/z = 335.2 [M+H].

Example 27

(6S,7aR)-4-(Difluoromethyl)-6-(methoxymethyl)-7,7a,8,9,10,11-hexahydro-6H-pyrazino[1,2-d]pyrido[3,2-b][1,4]oxazepine; 2,2,2-trifluoroacetic acid

(compound of formula Ia.11 wherein $R^1$ is methyl, $R^4$ is $CHF_2$, $R^5$ is H and $R^6$ is H)
$^1$H NMR (500 MHz, DMSO-$d_6$): δ 1.78 (dt, *J* = 15.8, 11.2 Hz, 1H), 2.02 (dt, *J* = 15.6, 2.9 Hz, 1H), 2.69 (t, *J* = 12.0 Hz, 1H), 3.17 - 3.32 (m, 6H), 3.45 (dd, *J* = 10.6, 5.4 Hz, 1H), 3.53 (dd, *J* = 10.6, 3.8 Hz, 1H), 3.86 (tt, *J* = 11.3, 3.1 Hz, 1H), 4.06 - 4.14 (m, 1H), 4.60 - 4.68 (m, 1H), 7.04 (t, *J* = 54.9 Hz, 1H), 7.07 (d, *J* = 5.1 Hz, 1H), 8.07 (d, *J* = 5.1 Hz, 1H), 8.95 (s, 2H).
$^{19}$F NMR (471 MHz, DMSO-$d_6$): δ -117.81 (dd, *J* = 307.1, 54.8 Hz, IF), -111.93 (dd, *J* = 307.4, 55.3 Hz, IF), -73.51(s, TFA).

LC-MS: m/z = 300.2 [M+H].

Example 28

(4aR,6S)-10,11-Difluoro-6-(methoxymethyl)-2,3,4,4a,5,6-hexahydro-1H-pyrazino[2,1-d][1,5]benzoxazepine-8-carbonitrile hydrochloride

(compound of formula Ia.2 wherein $R^1$ is methyl, $R^4$ is CN, $R^5$ is H, $R^6$ is F and $R^7$ is F)
$^1$H NMR (500 MHz, DMSO-$d_6$): δ 1.80 - 2.08 (m, 2H), 2.76 - 2.90 (m, 1H), 3.05 (d, *J* = 10.8 Hz, 1H), 3.23 (s, 3H), 3.26 - 3.40 (m, 3H), 3.42 - 3.52 (m, 2H), 3.59 (dd, *J* = 10.5, 3.5 Hz, 1H), 3.88 (t, *J* = 10.3 Hz, 1H), 4.63 (dq, *J* = 11.9, 4.1 Hz, 1H), 7.62 (dd, *J* = 9.8, 8.2 Hz, 1H), 9.36 (d, *J* = 57.2 Hz, 2H).
$^{19}$F NMR (471 MHz, DMSO-$d_6$): δ -139.90 (dd, *J* = 22.0, 9.8 Hz, IF), -136.61 (d, *J*= 22.1 Hz, IF).
LC-MS: m/z = 310.3 [M+H].

Example 29

(4aR,6S)-8-(Difluoromethyl)-11-fluoro-10-methoxy-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is $CHF_2$, $R^5$ is H, $R^6$ is $OCH_3$ and $R^7$ is F)
$^1$H NMR (600 MHz, DMSO-$d_6$): δ 1.45 - 1.92 (m, 2H), 2.84 (s, 1H), 3.08 (t, *J* = 10.5 Hz, 2H), 3.21 - 3.33 (m, 7H), 3.46 (dt, *J* = 13.6, 3.4 Hz, 1H), 3.74 - 3.97 (m, 4H), 4.49 (s, 1H), 6.96 - 7.26 (m, 2H), 9.35 (s, 2H).
$^{19}$F NMR (471 MHz, DMSO-$d_6$): δ -132.22 (s, IF), -117.21 - -113.51 (m, IF), -106.49 - -102.62 (m, IF).
LC-MS: m/z = 347.2 [M+H].

Example 30

(4aR,6S)-8-Fluoro-6-(methoxymethyl)-9-methyl-2,3,4,4a,5,6-hexahydro-1H-pyrazino[2,1-d] [1,5]benzoxazepine

(compound of formula Ia.2 wherein $R^1$ is methyl, $R^4$ is F, $R^5$ is $CH_3$, $R^6$ is H and $R^7$ is H)
$^1$H NMR (600 MHz, Methanol-$d_4$): δ 1.83 - 1.90 (m, 2H), 2.18 (d, *J* = 1.9 Hz, 3H), 2.90 (dd, *J* = 12.3, 10.6 Hz, 1H), 3.26 - 3.33 (m, 2H), 3.33 - 3.38 (m, 6H), 3.40 (dd, *J* = 10.4, 4.5 Hz, 1H), 3.50 (ddd, *J* = 10.2, 6.3, 0.9 Hz, 1H), 3.72 (dt, *J* = 14.1, 3.0 Hz, 1H), 3.75 - 3.83 (m, 1H), 4.45 (dtd, *J* = 10.7, 6.2, 4.5 Hz, 1H), 6.74 (dd, *J* = 8.4, 1.7 Hz, 1H), 6.87 (td, *J* = 8.3, 0.9 Hz, 1H).
$^{19}$F NMR (471 MHz, DMSO-$d_6$): δ -135.17 (d, *J* = 6.8 Hz).
LC-MS: m/z = 281.3 [M+H].

Example 31

(4aR,6S)-10-Chloro-9-fluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is H, $R^5$ is F, $R^6$ is Cl and $R^7$ is H)
$^1$H NMR (600 MHz, DMSO-$d_6$): δ 1.60 (dt, *J* = 15.3, 10.7 Hz, 1H), 1.87 (ddd, *J* = 15.4, 4.0, 2.3 Hz, 1H), 2.67 (dd, *J* = 12.4, 10.8 Hz, 1H), 3.09 - 3.20 (m, 3H), 3.26 - 3.33 (m, 5H), 3.42 (dd, *J* = 10.6, 6.5 Hz, 1H), 3.59 (dt, *J* = 14.1, 2.8 Hz, 1H), 3.67 (tt, *J* = 10.6, 2.5 Hz, 1H), 4.42 (ddt, *J* = 10.7, 6.5, 4.1 Hz, 1H), 6.87 (d, *J* = 9.8 Hz, 1H), 7.27 (d, *J* = 7.9 Hz, 1H), 8.79 (s, 2H).
$^{19}$F NMR (471 MHz, DMSO-$d_6$): δ -123.51 (t, *J* = 9.0 Hz).
LC-MS: m/z = 301.2/303.2 [M+H].

Example 32

(4aR,6R)-8-(Difluoromethyl)-10-fluoro-6-(methoxymethyl)-2,3,4,4a,5,6-hexahydro-1H-pyrazino[2,1-d][1,5]benzoxazepine hydrochloride

(compound of formula Ia.3 wherein $R^1$ is methyl, $R^4$ is $CHF_2$, $R^5$ is H, $R^6$ is F and $R^7$ is H)
$^1$H NMR (500 MHz, DMSO-$d_6$): δ 1.90 - 2.01 (m, 2H), 3.11 (d, *J* = 13.5 Hz, 2H), 3.17 - 3.25 (m, 1H), 3.25 - 3.38 (m, 6H), 3.38 - 3.52 (m, 3H), 4.53 - 4.67 (m, 1H), 6.78 - 7.17 (m, 3H), 9.34 (s, 2H).

$^{19}F$ NMR (471 MHz, DMSO-$d_6$): δ -117.09 (t, *J* = 9.9 Hz, IF), -114.51 (dd, *J* = 300.1, 55.2 Hz, IF), -110.83 (dd, *J* = 300.4, 55.8 Hz, IF).
LC-MS: m/z = 317.2 [M+H].

Example 33

(4aR,6S)-8-Fluoro-6-(methoxymethyl)-10-methyl-2,3,4,4a,5,6-hexahydro-1H-pyrazino[2,1-d] [1,5]benzoxazepine

(compound of formula Ia.2 wherein $R^1$ is methyl, $R^4$ is F, $R^5$ is H, $R^6$ is $CH_3$ and $R^7$ is H)
$^1H$ NMR (600 MHz, Methanol-$d_4$): δ 1.64 - 1.74 (m, 2H), 2.22 (s, 3H), 2.46 (dd, *J* = 12.3, 10.4 Hz, 1H), 2.82 - 2.87 (m, 1H), 2.88 - 2.94 (m, 2H), 3.14 (ddd, *J* = 13.1, 9.9, 4.5 Hz, 1H), 3.35 (s, 4H), 3.44 (ddd, *J* = 10.2, 6.7, 1.0 Hz, 1H), 3.48 - 3.56 (m, 2H), 4.34 - 4.41 (m, 1H), 6.46 (ddd, *J* = 10.5, 2.0, 0.8 Hz, 1H), 6.57 (d, *J* = 2.1 Hz, 1H).
$^{19}F$ NMR (471 MHz, DMSO-$d_6$): δ -132.20 (d, *J* = 59.4 Hz).
LC-MS: m/z = 281.3 [M+H].

Example 34

(4aR,6S)-8-Ethynyl-10-fluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is -C≡CH, $R^5$ is H, $R^6$ is F and $R^7$ is H)
$^1H$ NMR (500 MHz, DMSO-$d_6$): δ 1.61 - 1.74 (m, 1H), 1.92 (ddd, *J* = 15.6, 4.0, 2.2 Hz, 1H), 2.67 (dd, *J* = 12.5, 11.0 Hz, 1H), 3.08 - 3.23 (m, 3H), 3.25 (s, 3H), 3.35 - 3.47 (m, 2H), 3.56 (dd, *J* = 10.0, 4.9 Hz, 1H), 3.63 (dt, *J* = 14.5, 2.8 Hz, 1H), 3.77 (tt, *J* = 10.8, 2.5 Hz, 1H), 4.25 (s, 1H), 4.45 (dq, *J* = 11.2, 4.9 Hz, 1H), 6.80 (dd, *J* = 8.3, 3.0 Hz, 1H), 7.00 (dd, *J* = 10.9, 3.1 Hz, 1H), 9.27 (s, 2H).
$^{19}F$ NMR (471 MHz, DMSO-$d_6$): δ -117.93 (t, *J* = 10.1 Hz).
LC-MS: m/z = 291.2 [M+H].

Example 35

(4aR,6R)-8-Chloro-10-fluoro-6-(methoxymethyl)-2,3,4,4a,5,6-hexahydro-1H-pyrazino[2,1-d][1,5]benzoxazepine hydrochloride

(compound of formula Ia.3 wherein $R^1$ is methyl, $R^4$ is Cl, $R^5$ is H, $R^6$ is F and $R^7$ is H)
$^1H$ NMR (500 MHz, DMSO-$d_6$): δ 1.93 (dddd, *J* = 25.5, 15.7, 10.8, 4.8 Hz, 2H), 3.09 (d, *J* = 10.1 Hz, 2H), 3.22 (d, *J* = 12.2 Hz, 1H), 3.26 - 3.34 (m, 6H), 3.38 - 3.48 (m, 2H), 3.52 (dd, *J* = 10.3, 5.4 Hz, 1H), 4.53 (dq, *J* = 9.2, 4.7 Hz, 1H), 6.90 (dd, *J* = 10.4, 3.0 Hz, 1H), 7.00 (dd, *J* = 8.2, 3.0 Hz, 1H), 9.25 (s, 2H).
$^{19}F$ NMR (471 MHz, DMSO-$d_6$): δ -117.47 (t, *J* = 10.1 Hz).
LC-MS: m/z = 301.2/303.1 [M+H].

Example 36

(4aR,6S)-10-Ethynyl-8-fluoro-6-(methoxymethyl)-2,3,4,4a,5,6-hexahydro-1H-pyrazino[2,1-d][1,5]benzoxazepine; (2R,3R)-2,3-dihydroxybutanedioic acid

(compound of formula Ia.2 wherein $R^1$ is methyl, $R^4$ is F, $R^5$ is H, $R^6$ is -C≡CH and $R^7$ is H)
$^1H$ NMR (500 MHz, DMSO-$d_6$): δ 1.68 (dt, *J* = 15.5, 10.8 Hz, 1H), 1.82 - 1.92 (m, 1H), 2.64 (t, *J* = 11.6 Hz, 1H), 3.06 - 3.21 (m, 2H), 3.25 (s, 3H), 3.29 - 3.47 (m, 4H), 3.61 - 3.78 (m, 2H), 4.06 (s, 2H, tartrate), 4.17 (s, 1H), 4.50 (dq, *J* = 9.5, 4.6 Hz, 1H), 6.87 - 7.02 (m, 2H).
$^{19}F$ NMR (471 MHz, DMSO-$d_6$): δ -130.46 (d, *J* = 10.2 Hz).
LC-MS: m/z = 291.2 [M+H].

Example 37

(4aR,6R)-8-Chloro-10,11-difluoro-6-(methoxymethyl)-2,3,4,4a,5,6-hexahydro-1H-pyrazino[2,1-d][1,5]benzoxazepine hydrochloride

(compound of formula Ia.3 wherein $R^1$ is methyl, $R^4$ is Cl, $R^5$ is H, $R^6$ is F and $R^7$ is F)

$^1H$ NMR (500 MHz, DMSO-$d_6$): δ 1.88 (ddd, *J* = 15.2, 8.2, 3.9 Hz, 1H), 2.29 - 2.39 (m, 1H), 3.05 (dd, *J* = 12.5, 7.0 Hz, 1H), 3.10 - 3.26 (m, 3H), 3.34 (s, 3H), 3.40 (t, *J* = 5.0 Hz, 2H), 3.47 (dd, *J* = 10.8, 4.2 Hz, 1H), 3.55 (dd, *J* = 10.8, 5.8 Hz, 1H), 3.87 (ddt, *J* = 10.2, 7.1, 3.4 Hz, 1H), 4.33 (dq, *J* = 9.7, 5.1 Hz, 1H), 7.33 (dd, *J* = 10.1, 7.9 Hz, 1H), 9.31 (s, 2H). $^{19}F$ NMR (471 MHz, DMSO-$d_6$): δ -146.76 (d, *J* = 21.9 Hz, IF), -142.54 (s, IF). LC-MS: m/z = 319.1/321.1 [M+H].

Example 38

(4aR,6S)-8-Chloro-9,10-difluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is Cl, $R^5$ is F, $R^6$ is F and $R^7$ is H)
$^1H$ NMR (500 MHz, Methanol-$d_4$): δ 1.87 (ddd, *J* = 15.5, 4.2, 2.1 Hz, 1H), 1.97 (ddd, *J* = 15.3, 11.3, 9.8 Hz, 1H), 2.81 (dd, *J* = 12.4, 10.3 Hz, 1H), 3.16 - 3.25 (m, 3H), 3.32 - 3.38 (m, 4H), 3.52 (dd, *J* = 10.2, 4.4 Hz, 1H), 3.56 (dd, *J* = 10.2, 5.2 Hz, 1H), 3.63 (dt, *J* = 14.0, 3.1 Hz, 1H), 3.70 (tt, *J* = 10.0, 2.6 Hz, 1H), 4.44 - 4.52 (m, 1H), 6.99 (dd, *J* = 12.3, 8.2 Hz, 1H).
$^{19}F$ NMR (471 MHz, Methanol-$d_4$): δ -145.67 (dd, *J* = 22.6, 8.3 Hz, IF), -142.58 (dd, *J* = 22.6, 12.5 Hz, IF).
LC-MS: m/z = 319.2/321.2 [M+H].

Example 39

[(4aR,6S)-8-Fluoro-9-methyl-2,3,4,4a,5,6-hexahydro-1H-pyrazino[2,1-d][1,5]benzoxazepin-6-yl]methanol

(compound of formula Ia.2 wherein $R^1$ is H, $R^4$ is F, $R^5$ is $CH_3$, $R^6$ is H and $R^7$ is H)
$^1H$ NMR (600 MHz, Methanol-$d_4$): δ 1.70 (ddd, *J* = 15.1, 11.1, 9.6 Hz, 1H), 1.77 (ddd, *J* = 15.1, 4.3, 2.2 Hz, 1H), 2.16 (d, *J* = 1.5 Hz, 3H), 2.51 (dd, *J* = 12.3, 10.0 Hz, 1H), 2.87 (dd, *J* = 12.4, 2.9 Hz, 1H), 2.90 - 2.94 (m, 2H), 3.12 (ddd, *J* = 13.0, 8.6, 5.5 Hz, 1H), 3.43 - 3.49 (m, 3H), 3.64 - 3.69 (m, 1H), 4.24 (ddt, *J* = 11.1, 6.6, 4.7 Hz, 1H), 6.68 (dd, *J* = 8.3, 1.6 Hz, 1H), 6.80 (td, *J* = 8.3, 0.9 Hz, 1H).
$^{19}F$ NMR (470 MHz, DMSO-$d_6$): δ -135.21 (d, *J* = 8.6 Hz).
LC-MS: m/z = 267.2 [M+H].

Example 40

(4aR,6S)-8-Ethynyl-10,11-difluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is -C≡CH, $R^5$ is H, $R^6$ is F and $R^7$ is F)
$^1H$ NMR (500 MHz, DMSO-$d_6$): δ 1.88 (dt, *J* = 6.3, 3.6 Hz, 2H), 2.81 (d, *J* = 10.7 Hz, 1H), 3.05 (d, *J* = 10.1 Hz, 1H), 3.22 (s, 3H), 3.24 - 3.32 (m, 3H), 3.38 - 3.48 (m, 2H), 3.52 (dd, *J* = 10.3, 4.8 Hz, 1H), 3.78 - 3.88 (m, 1H), 4.28 (s, 1H), 4.41 - 4.53 (m, 1H), 7.16 (dd, *J* = 10.4, 8.6 Hz, 1H), 9.11 - 9.58 (m, 2H).
$^{19}F$ NMR (471 MHz, DMSO-$d_6$): δ -142.98 (s, IF), -141.81 (dd, *J* = 22.6, 10.5 Hz, IF). LC-MS: m/z = 309.2 [M+H].

Example 41

(4aR,6S)-10-Fluoro-6-(methoxymethyl)-8-methyl-2,3,4,4a,5,6-hexahydro-1H-pyrazino[2,1-d][1,5]benzoxazepine hydrochloride

(compound of formula Ia.2 wherein $R^1$ is methyl, $R^4$ is $CH_3$, $R^5$ is H, $R^6$ is F and $R^7$ is H)
$^1H$ NMR (600 MHz, Methanol-$d_4$): δ 1.80 - 1.90 (m, 2H), 2.21 (s, 3H), 2.91 (dd, *J* = 12.3, 10.1 Hz, 1H), 3.24 - 3.30 (m, 2H), 3.31 - 3.39 (m, 6H), 3.46 (dd, *J* = 10.4, 6.8 Hz, 1H), 3.65 - 3.74 (m, 2H), 4.33 - 4.40 (m, 1H), 6.58 (dd, *J* = 8.6, 3.0 Hz, 1H), 6.65 (dd, *J* = 10.2, 3.1 Hz, 1H).
$^{19}F$ NMR (470 MHz, DMSO-$d_6$): δ -118.94 (t, *J* = 9.8 Hz).
LC-MS: m/z = 281.2 [M+H].

Example 42

(4aR,6S)-8-(Difluoromethoxy)-6-(methoxymethyl)-2,3,4,4a,5,6-hexahydro-1H-pyrazino[2,1-d][1,5]benzoxazepine

(compound of formula Ia.2 wherein $R^1$ is methyl, $R^4$ is $OCHF_2$, $R^5$ is H, $R^6$ is H and $R^7$ is H)
$^1H$ NMR (600 MHz, Methanol-$d_4$): δ 1.83 - 1.90 (m, 2H), 2.84 (dd, *J* = 12.4, 10.4 Hz, 1H), 3.20 - 3.29 (m, 3H), 3.34 (s,

3H), 3.36 - 3.40 (m, 1H), 3.41 - 3.45 (m, 1H), 3.49 (dd, *J* = 10.2, 6.1 Hz, 1H), 3.74 (ddt, *J* = 20.4, 14.1, 3.4 Hz, 2H), 4.43 - 4.50 (m, 1H), 6.79 (dd, *J* = 78.3, 73.9 Hz, 1H), 6.78 - 6.81 (m, 1H), 6.94 (dd, *J* = 8.3, 1.5 Hz, 1H), 7.02 (t, *J* = 8.1 Hz, 1H).
$^{19}$F NMR (471 MHz, Methanol-$d_4$): δ -84.12 (dd, *J* = 167.4, 74.2 Hz, IF), -82.86 (dd, *J* = 167.4, 78.4 Hz, IF).
LC-MS: m/z = 315.2 [M+H].

Example 43

(4aR,6S)-10-(Difluoromethoxy)-8-fluoro-6-(methoxymethyl)-2,3,4,4a,5,6-hexahydro-1H-pyrazino[2,1-d][1,5]benzoxazepine

(compound of formula Ia.2 wherein $R^1$ is methyl, $R^4$ is F, $R^5$ is H, $R^6$ is $OCHF_2$ and $R^7$ is H)
$^1$H NMR (500 MHz, Methanol-$d_4$): δ 1.85 - 1.92 (m, 2H), 2.84 (dd, *J* = 12.4, 11.1 Hz, 1H), 3.21 - 3.30 (m, 3H), 3.34 (s, 3H), 3.37 - 3.46 (m, 2H), 3.48 (dd, *J* = 10.6, 6.2 Hz, 1H), 3.76 (dt, *J* = 14.3, 2.7 Hz, 1H), 3.86 (tq, *J* = 8.6, 2.8 Hz, 1H), 4.42 - 4.52 (m, 1H), 6.59 (dd, *J* = 10.3, 2.8 Hz, 1H), 6.62 - 6.94 (m, 2H).
$^{19}$F NMR (471 MHz, Methanol-$d_4$): δ -83.33 - -84.43 (m, 2F), -129.21 (d, *J* = 10.1 Hz, IF).
LC-MS: m/z = 333.2 [M+H].

Example 44

(4aR,6S)-8-Chloro-10-methoxy-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is Cl, $R^5$ is H, $R^6$ is $OCH_3$ and $R^7$ is H)
$^1$H NMR (600 MHz, Methanol-$d_4$): δ 6.61 (d, *J* = 3.0 Hz, 1H), 6.54 (d, *J* = 2.9 Hz, 1H), 4.47 - 4.39 (m, 1H), 3.91 - 3.86 (m, 2H), 3.80 (ddt, *J* = 10.7, 7.5, 3.0 Hz, 1H), 3.76 - 3.72 (m, 4H), 3.57 (dd, *J* = 10.1, 5.8 Hz, 1H), 3.46 - 3.38 (m, 2H), 3.34 (s, 3H), 3.23 - 3.19 (m, 2H), 2.91 - 2.85 (m, 1H), 1.91 - 1.85 (m, 2H).
LC-MS: m/z = 313.3/315.3 [M+H].

Example 45

(4aR,6S)-8-Chloro-6-(methoxymethyl)-10-(trifluoromethyl)-2,3,4,4a,5,6-hexahydro-1H-pyrazino[2,1-d][1,5]benzoxazepine

(compound of formula Ia.2 wherein $R^1$ is methyl, $R^4$ is Cl, $R^5$ is H, $R^6$ is $CF_3$ and $R^7$ is H)
$^1$H NMR (600 MHz, DMSO-$d_6$): δ 8.69 - 7.71 (m, 1H), 7.34 (d, *J* = 2.1 Hz, 1H), 7.29 (d, *J* = 2.2 Hz, 1H), 4.61 (dq, *J* = 11.5, 4.5 Hz, 1H), 3.76 - 3.62 (m, 2H), 3.51 (dd, *J* = 10.2, 5.0 Hz, 1H), 3.45 (dd, *J* = 10.3, 4.4 Hz, 1H), 3.41 - 3.30 (m, 1H), 3.25 (s, 3H), 3.11 (qt, *J* = 12.5, 2.2 Hz, 3H), 2.63 (t, *J* = 11.8 Hz, 1H), 1.90 (ddd, *J* = 15.6, 3.9, 2.2 Hz, 1H), 1.77 (dt, *J* = 15.6, 10.8 Hz, 1H, NH).
$^{19}$F NMR (471 MHz, DMSO-$d_6$): δ -60.55 (s, $CF_3$).
LC-MS: m/z = 351.2/353.2 [M+H].

Example 46

[(4aR,6S)-8-Chloro-10-(trifluoromethyl)-2,3,4,4a,5,6-hexahydro-1H-pyrazino[2,1-d][1,5]benzoxazepin-6-yl]methanol

(compound of formula Ia.2 wherein $R^1$ is H, $R^4$ is Cl, $R^5$ is H, $R^6$ is $CF_3$ and $R^7$ is H)
$^1$H NMR (500 MHz, DMSO-$d_6$): δ 7.27 (d, *J* = 2.1 Hz, 1H), 7.19 (d, *J* = 2.4 Hz, 1H), 4.80 (s, 1H), 4.42 - 4.32 (m, 1H), 3.73 - 3.64 (m, 1H), 3.54 - 3.38 (m, 3H), 3.16 (dt, *J* = 13.8, 8.1 Hz, 1H), 2.94 - 2.81 (m, 2H), 2.41 (t, *J* = 11.1 Hz, 1H), 1.88 (dt, *J* = 15.5, 3.6 Hz, 1H), 1.66 (dt, *J* = 15.6, 10.6 Hz, 1H).
$^{19}$F NMR (471 MHz, DMSO-$d_6$): δ -60.61 (s, $CF_3$).
LC-MS: m/z = 337.2/339.2 [M+H].

Example 47

(4aR,6S)-9,10,11-Trifluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is H, $R^5$ is F, $R^6$ is F and $R^7$ is F)

$^1H$ NMR (500 MHz, Methanol-$d_4$): δ 1.76 (ddd, *J* = 14.9, 4.8, 1.5 Hz, 1H), 1.85 (s, 1H), 2.84 (dd, *J* = 12.3, 9.4 Hz, 1H), 3.16 (tdd, *J* = 16.9, 9.8, 3.9 Hz, 4H), 3.32 - 3.36 (m, 1H), 3.38 (s, 3H), 3.44 (ddd, *J* = 11.8, 6.9, 4.9 Hz, 2H), 3.72 (s, 1H), 4.38 (s, 1H), 6.72 (ddd, *J* = 11.3, 7.7, 2.2 Hz, 1H).
$^{19}F$ NMR (471 MHz, Methanol-$d_4$): δ = -167.26 - -166.92 (m, 2F), -144.76 (s, IF). LC-MS: m/z = 303.2 [M+H].

Example 48

(4aR,6S)-8-Cyclopropyl-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is cyclopropyl, $R^5$ is H, $R^6$ is H and $R^7$ is H)
$^1H$ NMR (600 MHz, Chloroform-d): δ 0.64 (dtt, *J* = 10.3, 5.1, 3.1 Hz, 2H), 1.00 (ddd, *J* = 8.7, 2.9, 1.1 Hz, 2H), 2.25 (d, *J* = 15.4 Hz, 1H), 2.43 (tt, *J* = 8.5, 5.3 Hz, 1H), 2.99 (s, 1H), 3.38 (s, 3H), 3.53 (dd, *J* = 10.4, 4.4 Hz, 1H), 3.65 - 3.88 (m, 4H), 4.11 - 4.44 (m, 5H), 6.73 (d, *J* = 7.6 Hz, 1H), 7.07 (t, *J* = 8.0 Hz, 1H), 7.79 (s, 1H), 10.05 (s, 1H), 10.24 (s, 1H).
LC-MS: m/z = 289.3 [M+H].

Example 49

(4aR,6S)-8-Cyclopropyl-10-fluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is cyclopropyl, $R^5$ is H, $R^6$ is F and $R^7$ is H)
$^1H$ NMR (500 MHz, Chloroform-d): δ 0.50 - 0.69 (m, 2H), 0.93 - 1.06 (m, 2H), 1.94 (dt, *J* = 15.1, 3.6 Hz, 1H), 2.14 (q, *J* = 12.1, 10.5 Hz, 1H), 2.39 (dddd, *J* = 13.8, 8.4, 5.3, 1.4 Hz, 1H), 3.07 (d, *J* = 10.4 Hz, 1H), 3.37 (s, 5H), 3.55 - 3.70 (m, 4H), 3.75 (dt, *J* = 14.6, 3.9 Hz, 1H), 4.02 (pd, *J* = 6.1, 4.1 Hz, 1H), 4.39 (ddt, *J* = 8.7, 6.6, 4.3 Hz, 1H), 6.15 (dd, *J* = 9.4, 2.9 Hz, 1H), 6.65 (dd, *J* = 9.8, 3.0 Hz, 1H), 10.05 (s, 2H).
$^{19}F$ NMR (471 MHz, Chloroform-d): δ -116.52 (s).
LC-MS: m/z = 307.2 [M+H].

Example 50

(4aR,6S)-8-Fluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is F, $R^5$ is H, $R^6$ is H and $R^7$ is H)
$^1H$ NMR (500 MHz, DMSO-$d_6$): δ 1.67 (dt, *J* = 15.4, 10.6 Hz, 1H), 1.90 (ddd, *J* = 15.3, 4.3, 2.0 Hz, 1H), 2.69 (tt, *J* = 12.1, 6.8 Hz, 1H), 3.14 (s, 1H), 3.19 - 3.24 (m, 2H), 3.26 (s, 3H), 3.28 - 3.35 (m, 2H), 3.43 (dd, *J* = 10.4, 5.8 Hz, 1H), 3.66 (dt, *J* = 14.4, 2.8 Hz, 1H), 3.74 - 3.81 (m, 1H), 4.46 (dq, *J* = 10.1, 4.7 Hz, 1H), 6.78 (ddd, *J* = 9.9, 8.2, 1.4 Hz, 1H), 6.86 (dt, *J* = 8.2, 1.5 Hz, 1H), 6.98 (td, *J* = 8.2, 6.0 Hz, 1H), 9.05 (s, 2H).
$^{19}F$ NMR (471 MHz, DMSO-$d_6$): δ = -131.32 (t, *J* = 7.2 Hz).
LC-MS: m/z = 267.2 [M+H].

Example 51

(4aR,6S)-9-Fluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is H, $R^5$ is F, $R^6$ is H and $R^7$ is H)
$^1H$ NMR (500 MHz, DMSO-$d_6$): δ 1.63 (dt, *J* = 15.3, 10.6 Hz, 1H), 1.87 (ddd, *J* = 15.2, 3.9, 2.4 Hz, 1H), 2.65 - 2.77 (m, 1H), 3.10 - 3.25 (m, 3H), 3.26 - 3.36 (m, 5H), 3.43 (dd, *J* = 10.5, 6.6 Hz, 1H), 3.50 - 3.59 (m, 1H), 3.68 (dt, *J* = 10.1, 2.5 Hz, 1H), 4.38 (ddd, *J* = 10.7, 6.7, 4.0 Hz, 1H), 6.66 (dd, *J* = 9.4, 3.0 Hz, 1H), 6.88 (td, *J* = 8.5, 3.1 Hz, 1H), 7.09 (dd, *J* = 9.0, 6.0 Hz, 1H), 9.10 (d, *J* = 23.6 Hz, 2H).
$^{19}F$ NMR (471 MHz, DMSO-$d_6$): δ -120.11 - -120.02 (m).
LC-MS: m/z = 267.2 [M+H].

Example 52

(4aR,6S)-8,10-Dichloro-6-(methoxymethyl)-2,3,4,4a,5,6-hexahydro-1H-pyrazino[2,1-d][1,5]benzoxazepine

(compound of formula Ia.2 wherein $R^1$ is methyl, $R^4$ is Cl, $R^5$ is H, $R^6$ is Cl and $R^7$ is H)

$^1H$ NMR (600 MHz, Methanol-$d_4$): δ 1.85 - 1.96 (m, 2H), 2.82 (dd, *J* = 12.4, 10.7 Hz, 1H), 3.19 - 3.29 (m, 3H), 3.34 (s, 3H), 3.40 (ddd, *J* = 14.5, 11.2, 3.6 Hz, 1H), 3.49 (dd, *J* = 10.2, 4.6 Hz, 1H), 3.57 (dd, *J* = 10.2, 5.3 Hz, 1H), 3.70 (dt, *J* = 14.3, 2.9 Hz, 1H), 3.76 (ddt, *J* = 11.3, 8.7, 2.8 Hz, 1H), 4.50 (dq, *J* = 10.2, 5.1 Hz, 1H), 7.01 (d, *J* = 2.5 Hz, 1H), 7.02 (d, *J* = 2.5 Hz, 1H).
LC-MS: m/z = 317.1/319.1 [M+H].

Example 53

(4aR,6S)-8-Chloro-10,11-difluoro-9-methoxy-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is Cl, $R^5$ is $OCH_3$, $R^6$ is F and $R^7$ is F)
$^1H$ NMR (500 MHz, DMSO-$d_6$): δ 1.79 - 2.05 (m, 2H), 2.85 (t, *J* = 10.2 Hz, 2H), 3.01 - 3.13 (m, 1H), 3.18 - 3.29 (m, 4H), 3.43 (tdd, *J* = 20.9, 14.2, 9.3 Hz, 4H), 3.77 (t, *J* = 10.0 Hz, 1H), 3.86 (s, 3H), 4.48 (s, 1H), 9.28 (s, 2H).
$^{19}F$ NMR (471 MHz, DMSO-$d_6$): δ -157.31 (d, *J* = 22.0 Hz, IF), -146.28 (s, IF). LC-MS: m/z = 349.1/351.1 [M+H].

Example 54

(4aR,6S)-11-Fluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is H, $R^5$ is H, $R^6$ is H and $R^7$ is F)
$^1H$ NMR (500 MHz, DMSO-$d_6$): δ 1.53 - 1.71 (m, 1H), 1.83 (dd, *J* = 14.9, 5.0 Hz, 1H), 2.83 (s, 1H), 3.03 - 3.25 (m, 3H), 3.25 - 3.30 (m, 6H), 3.40 (d, *J* = 13.7 Hz, 1H), 3.81 (t, *J* = 10.1 Hz, 1H), 4.33 - 4.46 (m, 1H), 6.72 (d, *J* = 7.8 Hz, 1H), 6.86 - 7.10 (m, 2H), 8.93 (s, 2H).
$^{19}F$ NMR (471 MHz, DMSO-$d_6$): δ -121.74 (s).
LC-MS: m/z = 267.2 [M+H].

Example 55

(4aR,6S)-8-Chloro-9,10,11-trifluoro-6-(methoxymethyl)-2,3,4,4a,5,6-hexahydro-1H-pyrazino[2,1-d][1,5]benzoxazepine hydrochloride

(compound of formula Ia.2 wherein $R^1$ is methyl, $R^4$ is Cl, $R^5$ is F, $R^6$ is F and $R^7$ is F)
$^1H$ NMR (500 MHz, Methanol-$d_4$): δ 1.84 (ddd, *J* = 15.5, 4.3, 1.3 Hz, 1H), 2.13 (s, 1H), 2.96 (t, *J* = 11.0 Hz, 1H), 3.20 - 3.30 (m, 3H), 3.31 (s, 3H), 3.32 - 3.37 (m, 1H), 3.46 - 3.61 (m, 3H), 3.80 (t, *J* = 9.8 Hz, 1H), 4.51 (s, 1H).
$^{19}F$ NMR (471 MHz, Methanol-$d_4$): δ -165.60 (t, *J* = 21.4 Hz, IF), -146.96 (br. s, 1F), - 143.84 (br. s, IF).
LC-MS: m/z = 337.2/339.2 [M+H].

Example 56

(4aR,6S)-10,11-Difluoro-6-(methoxymethyl)-8-(trifluoromethyl)-2,3,4,4a,5,6-hexahydro-1H-benzo[b]pyrazino[1,2-d][1,4]oxazepine hydrochloride

(compound of formula Ia.2 wherein $R^1$ is methyl, $R^4$ is $CF_3$, $R^5$ is H, $R^6$ is F and $R^7$ is F)
$^1H$ NMR (600 MHz, DMSO-$d_6$): δ 1.86 - 1.95 (m, 1H), 2.34 (s, 1H), 2.50 - 2.53 (m, 1H), 3.01 - 3.25 (m, 5H), 3.27 (s, 3H), 3.40 - 3.52 (m, 2H), 3.55 (dd, *J* = 10.5, 4.4 Hz, 1H), 4.33 (s, 1H), 7.57 (t, *J* = 9.2 Hz, 1H), 8.72 - 9.13 (m, 2H).
$^{19}F$ NMR (471 MHz, DMSO-$d_6$): δ -140.61 (dd, *J* = 23.8, 10.2 Hz, IF), -137.20 (br. s, IF), -59.16 (s, 3 F).
LC-MS: m/z = 353.0 [M+H].

II. Biological tests

Functional activity

1. Human 5-$HT_{2C}$ Functional Assay

The functional activity of compounds of formula I was assayed by incubation with U2OS_$HTR_{2C}$_β-Arrestin cells (DiscoverX, 93-0289C3) to induce beta-arrestin2 recruitment to the 5-$HT_{2C}$ receptor. The agonist-induced recruitment and proximity of the receptor and beta-arrestin2 leads to complementation and formation of active β-galactosidase.

The enzyme complementation results in enzyme activity, which is measured following the termination of the agonist incubation using DiscoveRx's detection reagent, which contains a chemiluminescent substrate which produces a high intensity signal. Cells were plated and a medium-change to a 1% serum containing medium was performed 24h later. The next day, test compounds were added and incubated for 1.5 h before addition of detection reagent.

The response produced was measured and compared with the response produced by 10 [mu]M 5-HT or the maximal effect induced by 5-HT (defined as 100%) to which it was expressed as a percentage response (relative efficacy). Dose response curves were constructed using Graphpad Prism (Graph Software Inc.) or using in house adapted software using a 4 parameter dose response model with variable slope (fit = (Bottom + (Top-Bottom)/(1+10^((LogEC50-x)*HillSlope))res = (y-fit)). Results are compiled in the table below.

2. Human 5-$HT_{2A}$ Functional Assay

Functional activity on the 5-$HT_{2A}$ receptor was determined by testing the effect of the compounds I on calcium mobilisation in CHO-K1 cells, stably transfected with human 5-$HT_{2A}$ receptor. Cells were seeded into sterile black 384-well plates with clear bottom at 25,000 cells/well in a volume of 25 $\mu$l and grown for 5-6 hours at 37°C, in 5% $CO_2$ in tissue culture medium ("Ultra CHO" by LONZA), containing 1% dialysed FCS and 50 $\mu$g/ml gentamicin (Invitrogen). After this incubation, medium was replaced by a serum free version of the same tissue culture medium followed by incubation overnight at 37°C and in 5% $CO_2$. Cells were then loaded with a fluorescent calcium-sensitive dye in the presence of 0.07% probenecid for an hour at 37°C, according to the manufacturer's protocol (Ca5-Assay Kit, Molecular Devices), followed by an additional 60 min incubation at room temperature. Serial compound dilutions (final concentrations of $10^{-10}$ to $10^{-5}$M, prepared in HBSS + 50 mM HEPES) were first added to the cells alone ("first addition" to assess agonism on the 5-$HT_{2A}$ receptor), then after 8 min, serotonin was added to the same wells at a final concentration of $3x10^{-8}$ M ("second addition" to see potential antagonistic effect) and the maximal calcium response was determined using a FLIPR® Tetra instrument (Molecular Devices) in each of the two steps. The relative efficacy of the compounds was calculated as a percentage of the maximal effect induced by serotonin alone (defined as 100%). To determine $EC_{50}$ / $IC_{50}$ values, concentration-response curves were fitted using a four-parameter logistic equation (IDBS Biobook™). $K_b$ values were calculated from $IC_{50}$ values, according to Cheng & Prusoff.

3. Human 5-$HT_{2B}$ Functional Assay

Functional activity on the 5-$HT_{2B}$ receptor was determined by testing the effect of the compounds I on calcium mobilisation in CHO-FlpIn cells, stably transfected with human 5-$HT_{2B}$ receptor. Cells were seeded into sterile black 384-well plates with clear bottom at 30,000 cells/well in a volume of 25 $\mu$l and grown overnight at 37°C, in 5% $CO_2$ in tissue culture medium ("CHO-S-SFM II" by Invitrogen), containing 1% dialysed FCS and 50 $\mu$g/ml gentamicin (Invitrogen). On the next morning, medium was replaced by a serum free version of the same tissue culture medium for a further incubation for 4 hours at 37°C and in 5% $CO_2$. Cells were then loaded with a fluorescent calcium-sensitive dye in the presence of 0.07% probenecid for an hour at 37°C, according to the manufacturer's protocol (Ca5-Assay Kit, Molecular Devices), followed by an additional 60 min incubation at room temperature. Serial compound dilutions (final concentrations of $10^{-10}$ to $10^{-5}$M, prepared in HBSS + 50 mM HEPES) were first added to the cells alone ("first addition" to assess agonism on the 5-$HT_{2B}$ receptor), then after 8 min, serotonin was added to the same wells at a final concentration of $10^{-8}$ M ("second addition" to see potential antagonistic effect) and the maximal calcium response was determined using a FLIPR® Tetra instrument (Molecular Devices) in each of the two steps. The relative efficacy of the compounds was calculated as a percentage of the maximal effect induced by serotonin alone (defined as 100%). To determine $EC_{50}$/ $IC_{50}$ values, concentration-response curves were fitted using a four-parameter logistic equation (IDBS Biobook™). Kb values were calculated from $IC_{50}$ values, according to Cheng & Prusoff.

4. Metabolic Stability

Samples of the tested compounds (0.5 $\mu$M) were preincubated together with human liver microsomes (0.25 mg of microsomal protein/mL) in 0.05 M potassium phosphate buffer of pH 7.4 in microtiter plates at 37 °C for 5 minutes. The reaction was started by adding NADPH (1.0 mM). After 0, 5, 10, 15, 20 and 30 minutes, an aliquot was removed, the reaction was cooled and stopped by adding twice the amount of quench solution consisting of acetonitrile/methanol 1:1, and containing 0.2 $\mu$M carbutamide as internal standard. The samples were frozen until analyzed. The remaining concentration of undegraded test substance was determined by liquid chromatography-tandem mass spectrometry (LC-MS/MS). The half-life ($t_{1/2}$) was determined from the gradient of the ratio of the signal of (test substance/internal standard)/unit time plot, allowing the calculation of the half-life of the test substance, assuming first order kinetics, from the decrease in the concentration of the compound with time. The microsomal clearance (mClint) was calculated as follows: mClint = ((ln(2)/t 1/2)/Microsomal Protein Concentration (mg/ml))*1000 , leading to the unit of uL/min/mg. The scaled

clearance (mClin_scaled) was calculated as mCLint_scaled = m CLint * (Microsomal Yield (mg/kg BW))/1000000*60, leading to the units L/h/kg. The Microsomal Yield is defined by the specifics of the used microsomes. Calculations were modified from references: Di, The Society for Biomolecular Screening, 2003, 453-462; Obach, DMD, 1999 vol 27. N 11, 1350-1359.

Unbound Fraction in Microsomes (fu mic)

A suspension of 0.25 mg/ml microsomal protein spiked with 0.5 μM of test compound was pipetted on one side of a HTDialysis device (HTDialysis LLC,37 Ledgewood Drive, Gales Fery CT 06335) separated by a membrane with a MWcut off 12-14 K. 50 mM K-Phosphate buffer (pH 7.4) was added on the other side of the well. After incubation at 37°C for 4 h while shaking at 150 rpm, aliquots of both sides were taken, quenched with MeOH/ACN 1:1 and 0.2μM of internal standard and frozen until analysis by LCMSMS

Calculation of unbound intrinsic clearance

$$Cl\ int\ unbound = Cl\ int\ /\ fu\ mic$$

| # | EC50 5-$HT_{2C}$[1] | % efficacy | Selectivity over 5-$HT_{2A}$[2] | Selectivity over 5-$HT_{2B}$[3] | Cl int,mic unb. (h)[4] [l/h/kg] |
|---|---|---|---|---|---|
| 1 | +++ | +++ | +++ | +++ | +++ |
| 2 | ++ | ++ | +++ | +++ | +++ |
| 3 | + | ++ | + | + | +++ |
| 5 | +++ | +++ | +++ | + | +++ |
| 6 | +++ | +++ | +++ | ++ | +++ |
| 7 | +++ | +++ | +++ | +++ | +++ |
| 8 | ++ | +++ | +++ | +++ | +++ |
| 9 | +++ | +++ | +++ | +++ | +++ |
| 10 | ++ | | ++ | ++ | ++ |
| 11 | ++ | +++ | +++ | ++ | +++ |
| 12 | ++ | +++ | +++ | + | ++ |
| 13 | ++ | +++ | +++ | ++ | +++ |
| 14 | ++ | +++ | +++ | | +++ |
| 15 | +++ | +++ | +++ | +++ | +++ |
| 16 | +++ | +++ | +++ | +++ | +++ |
| 17 | +++ | +++ | | | +++ |
| 18 | ++ | +++ | +++ | +++ | +++ |
| 19 | +++ | +++ | +++ | ++ | +++ |
| 20 | ++ | +++ | +++ | | +++ |
| 21 | ++ | + | ++ | ++ | +++ |
| 22 | +++ | +++ | +++ | ++ | +++ |
| 23 | +++ | +++ | + | | +++ |
| 24 | ++ | ++ | +++ | ++ | ++ |
| 25 | ++ | +++ | +++ | | +++ |
| 26 | +++ | +++ | +++ | +++ | +++ |
| 27 | ++ | +++ | ++ | | +++ |
| 28 | ++ | +++ | ++ | + | +++ |
| 29 | ++ | +++ | +++ | +++ | ++ |

(continued)

| # | EC50 5-$HT_{2C}$[1] | % efficacy | Selectivity over 5-$HT_{2A}$[2] | Selectivity over 5-$HT_{2B}$[3] | Cl int,mic unb. (h)[4] [l/h/kg] |
|---|---|---|---|---|---|
| 30 | ++ | ++ | ++ | ++ | +++ |
| 31 | ++ | + | ++ | ++ | +++ |
| 32 | + | +++ | ++ | ++ | +++ |
| 33 | + | ++ | + | + | +++ |
| 34 | +++ | +++ | +++ | + | +++ |
| 35 | ++ | +++ | ++ | + | +++ |
| 36 | + | ++ | + | + | ++ |
| 37 | + | + | + | + | +++ |
| 38 | +++ | +++ | ++ | ++ | +++ |
| 39 | ++ | ++ | ++ | ++ | +++ |
| 40 | +++ | +++ | +++ | + | +++ |
| 41 | +++ | +++ | +++ | | +++ |
| 42 | +++ | +++ | + | | +++ |
| 44 | ++ | +++ | | ++ | +++ |
| 45 | ++ | +++ | +++ | +++ | ++ |
| 47 | ++ | +++ | +++ | +++ | +++ |
| 48 | +++ | +++ | + | | +++ |
| 49 | +++ | +++ | ++ | | +++ |
| 50 | ++ | +++ | ++ | | +++ |
| 51 | + | ++ | + | ++ | +++ |
| 52 | ++ | +++ | +++ | ++ | +++ |
| 53 | ++ | ++ | +++ | +++ | ++ |
| 54 | + | + | + | + | +++ |
| 55 | +++ | +++ | +++ | +++ | |
| 56 | ++ | +++ | +++ | +++ | +++ |

[1] Potency (EC50 5-$HT_{2C}$) in functional assay
[2] Selectivity (based on agonism): EC50 5-$HT_{2A}$ / EC50 5-$HT_{2C}$
[3] Selectivity (based on agonism): EC50 5-$HT_{2B}$ / EC50 5-$HT_{2C}$
[4] unbound intrinsic clearance (human)

Potency (EC50):

| | |
|---|---|
| + | from 200 nM to < 1μM |
| ++ | from 20 nM to < 200 nM |
| +++ | < 20 nM |

% Efficacy:

| | |
|---|---|
| + | from 30 to 50% |
| ++ | from > 50 to 70% |
| +++ | > 70% |

| Selectivity over 5-$HT_{2A}$ or 5-$HT_{2B}$: | |
|---|---|
| + | from 10 to 30 |
| ++ | from >30 to 100 |
| +++ | > 100 |

Compounds with a lower selectivity over the 5-$HT_{2A}$ or 5-$HT_{2B}$ receptor show in several cases an only weak efficacy (e.g. Emax < 30%) in the described 5-$HT_{2A}$ or 5-$HT_{2B}$ functional assay and are thus advantageous.

| Unbound intrinsic clearance: | |
|---|---|
| + | from 50 to 500 1/h/kg |
| ++ | from 5 to < 50 l/h/kg |
| +++ | < 5 l/h/kg |

## Claims

1. A compound of formula (I)

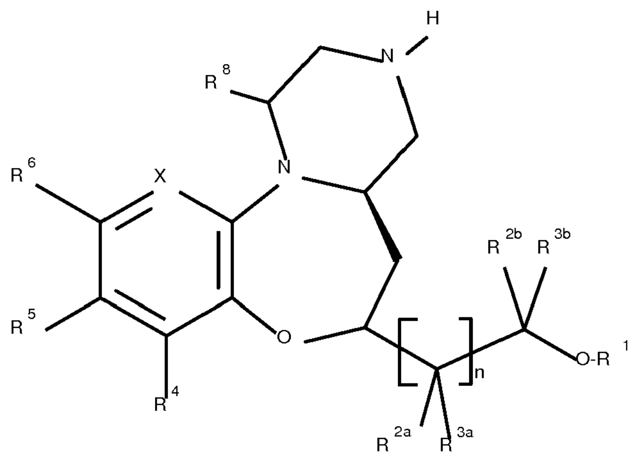

(I)

wherein

X is $CR^7$ or N;
$R^1$ is selected from the group consisting of hydrogen, methyl, deuterated methyl, and fluorinated methyl;
$R^{2a}$, $R^{2b}$, $R^{3a}$ and $R^{3b}$, independently of each other, are selected from the group consisting of hydrogen, $C_1$-$C_4$-alkyl, $C_1$-$C_4$-haloalkyl, $C_3$-$C_6$-cycloalkyl, and $C_3$-$C_6$-halocycloalkyl; or

$R^{2a}$ and $R^{3a}$, or $R^{2b}$ and $R^{3b}$, together with the carbon atom they are bound to, form a 3-membered saturated carbocyclic ring; or
$R^1$ and $R^{2a}$, if present, form together a group -$[CH_2]_s$-, where s is 1, 2, 3 or 4; or
$R^1$ and $R^{2b}$ form together a group -$[CH_2]_s$-, where s is 1, 2, 3 or 4;

$R^4$, $R^5$, $R^6$ and $R^7$, independently of each other, are selected from the group consisting of hydrogen, halogen, cyano, $C_1$-$C_4$-alkyl, fluorinated $C_1$-$C_4$-alkyl, $C_1$-$C_4$-hydroxyalkyl, $C_2$-$C_4$-alkenyl, fluorinated $C_2$-$C_4$-alkenyl, $C_2$-$C_4$-alkynyl, fluorinated $C_2$-$C_4$-alkynyl, $C_3$-$C_6$-cycloalkyl, fluorinated $C_3$-$C_6$-cycloalkyl, $C_1$-$C_4$-alkoxy, fluorinated $C_1$-$C_4$-alkoxy, $C_1$-$C_4$-alkylthio, and fluorinated $C_1$-$C_4$-alkylthio;
$R^8$ is selected from the group consisting of hydrogen, $C_1$-$C_4$-alkyl, fluorinated $C_1$-$C_4$-alkyl, and $C_1$-$C_4$-hydroxyalkyl;
n is 0 or 1;

or an N-oxide, a stereoisomer or pharmaceutically acceptable salt thereof, or a compound of the general formula

(I), wherein at least one of the atoms has been replaced by its stable, non-radioactive isotope.

**2.** The compound as claimed in claim 1, where n is 0 and $R^1$ is methyl.

**3.** The compound as claimed in any of the preceding claims, where n is 0 and $R^{2b}$ and $R^{3b}$, independently of each other, are selected from the group consisting of hydrogen, methyl, $CHF_2$, $CF_3$, and cyclopropyl.

**4.** The compound as claimed in claim 3, where n is 0 and $R^{2b}$ and $R^{3b}$ are hydrogen.

**5.** The compound as claimed in claim 1, where n is 0, $R^1$ and $R^{2b}$ form together a group $-[CH_2]_s-$, where s is 2, and $R^{3b}$ is as defined in any of claims 1, 2 or 3.

**6.** The compound as claimed in claim 5, where n is 0, $R^1$ and $R^{2b}$ form together a group $-[CH_2]_s-$, where s is 2, and $R^{3b}$ is hydrogen.

**7.** The compound as claimed in any of the preceding claims, where

$R^4$ is selected from the group consisting of hydrogen, fluorine, chlorine, cyano, $C_1$-$C_2$-alkyl, fluorinated $C_1$-$C_2$-alkyl, $C_2$-$C_3$-alkynyl, cyclopropyl, $C_1$-$C_2$-alkoxy, fluorinated $C_1$-$C_2$-alkoxy, $C_1$-$C_2$-alkylthio, and fluorinated $C_1$-$C_2$-alkylthio;
$R^5$ is selected from the group consisting of hydrogen, halogen, $C_1$-$C_2$-alkyl, and $C_1$-$C_2$-alkoxy;
$R^6$ is selected from the group consisting of hydrogen, fluorine, chlorine, $C_1$-$C_2$-alkyl, fluorinated $C_1$-$C_2$-alkyl, $C_2$-$C_3$-alkynyl, $C_1$-$C_2$-alkoxy, and fluorinated $C_1$-$C_2$-alkoxy; and
$R^7$ is selected from the group consisting of hydrogen, fluorine, chlorine, $C_1$-$C_2$-alkyl, fluorinated $C_1$-$C_2$-alkyl, and $C_1$-$C_2$-hydroxyalkyl.

**8.** The compound as claimed in claim 7, where

$R^4$ is selected from the group consisting of fluorine, chlorine, cyano, methyl, fluorinated methyl, $C_2$-$C_3$-alkynyl, cyclopropyl, methoxy, fluorinated methoxy, methylthio, and fluorinated methylthio;
$R^5$ is selected from the group consisting of hydrogen, halogen, and $C_1$-$C_2$-alkyl;
$R^6$ is selected from the group consisting of fluorine, chlorine, methyl, fluorinated methyl, methoxy, and fluorinated methoxy; and
$R^7$ is selected from the group consisting of hydrogen, fluorine, chlorine, $C_1$-$C_2$-alkyl, fluorinated $C_1$-$C_2$-alkyl, and $C_1$-$C_2$-hydroxyalkyl.

**9.** The compound as claimed in any of the preceding claims, where $R^4$ is selected from the group consisting of fluorine, chlorine, cyano, methyl, $CHF_2$, $CF_3$, and $C_3$-alkynyl.

**10.** The compound as claimed in any of the preceding claims, where $R^8$ is hydrogen.

**11.** The compound as claimed in any of the preceding claims, where X is $CR^7$.

**12.** The compound as claimed in any of the preceding claims, of formula (I.cis)

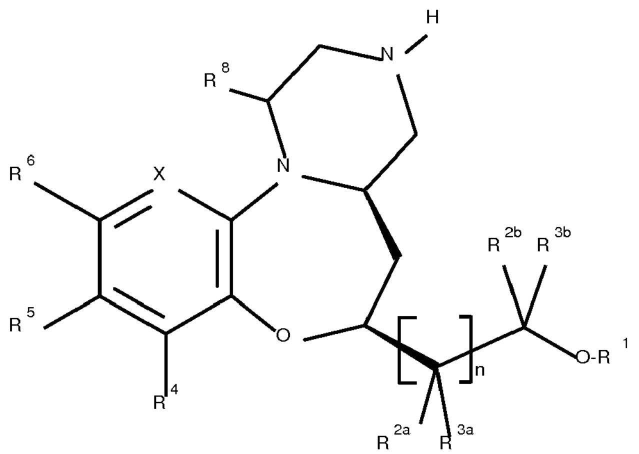

(I.cis)

where X, $R^1$, $R^{2a}$, $R^{2b}$, $R^{3a}$, $R^{3b}$, $R^4$, $R^5$, $R^6$, $R^8$ and n are as defined in any of the preceding claims.

**13.** A compound selected from the group consisting of:

(4aR,6S)-8-chloro-6-(methoxymethyl)-2,3,4,4a,5,6-hexahydro-1H-pyrazino[2,1-d][1,5]benzoxazepine;
(4aR,6R)-8-chloro-6-(methoxymethyl)-2,3,4,4a,5,6-hexahydro-1H-pyrazino[2,1-d][1,5]benzoxazepine;
(4aR,6S)-6-(methoxymethyl)-2,3,4,4a,5,6-hexahydro-1H-pyrazino[2,1-d][1,5]benzoxazepine;
(4aR,6R)-6-(methoxymethyl)-2,3,4,4a,5,6-hexahydro-1H-pyrazino[2,1-d][1,5]benzoxazepine;
(4aR,6S)-8-chloro-9-fluoro-6-(methoxymethyl)-2,3,4,4a,5,6-hexahydro-1H-pyrazino[2,1-d][1,5]benzoxazepine;
(4aR,6S)-8-(difluoromethyl)-6-(methoxymethyl)-2,3,4,4a,5,6-hexahydro-1H-pyrazino[2,1-d][1,5]benzoxazepine;
(4aR,6S)-8-chloro-10-fluoro-6-(methoxymethyl)-2,3,4,4a,5,6-hexahydro-1H-pyrazino[2,1-d][1,5]benzoxazepine;
(4aR,6S)-10-fluoro-6-(methoxymethyl)-2,3,4,4a,5,6-hexahydro-1H-pyrazino[2,1-d] [1,5]benzoxazepine;
(4aR,6S)-8-(difluoromethyl)-10-fluoro-6-(methoxymethyl)-2,3,4,4a,5,6-hexahydro-1H-pyrazino[2,1-d][1,5]benzoxazepine;
(4aR,6S)-10-chloro-6-(methoxymethyl)-2,3,4,4a,5,6-hexahydro-1H-pyrazino[2,1-d] [1,5]benzoxazepine;
(4aR,6S)-8,10-difluoro-6-(methoxymethyl)-2,3,4,4a,5,6-hexahydro-1H-pyrazino[2,1-d][1,5]benzoxazepine;
(4aR,6S)-6-(methoxymethyl)-2,3,4,4a,5,6-hexahydro-1H-pyrazino[2,1-d] [1,5]benzoxazepine-8-carbonitrile;
(4aR,6S)-10-fluoro-6-(methoxymethyl)-2,3,4,4a,5,6-hexahydro-1H-pyrazino[2,1-d] [1,5]benzoxazepine-8-carbonitrile;
[(4aR,6S)-8-chloro-2,3,4,4a,5,6-hexahydro-1H-pyrazino[2,1-d][1,5]benzoxazepin-6-yl]methanol;
(4aR,6S)-8-chloro-11-fluoro-6-(methoxymethyl)-2,3,4,4a,5,6-hexahydro-1H-pyrazino[2,1-d][1,5]benzoxazepine;
(4aR,6S)-8-chloro-10,11-difluoro-6-(methoxymethyl)-2,3,4,4a,5,6-hexahydro-1H-pyrazino[2,1-d][1,5]benzoxazepine;
[(4aR,6S)-8-chloro-10-fluoro-2,3,4,4a,5,6-hexahydro-1H-pyrazino[2,1-d][1,5]benzoxazepin-6-yl]methanol;
(4aR,6S)-8,10,11-trifluoro-6-(methoxymethyl)-2,3,4,4a,5,6-hexahydro-1H-pyrazino[2,1-d][1,5]benzoxazepine;
(4aR,6S)-8-chloro-10-fluoro-6-(trideuteriomethoxymethyl)-2,3,4,4a,5,6-hexahydro-1H-pyrazino[2,1-d][1,5]benzoxazepine;
[(4aR,6S)-8-chloro-10,11-difluoro-2,3,4,4a,5,6-hexahydro-1H-pyrazino[2,1-d][1,5]benzoxazepin-6-yl]methanol;
(4aR,6S)-10,11-difluoro-6-(methoxymethyl)-2,3,4,4a,5,6-hexahydro-1H-pyrazino[2,1-d][1,5]benzoxazepine;
(4aR,6S)-10-fluoro-6-(methoxymethyl)-8-(trifluoromethyl)-2,3,4,4a,5,6-hexahydro- 1H-pyrazino[2,1-d][1,5]benzoxazepine;
[(4aR,6S)-8-(difluoromethyl)-10-fluoro-2,3,4,4a,5,6-hexahydro-1H-pyrazino[2,1-d][1,5]benzoxazepin-6-yl]methanol;
(4aR,6S)-8-chloro-6-(difluoromethoxymethyl)-10-fluoro-2,3,4,4a,5,6-hexahydro-1H-pyrazino[2,1-d][1,5]benzoxazepine;
(4aR,6S)-10-chloro-8-fluoro-6-(methoxymethyl)-2,3,4,4a,5,6-hexahydro-1H-pyrazino[2,1-d][1,5]benzoxazepine;

(4aR,6S)-8-(difluoromethyl)-10,11-difluoro-6-(methoxymethyl)-2,3,4,4a,5,6-hexahydro-1H-pyrazino[2,1-d][1,5]benzoxazepine;
(6S,7aR)-4-(difluoromethyl)-6-(methoxymethyl)-7,7a,8,9,10,11-hexahydro-6H-pyrazino[1,2-d]pyrido[3,2-b][1,4]oxazepine 2,2,2-trifluoroacetic acid;
(4aR,6S)-10,11-difluoro-6-(methoxymethyl)-2,3,4,4a,5,6-hexahydro-1H-pyrazino[2,1-d][1,5]benzoxazepine-8-carbonitrile;
(4aR,6S)-8-(difluoromethyl)-11-fluoro-10-methoxy-6-(methoxymethyl)-2,3,4,4a,5,6-hexahydro-1H-pyrazino[2,1-d][1,5]benzoxazepine;
(4aR,6S)-8-fluoro-6-(methoxymethyl)-9-methyl-2,3,4,4a,5,6-hexahydro-1H-pyrazino[2,1-d][1,5]benzoxazepine;
(4aR,6S)-10-chloro-9-fluoro-6-(methoxymethyl)-2,3,4,4a,5,6-hexahydro-1H-pyrazino[2,1-d][1,5]benzoxazepine;
(4aR,6R)-8-(difluoromethyl)-10-fluoro-6-(methoxymethyl)-2,3,4,4a,5,6-hexahydro-1H-pyrazino[2,1-d][1,5]benzoxazepine;
(4aR,6S)-8-fluoro-6-(methoxymethyl)-10-methyl-2,3,4,4a,5,6-hexahydro-1H-pyrazino[2,1-d][1,5]benzoxazepine;
(4aR,6S)-8-ethynyl-10-fluoro-6-(methoxymethyl)-2,3,4,4a,5,6-hexahydro-1H-pyrazino[2,1-d][1,5]benzoxazepine;
(4aR,6R)-8-chloro-10-fluoro-6-(methoxymethyl)-2,3,4,4a,5,6-hexahydro-1H-pyrazino[2,1-d][1,5]benzoxazepine;
(4aR,6S)-10-ethynyl-8-fluoro-6-(methoxymethyl)-2,3,4,4a,5,6-hexahydro-1H-pyrazino[2,1-d][1,5]benzoxazepine;
(4aR,6R)-8-chloro-10,11-difluoro-6-(methoxymethyl)-2,3,4,4a,5,6-hexahydro-1H-pyrazino[2,1-d][1,5]benzoxazepine;
(4aR,6S)-8-chloro-9,10-difluoro-6-(methoxymethyl)-2,3,4,4a,5,6-hexahydro-1H-pyrazino[2,1-d][1,5]benzoxazepine;
[(4aR,6S)-8-fluoro-9-methyl-2,3,4,4a,5,6-hexahydro-1H-pyrazino[2,1-d][1,5]benzoxazepin-6-yl]methanol;
(4aR,6S)-8-ethynyl-10,11-difluoro-6-(methoxymethyl)-2,3,4,4a,5,6-hexahydro-1H-pyrazino[2,1-d][1,5]benzoxazepine;
(4aR,6S)-10-fluoro-6-(methoxymethyl)-8-methyl-2,3,4,4a,5,6-hexahydro-1H-pyrazino[2,1-d][1,5]benzoxazepine;
(4aR,6S)-8-(difluoromethoxy)-6-(methoxymethyl)-2,3,4,4a,5,6-hexahydro-1H-pyrazino[2,1-d][1,5]benzoxazepine;
(4aR,6S)-10-(difluoromethoxy)-8-fluoro-6-(methoxymethyl)-2,3,4,4a,5,6-hexahydro-1H-pyrazino[2,1-d][1,5]benzoxazepine;
(4aR,6S)-8-chloro-10-methoxy-6-(methoxymethyl)-2,3,4,4a,5,6-hexahydro-1H-pyrazino[2,1-d][1,5]benzoxazepine;
(4aR,6S)-8-chloro-6-(methoxymethyl)-10-(trifluoromethyl)-2,3,4,4a,5,6-hexahydro-1H-pyrazino[2,1-d][1,5]benzoxazepine;
[(4aR,6S)-8-chloro-10-(trifluoromethyl)-2,3,4,4a,5,6-hexahydro-1H-pyrazino[2,1-d][1,5]benzoxazepin-6-yl]methanol;
(4aR,6S)-9,10,11-trifluoro-6-(methoxymethyl)-2,3,4,4a,5,6-hexahydro-1H-pyrazino[2,1-d][1,5]benzoxazepine;
(4aR,6S)-8-cyclopropyl-6-(methoxymethyl)-2,3,4,4a,5,6-hexahydro-1H-pyrazino[2,1-d][1,5]benzoxazepine;
(4aR,6S)-8-cyclopropyl-10-fluoro-6-(methoxymethyl)-2,3,4,4a,5,6-hexahydro-1H-pyrazino[2,1-d][1,5]benzoxazepine;
(4aR,6S)-8-fluoro-6-(methoxymethyl)-2,3,4,4a,5,6-hexahydro-1H-pyrazino[2,1-d][1,5]benzoxazepine;
(4aR,6S)-9-fluoro-6-(methoxymethyl)-2,3,4,4a,5,6-hexahydro-1H-pyrazino[2,1-d][1,5]benzoxazepine;
(4aR,6S)-8,10-dichloro-6-(methoxymethyl)-2,3,4,4a,5,6-hexahydro-1H-pyrazino[2,1-d][1,5]benzoxazepine;
(4aR,6S)-8-chloro-10,11-difluoro-9-methoxy-6-(methoxymethyl)-2,3,4,4a,5,6-hexahydro-1H-pyrazino[2,1-d][1,5]benzoxazepine;
(4aR,6S)-11-fluoro-6-(methoxymethyl)-2,3,4,4a,5,6-hexahydro-1H-pyrazino[2,1-d][1,5]benzoxazepine;
(4aR,6S)-8-chloro-9,10,11-trifluoro-6-(methoxymethyl)-2,3,4,4a,5,6-hexahydro-1H-pyrazino[2,1-d][1,5]benzoxazepine; and
(4aR,6S)-10,11-difluoro-6-(methoxymethyl)-8-(trifluoromethyl)-2,3,4,4a,5,6-hexahydro-1H-benzo[b]pyrazino[1,2-d][1,4]oxazepine;

or an N-oxide or a pharmaceutically acceptable salt thereof.

**14.** A compound as claimed in claim 13, selected from the group consisting of:

(4aR,6S)-8-Chloro-10-fluoro-6-(methoxymethyl)-2,3,4,4a,5,6-hexahydro-1H-pyrazino[2,1-d][1,5]benzoxazepine;
(4aR,6S)-8-(Difluoromethyl)-10-fluoro-6-(methoxymethyl)-2,3,4,4a,5,6-hexahydro-1H-pyrazino[2,1-d][1,5]benzoxazepine;
(4aR,6S)-8-Chloro-10,11-difluoro-6-(methoxymethyl)-2,3,4,4a,5,6-hexahydro-1H-pyrazino[2,1-d][1,5]benzoxazepine; and
(4aR,6S)-8-Chloro-9,10,11-trifluoro-6-(methoxymethyl)-2,3,4,4a,5,6-hexahydro-1H-pyrazino[2,1-d][1,5]benzoxazepine;

or an N-oxide or a pharmaceutically acceptable salt thereof.

Europäisches Patentamt
European Patent Office
Office européen des brevets

# EUROPEAN SEARCH REPORT

Application Number
EP 19 21 2082

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 2011/130382 A1 (WANG ET. AL.) 2 June 2011 (2011-06-02) * page 43, paragraph [0788]; claims; examples 252, 253 * ----- | 1-14 | INV. C07D498/04 C07D498/14 A61K31/553 A61P25/24 |
| A | EP 2 213 675 A1 (TAKEDA PHARMACEUTICAL CO.LTD.) 4 August 2010 (2010-08-04) * claims; examples * ----- | 1-14 | |

TECHNICAL FIELDS SEARCHED (IPC)

C07D
A61K
A61P

The present search report has been drawn up for all claims

1

| Place of search | Date of completion of the search | Examiner |
|---|---|---|
| Munich | 2 January 2020 | Helps, Ian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 19 21 2082

This annex lists the patent family members relating to the patent documents cited in the above-mentioned European search report. The members are as contained in the European Patent Office EDP file on The European Patent Office is in no way liable for these particulars which are merely given for the purpose of information.

02-01-2020

| Patent document cited in search report | Publication date | Patent family member(s) | Publication date |
|---|---|---|---|
| US 2011130382 A1 | 02-06-2011 | NONE | |
| EP 2213675 A1 | 04-08-2010 | EP 2213675 A1 | 04-08-2010 |
| | | JP 5361733 B2 | 04-12-2013 |
| | | JP WO2009063991 A1 | 31-03-2011 |
| | | US 2010286120 A1 | 11-11-2010 |
| | | WO 2009063991 A1 | 22-05-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02100350 A
- WO 2010124042 A
- WO 2011133182 A
- US 20110130382 A
- WO 0364572 A
- WO 1997010223 A
- WO 2005099353 A
- WO 1995007271 A
- WO 2006008754 A
- US 7538189 B
- US 7534814 B
- US 7531685 B
- US 7528131 B
- US 7521421 B
- US 7514068 B
- US 7511013 B
- US 20090137457
- US 20090131485 A
- US 20090131363 A
- US 20090118238 A
- US 20090111840 A
- US 20090105338 A
- US 20090105307 A
- US 20090105147 A
- US 20090093422 A
- US 20090088416 A
- US 20090082471 A
- US 20070225277 A
- US 20080146583 A
- US 20070225274 A

### Non-patent literature cited in the description


- **DAVIS KL ; KAHN RS ; KO G ; DAVIDSON M.** Dopamine in schizophrenia: a review and re-conceptualization. *Am J Psychiatry,* 1991, vol. 148, 1474-86
- **WEINBERGER DR ; BERMAN KF.** Prefrontal function in schizophrenia: confounds and controversies. *Philos Trans R Soc Lond B Biol Sci,* 1996, vol. 351, 1495-503
- **REMINGTON G ; KAPUR S.** Atypical antipsychotics: are some more atypical than others?. *Psychopharmacol,* 2000, vol. 148, 3-15
- **KAUFMAN MJ ; HIRATA F.** Cyclic GMP inhibits phosphoinositide turnover in choroid plexus: evidence for interactions between second messengers concurrently triggered by 5-HT2C receptors. *Neurosci Lett,* 1996, vol. 206, 153-156
- **POMPEIANO M ; PALACIOS JM ; MENGOD G.** Distribution of the serotonin 5-HT2 receptor family mRNAs: comparison between 5-HT2A and 5-HT2C receptors. *Brain Res Mol Brain Res,* 1994, vol. 23, 163-178
- **LOPEZ-GIMENEZ JF ; MENGOD G ; PALACIOS JM ; VILARO MT.** Regional distribution and cellular localization of 5-HT2C receptor mRNA in monkey brain: comparison with [3H]mesulergine binding sites and choline acetyltransferase mRNA. *Synapse,* 2001, vol. 42, 12-26
- **G. J. DIAZ et al.** *Journal of Pharmacological and Toxicological Methods,* 2004, vol. 50, 187-199
- Fortschritte der Arzneimittelforschung. Advances in drug research. Birkhäuser Verlag, 1966, vol. 10, 224
- *Tetrahedron,* 2001, vol. 57, 9199 ff-9225 ff
- Microwaves in Organic Synthesis. Wiley-VCH, 2002
- *Journal of Medicinal Chemistry,* 1995, vol. 38 (11), 1892-1903
- *Journal of Heterocyclic Chemistry,* 1981, vol. 18 (7), 1305-1308
- *Journal of the American Chemical Society,* 2001, vol. 123 (25), 5962-5973
- **T. GREENE ; P. WUTS.** Protective Groups in Organic Synthesis. John Wiley & Sons, 1999
- **BLAKE et al.** *J. Pharm. Sci.,* 1975, vol. 64 (3), 367-391
- **FOSTER et al.** Advances in Drug Research. Academic press, 1985, vol. 14, 2-36
- **KATO et al.** *J. Labelled Comp. Radiopharmaceut.,* 1995, vol. 36 (10), 927-932
- **KUSHNER et al.** *Can. J. Physiol. Pharmacol.,* 1999, vol. 77, 79-88
- **PONS G ; REY E.** *Pediatrics,* 1999, vol. 104, 633
- **COWARD W A et al.** *Lancet,* 1979, vol. 7, 13
- **SCHWARCZ H P.** *Control. Clin. Trials,* 1984, vol. 5, 573
- **RODEWALD L E et al.** *J. Pediatr.,* 1989, vol. 114, 885
- **BUTTE N F et al.** *Br. J. Nutr.,* 1991, vol. 65, 3

- **MACLENNAN A H et al.** *Am. J. Obstet Gynecol.,* 1981, vol. 139, 948
- **CZAJKA D M ; FINKEL A J.** *Ann. N.Y. Acad. Sci.,* 1960, vol. 84, 770
- **THOMSON J F.** *Ann. New York Acad. Sci,* 1960, vol. 84, 736
- **CZAKJA D M et al.** *Am. J. Physiol.,* 1961, vol. 201, 357
- **BLAGOJEVIC N et al.** *Dosimetry & Treatment Planning for Neutron Capture Therapy,* 1994, 125-134
- *Diabetes Metab.,* 1997, vol. 23, 251
- **LIZONDO, J et al.** *Drugs Fut,* 1996, vol. 21 (11), 1116
- **BRICKNER, S J et al.** *J Med Chem,* 1996, vol. 39 (3), 673
- **MALLESHAM, B et al.** *Org Lett,* 2003, vol. 5 (7), 963
- **TECOTT LH ; SUN LM ; AKANA SF ; STRACK AM ; LOWENSTEIN DH ; DALLMAN MF ; JULIUS D.** Eating disorder and epilepsy in mice lacking 5-HT2C serotonin receptors. *Nature,* 1995, vol. 374, 542-546
- **CHOU-GREEN JM ; HOLSCHER TD ; DALLMAN MF ; AKANA SF.** Compulsive behavior in the 5-HT2C receptor knockout mouse. *Phys. Behav.,* 2003, vol. 78, 641-649
- **CHOU-GREEN JM ; HOLSCHER TD ; DALLMAN MF ; AKANA SF.** Repeated stress in young and old 5-HT2C receptor knockout mouse. *Phys. Behav.,* 2003, vol. 79, 217-226
- **FRANK MG ; STRYKER MP ; TECOTT LH.** Sleep and sleep homeostasis in mice lacking the 5-HT2C receptor. *Neuropsychopharmacology,* 2002, vol. 27, 869-873
- **ROCHA BA ; GOULDING EH ; O'DELL LE ; MEAD AN ; COUFAL NG ; PARSONS LH ; TECOTT LH.** Enhanced locomotor, reinforcing and neurochemical effects of cocaine in serotonin 5-hydroxytryptamine 2C receptor mutant mice. *J. Neurosci.,* 2002, vol. 22, 10039-10045
- **WERRY, TD ; LOIACONO R ; SEXTON PA ; CHRISTOPOULOS A.** RNA editing of the serotonin 5-HT2C receptor and its effects on cell signaling, pharmacology and brain function. *Pharmac. Therap.,* 2008, vol. 119, 7-23
- **SCHMAUSS C.** Serotonin 2C receptors: suicide, serotonin, and runaway RNA editing. *Neuroscientist,* 2003, vol. 9, 237-242
- **IWAMOTO K ; KATO T.** RNA editing of serotonin 2C receptor in human postmortem brains of major mental disorders. *Neurosci. Lett.,* 2003, vol. 346, 169-172
- **IWAMOTOA K ; NAKATANIB N ; BUNDOA M ; YOSHIKAWAB T ; KATOA T.** Altered RNA editing of serotonin 2C receptor in a rat model of depression. *Neurosci. Res.,* 2005, vol. 53, 69-76
- **DU Y ; STASKO M ; COSTA AC ; DAVISSONE MT ; GARDINER KJ.** Editing of the serotonin 2C receptor pre-mRNA Effects of the Morris Water Maze. *Gene,* 2007, vol. 391, 186-197
- **NISWENDER CM ; HERRICK-DAVIS K ; DILLEY GE ; MELTZER HY ; OVERHOLSER JC ; STOCKMEIER CA ; EMESON RB ; SANDERS-BUSH E.** RNA Editing of the Human Serotonin 5-HT2C Receptor: Alterations in Suicide and Implications for Serotonergic. *Pharmacotherapy. Neuropsychopharm.,* 2001, vol. 24, 478-491
- **SMITH BM et al.** Discovery and structure-activity relationship of (1R)-8-chloro-2,3,4,5-tetrahydro-1-methyl-1H-3-benzazepine (Lorcaserin), a selective serotonin 5-HT2C receptor agonist for the treatment of obesity. *J Med Chem,* 2008, vol. 51, 305-313
- **THOMSEN WJ ; GROTTICK AJ ; MENZAGHI F ; REYES-SALDANA H ; ESPITIA S ; YUSKIN D ; WHELAN K ; MARTIN M ; MORGAN M ; CHEN W.** A Novel Selective Human 5-HT2C Agonist: In Vitro and In Vivo Pharmacological Characterization. *J Pharmacol Exp Ther.,* 2008, vol. 325, 577-587
- **ROSENZWEIG-LIPSON S ; ZHANG J ; MAZANDARANI H ; HARRISON BL ; SABB A ; SABALSKI J ; STACK G ; WELMAKER G ; BARRETT JE ; DUNLOP J.** Antiobesity-like effects of the 5-HT2C receptor agonist WAY-161503. *Brain Res.,* 2006, vol. 1073-1074, 240-251
- **DUNLOP J ; SABB AL ; MAZANDARANI H ; ZHANG J ; KALGAONKER S ; SHUKHINA E ; SUKOFF S ; VOGEL RL ; STACK G ; SCHECHTER L.** WAY-163909 [97bR, 10aR)-1,2,3,4,8,9,10,10a-octahydro-7bH-cyclopenta-[b][1,4]diazepino[6,7,1hi]indole], a novel 5-hydroxytryptamine 2C receptor -selective agonist with anorectic activity. *J Pharmacol Exp Ther.,* 2005, vol. 313, 862-869
- **ROSENZWEIG-LIPSON S ; SABB A ; STACK G ; MITCHELL P ; LUCKI I ; MALBERG JE ; GRAUER S ; BRENNAN J ; CRYAN JF ; SUKOFF RIZZO SJ.** Antidepressant-like effects of the novel, selective, 5-HT2C receptor agonist WAY-163909 in rodents. *Psychopharmacology,* 2007, vol. 192, 159-170
- **ROSENZWEIG-LIPSON S ; DUNLOP J ; MARQUIS KL.** 5-HT2C receptor agonists as an innovative approach for psychiatric disorders. *Drug news Perspect,* 2007, vol. 20, 565-571
- **CRYAN, JF ; LUCKI I.** Antidepressant-like behavioral effects mediated by 5-Hydroxytryptamine 2C receptors. *J. Pharm. Exp. Ther.,* 2000, vol. 295, 1120-1126
- **DI MATTEO, V. ; DI GIOVANNI, G. ; DI MASCIO, M. ; ESPOSITO, E.** SB 242084, a selective serotonin 2C receptor antagonist, increases dopaminergic transmission in the mesolimbic system. *Neuropharmacology,* 1999, vol. 38, 1195-1205

- **DI GIOVANNI, G. ; DI MATTEO, V. ; DI MASCIO, M. ; ESPOSITO, E.** Preferential modulation of mesolimbic vs. nigrostriatal dopaminergic function by serotonin2C/2B receptor agonists: a combined in vivo electrophysiological and microdialysis study. *Synapse,* 2000, vol. 35, 53-61
- **MARQUIS KL ; SABB AL ; LOGUE SF ; BRENNAN JA ; PIESLA MJ ; COMERY TA ; GRAUER SM ; ASHBY CR, JR. ; NGUYEN HQ ; DAWSON LA.** WAY-163909 [(7bR,10aR)-1,2,3,4,8,9,10,10a-octahydro-7bH-cyclopenta-[b][1,4]diazepino[6,7,1hi]indole]: A novel 5-hydroxytryptamine 2C receptor-selective agonist with preclinical antipsychotic-like activity. *J Pharmacol Exp Ther,* 2007, vol. 320, 486-496
- **SIUCIAK JA ; CHAPIN DS ; MCCARTHY SA ; GUANOWSKY V ; BROWN J ; CHIANG P ; MARALA R ; PATTERSON T ; SEYMOUR PA ; SWICK A.** CP-809,101, a selective 5-HT2C agonist, shows activity in animal models of antipsychotic activity. *Neuropharmacology,* 2007, vol. 52, 279-290
- **DUNLOP J ; MARQUIS KL ; LIM HK ; LEUNG L ; KAO J ; CHEESMAN C ; ROSENZWEIG-LIPSON S.** Pharmacological profile of the 5-HT2C receptor agonist WAY-163909; therapeutic potential in multiple indications. *CNS Dug Rev.,* 2006, vol. 12, 167-177
- **ISAAC M.** Serotonergic 5-HT2C receptors as a potential therapeutic target for the antiepileptic drugs. *Curr. Topics Med. Chem.,* 2005, vol. 5 (59), 67
- **THORSLUND K ; NORDLIND K.** Serotonergic drugs-a possible role in the treatment of psoriasis?. *Drug News Perspect,* 2007, vol. 20, 521-525
- **ESPOSITO E ; DI MATTEO V ; PIERUCCI M ; BENIGNO A ; DI GIAVANNI, G.** Role of central 5-HT2C receptor in the control of basal ganglia functions. *The Basal Ganglia Pathophysiology: Recent Advances,* 2007, 97-127
- **BARR AM ; LAHMANN-MASTEN V ; PAULUS M ; GAINETDINOV RP ; CARON MG ; GEYER MA.** The selective serotonin-2A receptor antagonist M100907 reverses behavioral deficits in dopamine transporter knockout mice. *Neuropsychopharmacology,* 2004, vol. 29, 221-228
- **DEKEYNE A ; MANNOURY LA COUR C ; GOBERT A ; BROCCO M ; LEJUENE F ; SERRES F ; SHARP T ; DASZUTA A ; SOUMIER A ; PAPP M.** S32006, a novel 5-HT2C receptor antagonists displaying broad-based antidepressant and anxiolytic properties in rodent models. *Psychopharmacology,* vol. 199, 549-568
- **NUNES-DE-SOUZA V ; NUNES-DE-SOUZA RL ; RODGERS RJ ; CANTO-DE-SOUZA A.** 5-HT2 receptor activation in the midbrain periaqueductal grey (PAG) reduces anxiety-like behavior in mice. *Behav. Brain Res.,* 2008, vol. 187, 72-79
- **LEONE M ; RIGAMONTI A ; D'AMICO D ; GRAZZI L ; USAI S ; BUSSONE G.** The serotonergic system in migraine. *Journal of Headache and Pain,* 2001, vol. 2 (1), S43-S46
- **ARJONA AA ; POOLER AM ; LEE RK ; WURTMAN RJ.** Effect of a 5-HT2C serotonin agonist, dexnorfenfluramine, on amyloid precursor protein metabolism in guinea pigs. *Brain Res.,* 2002, vol. 951, 135-140
- **NAKAE A ; NAKAI K ; TANAKA T ; HAGIHIRA S ; SHIBATA M ; UEDA K ; MASIMO T.** The role of RNA editing of the serotonin 2C receptor in a rat model of oro-facial neuropathic pain. *The European Journal of Neuroscience,* 2008, vol. 27, 2373-2379
- **NAKAE A ; NAKAI K ; TANAKA T ; TAKASHINA M ; HAGIHIRA S ; SHIBATA M ; UEDA K ; MASHIMO T.** Serotonin 2C receptor mRNA editing in neuropathic pain model. *Neurosci. Res.,* 2008, vol. 60, 228-231
- **KAO T ; SHUMSKY JS ; JACOB-VADAKOT S ; TIMOTHY HB ; MURRAY M ; MOXON, KA.** Role of the 5-HT2C receptor in improving weight-supported stepping in adult rats spinalized as neonates. *Brain Res.,* 2006, vol. 1112, 159-168
- **MOTOFEI IG.** A dual physiological character for sexual function: the role of serotonergic receptors. *BJU International,* 2008, vol. 101, 531-534
- **SHIMADA I ; MAENO K ; KONDOH Y ; KAKU H ; SUGASAWA K ; KIMURA Y ; HATANAKA K ; NAITOU Y ; WANIBUCHI F ; SAKAMOTO S.** Synthesis and structure-activity relationships of a series of benzazepine derivatives as 5-HT2C receptor agonists. *Bioorg. Med. Chem.,* 2008, vol. 16, 3309-3320
- **FLETCHER PJ ; LE AD ; HIGGINS GA.** Serotonin receptors as potential targets for modulation of nicotine use and dependence. *Progress Brain Res.,* 2008, vol. 172, 361-83
- **BUBAR MJ ; CUNNINGHAM KA.** Prospects for serotonin 5-HT2R pharmacotherapy in psychostimulant abuse. *Progress Brain Res.,* 2008, vol. 172, 319-46
- **SHARIF NA ; MCLAUGHLIN MA ; KELLY CR.** AL-34662: a potent, selective, and efficacious ocular hypotensive serotonin-2 receptor agonist. *J Ocul Pharmacol Ther.,* 2006, vol. 23, 1-13
- **OBATA, HIDEAKI ; ITO, NAOMI ; SASAKI, MASAYUKI ; SAITO, SHIGERU ; GOTO, FUMIO.** Possible involvement of spinal noradrenergic mechanisms in the antiallodynic effect of intrathecally administered 5 - HT2C receptor agonists in the rats with peripheral nerve injury. *European Journal of Pharmacology,* 2007, vol. 567 (1-2), 89-94

- Department of Anesthesiology & Intensive Care Medicine. **NAKAE, AYA ; NAKAI, KUNIHIRO ; TANAKA, TATSUYA ; TAKASHINA, MASAKI ; HAGIHIRA, SATOSHI ; SHIBATA, MASAHIKO ; UEDA, KOICHI ; MASHIMO, TAKASHI.** Neuroscience Research. Graduate School of Medicine, Osaka University, 2008, vol. 60, 228-231
- Department of Anesthesiology. **OBATA, HIDEAKI ; SAITO, SHIGERU ; SAKURAZAWA, SHINOBU ; SASAKI, MASAYUKI ; USUI, TADASHI ; GOTO, FUMIO.** Pain. Gunma University Graduate School of Medicine, 2004, vol. 108, 163-169
- Department of Pharmacology. **BRUS, RYSZARD ; KASPERSKA, ALICJA ; OSWIECIMSKA, JOANNA ; SZKILNIK, RYSZARD.** Medical Science Monitor. Silesian Medical University, 1997, vol. 3, 654-656
- **BRENNAN, PAUL E. ; WHITLOCK, GAVIN A. ; HO, DANNY K. H. ; CONLON, KELLY ; MCMURRAY, GORDON.** *Bioorganic & Medicinal Chemistry Letters,* 2009, vol. 19 (17), 4999-5003
- Department of Pharmacology. **MBAKI, Y. ; RAMAGE, A. G.** British Journal of Pharmacology. University College London, 2008, vol. 155, 343-356
- **SHEN JHQ et al.** A 6-week randomized, double-blind, placebo-controlled, comparator referenced trial of vabicaserin in acute schizophrenia. *Journal of Psychiatric Research,* 2014, vol. 53, 14-22
- **LIU J et al.** Prediction of Efficacy of Vabicaserin, a 5-HT2C Agonist, for the Treatment of Schizophrenia Using a Quantitative Systems Pharmacology Model. *CPT Pharmacometrics Syst. Pharmacol.,* 2014, vol. 3, e111
- **DUNLOP J et al.** Characterization of Vabicaserin (SCA-136), a Selective 5-Hydroxytryptamine 2C Receptor Agonist. *J Pharmacol Exp Ther,* 2011, vol. 337, 673-80
- **SIUCIAK J et al.** CP-809,101, a selective 5-HT2C agonist, shows activity in animal models of antipsychotic activity. *Neuropharmacology,* 2007, vol. 52, 279-290
- **MOSIENKO V et al.** Exaggerated aggression and decreased anxiety in mice deficient in brain serotonin. *Transl Psychiatry,* 2012, vol. 2, e122
- **DEL GUIDICE T et al.** Stimulation of 5-HT2C Receptors Improves Cognitive Deficits Induced by Human Tryptophan Hydroxylase2 Loss of Function Mutation. *Neuropsychopharmacology,* 2014, vol. 39, 1125-1134
- **ROSENZWEIG-LIPSON et al.** Antidepressant-like effects of the novel, selective, 5-HT2C receptor agonist WAY-163909 in rodents. *Psychopharmacology,* 2007, vol. 192, 159-170
- Methods in Cell Biology. Academic Press, 1976, vol. XIV, 33
- **S. M. BERGE et al.** pharmaceutically acceptable salts. *J. Pharmaceutical Sciences,* 1977, vol. 66, 1
- *The Society for Biomolecular Screening,* 2003, 453-462
- *DMD,* 1999, vol. 27 (11), 1350-1359